# EUROPEAN PATENT SPECIFICATION

(11) **EP 4 706 834 B1**
(45) Date of publication and mention of the grant of the patent: **11.11.2026**
(21) Application number: 26153493.7
(22) Date of filing: 16.08.2023
(51) Int. Cl.: A61N 7/02, B06B 1/06

(54) **AIR-BACKED TRANSDUCERS FOR ULTRASOUND DENERVATION AND SYSTEMS THAT USE AIR-BACKED TRANSDUCERS**
LUFTGESTÜTZTE WANDLER FÜR ULTRASCHALL-DENERVIERUNG UND SYSTEME MIT LUFTGESTÜTZTEN WANDLERN
TRANSDUCTEURS À AIR POUR DÉNERVATION ULTRASONORE ET SYSTÈMES UTILISANT DES TRANSDUCTEURS À AIR

(30) Priority: 16.08.2022 US 202263371635 P; 16.08.2022 US 202263371638 P; 30.03.2023 US 202363493268 P
(43) Date of publication of application: 11.03.2026
(62) Document number(s) of the earlier application(s) in accordance with Art. 76 EPC: 23762011.7 / 4 572 849
(73) Proprietor: Otsuka Medical Devices Co., Ltd., Tokyo 101-0048 (JP)
(72) Inventor: THIRUMALAI, Shruthi, Palo Alto, CA 94303 (US); ZHAI, Liang, Palo Alto, CA 94303 (US); MERINO, Jaime, Palo Alto, CA 94303 (US); LARIOS, Maria Veronica, Palo Alto, CA 94303 (US); GABEL, Peter, Palo Alto, CA 94303 (US)
(74) Representative: Barker Brettell Sweden AB

(56) References cited:
- CN-A- 111 821 587
- GB-A- 901 195
- JP-A- 2002 113 005
- US-A1- 2016 000 499
- US-B1- 6 210 356
- US-B2- 10 456 605

## Description

### PRIORITY CLAIM

This application claims priority to each of the following U.S. Provisional Patent Applications:
U.S. Provisional Patent Application No. 63/493,268, to Thirumalai et al., filed March 30, 2023 (PMD00095US); U.S. Provisional Patent Application No. 63/371,638, to Thirumalai et al., filed August 16, 2022 (PMD00059US); and U.S. Provisional Patent Application No. 63/371,635, to Zhai, filed August 16, 2022 (PMD00058US).

### BACKGROUND

### FIELD

This application relates generally to minimally-invasive apparatuses and systems that provide energy delivery to a targeted anatomical location of a subject, and more specifically, to catheter-based, intraluminal devices and systems configured to deliver ultrasonic energy to treat tissue, such as nerve tissue. Methods disclosed hereafter do not form part of the claimed invention.

### BACKGROUND INFORMATION

High blood pressure, also known as hypertension, commonly affects adults. Left untreated, hypertension can result in renal disease, arrhythmias, and heart failure. Treatment of hypertension has focused on interventional approaches to inactivate the renal nerves surrounding a renal artery. Autonomic nerves tend to follow blood vessels to the organs that they innervate. Intraluminal devices, such as catheters, may reach specific structures, such as the renal nerves, that are proximate to the lumens in which the catheters travel. Accordingly, catheter-based systems can deliver energy from within the lumens to inactivate the renal nerves in and/or surrounding the vessel walls.

One approach to renal nerve deactivation uses radio frequency (RF) energy. The RF energy is delivered to a catheter having multiple electrodes placed against the intima of the renal artery to create an electrical field in the vessel wall and surrounding tissue. The electrical field results in resistive (ohmic) heating of the tissue to ablate the tissue and the renal nerve passing through that tissue. To treat the renal nerves surrounding the renal arteries, the RF electrodes are repositioned several times around the inside of the renal artery.

A system having an ultrasound transducer that emits one or more therapeutic doses of unfocused ultrasound energy has advantages over RF systems. The ultrasound transducer can be mounted at a distal end of catheter, and the unfocused ultrasound energy can heat tissue adjacent to a body lumen within which the catheter (and the transducer) is disposed. The unfocused ultrasound energy system may also include a balloon mounted at the distal end of the catheter around the ultrasound transducer. A cooling fluid can be circulated through the balloon to cool the transducer and body lumen during ultrasound energy delivery. Such an unfocused ultrasound energy system may, for example, ablate target nerves surrounding the body lumen, without damaging non-target tissue such as the inner lining of the body lumen or unintended organs outside of the body lumen. Such a design enables creation of one or more ablation zones sufficient to achieve long-term nerve inactivation at different locations around the circumference of the blood vessel.

Catheters that output ultrasound energy advantageously allow ablative energy to be distributed around a vessel wall at greater depths than permissible with a radiofrequency ablative catheter. Ultrasound energy can be applied to nerves arranged around the vessel. For instance, ultrasound energy can be applied to the renal nerves surrounding the renal artery in order to deactivate these nerves. However, the arrangement of the nerves can change from patient to patient and can be at different locations around the vessel. Additionally, the vessel can be located near tissues and/or organs. As a result, it would be desirable to be able to limit the application of ultrasound energy to the targeted nerves while eliminating or reducing the application of ultrasound energy to the tissues and/or organs in order to optimize procedural efficacy and safety. Further, the vessels can include features such as calcification or plaque. Depending on the conditions, it may be desirable to apply ultrasound energy to the feature or to avoid the feature. As a result, it is desirable to be able to control the application of ultrasound energy within the vessel.
US 6 210 356 B1 discloses a catheter including an elongated body with an exterior surface and an ultrasound transducer with a longitudinal length. A support member supports the ultrasound transducer at the exterior surface of the elongated body and defines a chamber adjacent to the exterior surface of the elongated body. The chamber reduces transmission of ultrasound energy from the ultrasound transducer into the elongated body along the longitudinal length of the ultrasound transducer.
GB 901 195 A discloses an accelerometer or strain gauge comprising a longitudinally polarized tube of ceramic piezoelectric material provided with electrodes on inner and outer cylindrical surfaces and supported from at least one of these surfaces. The element is supported by a nickel-iron plug and responds to shear stress caused by axial movement of the plug. A threaded shank of the plug enables attachment to a piece of apparatus whose behaviour is being investigated. The inertia of the element may be increased by surrounding it by a massive metal sleeve. Copper electrodes formed by electrodeposition may be used. Contact between the plug and the inner electrode may be improved by knurling the plug and filling the interstices between the plug and the electrode with a conducting cement. Sensitivity to lateral forces may be reduced by ensuring that the electric axis of the tube is strictly parallel with its electric axis. This may be done by bending the axis of the supporting spigot. A flange of the spigot may be hermetically sealed to a housing which is provided with a further seal at its other extremity. Electrical connections are made to the housing and to the sleeve. In a modification, the ceramic tube is supported by a sleeve which fits closely between the tube and the housing. The spigot is replaced by a close-fitting metallic core which is unattached to the housing and merely provides inertia. The housing is attached to the apparatus being investigated by an external thread.
US 10 456 605 B2 discloses a neuromodulation system including a catheter has a balloon along its distal end. An ultrasound transducer positioned within an interior of the balloon can be selectively activated to emit acoustic energy radially outwardly, targeting nerve tissue and other portions of the subject anatomy. Targeted nerve tissue can be heated by the application of ultrasonic energy to neuromodulate the tissue. The system may be delivered over a guidewire. A catheter enhances fluid delivery to a distal end of the catheter while reducing an overall diameter of the catheter. The catheter comprises a guidewire lumen that is eccentric relative to a center axis of the catheter.
JP 2002113005 A discloses an ultrasonic probe provided with a backing frame containing an inner structure composed of a piezoelectric element, a first and a second sound matching layers, and backing material, an outer member of a sound lens engaged with the backing frame to enclose the outer circumference of the sound lens as a sound emission surface of the sound lens is opened, and a hermetic connector disposed on the back surface side of the backing frame, and having a signal terminal engaged with the backing frame. The backing frame and the exterior member are gas-tightly jointed with each other by a first gas-tight joint part. The backing frame and the hermetic connector are gas-tightly jointed with each other by a second gas-tight joint part.
US 2016000499 A1 discloses methods and endovascular catheters for assessing, and treating patients having sympathetically mediated disease, involving augmented peripheral chemoreflex and heightened sympathetic tone by reducing chemosensor input to the nervous system via transmural carotid body modulation using a catheter with an ionic liquid stream electrode.
CN 111821587 A discloses a urethral catheter assembly comprising a urethral catheter, an adjusting assembly and at least one ultrasonic transducer set, wherein each ultrasonic transducer set comprises two ultrasonic transducers; the two ultrasonic transducers in the same ultrasonic transducer set are arranged on the periphery of the urethral catheter at intervals in the axis direction of the urethral catheter; and the adjusting assembly is used for adjusting the angles between the outer surfaces of the ultrasonic transducers and the axis direction of the urethral catheter, so that ultrasonic waves emitted by the two ultrasonic transducers in the same ultrasonic transducer set are converged or focused. The ultrasonic transducers can focus the ultrasonic waves from the interior of the human body to a hidden affected part under the adjusting effect of the adjusting assembly, so that the ultrasonic wave focusing accuracy is guaranteed; and due to the fact that the distance between the urethral catheter assembly and the affected part is smaller, the treatment effect can be achieved with less energy, and meanwhile, the harm of high temperature generated by the assembly in a high-power state to a patient is avoided.

### SUMMARY

The invention is defined by independent claim 1. Aspects of the disclosure useful for understanding the invention are described below. An apparatus comprising an ultrasound transducer configured for emitting ultrasound is provided. The ultrasound transducer comprises a hollow piezoelectric transducer body having a longitudinal axis and a radially inner surface, a tubular backing support member extending longitudinally through the piezoelectric transducer body and having a radially outer surface, a chamber defined between the radially inner surface of the piezoelectric transducer body and the radially outer surface of the tubular backing support member and between a first end and an opposite second end in an axial direction, wherein said ends are each formed by a conductive part and a gas-tight sealing layer of metal solder material in contact with the surface of the conductive part, which is opposite to the chamber.

An apparatus comprising an ultrasound transducer configured for emitting ultrasoundis provided. The ultrasound transducer comprises a hollow piezoelectric transducer body which comprises a tube of piezoelectric material, wherein said tube has a radially inner surface and a radially outer surface; an inner electrode disposed on at least a portion of the inner surface of the tube of piezoelectric material; an outer electrode disposed on at least a portion of the outer surface of the tube of piezoelectric material; a tubular backing support member extending longitudinally through the piezoelectric transducer body and having a radially outer surface, a gas-tight chamber defined between the radially inner surface of the piezoelectric transducer body and the radially outer surface of the tubular backing support member and having a first end and an opposite second end in an axial direction, wherein the piezoelectric material is made from Navy Type III (PZT-8) high density lead zirconate titanate (PZT) piezoelectric material.

A non-claimed method for use with a catheter including an air-backed ultrasound transducer on a distal portion of the catheter is provided. The method comprises inserting the distal portion of the catheter into a body lumen of a patient so that the air-backed ultrasound transducer is positioned proximate to nerves surrounding the body lumen that are to be denervated; causing the air-backed ultrasound transducer to emit ultrasonic waves having a first power density for a first period of time, while the air-backed ultrasound transducer is positioned proximate to the nerves surrounding the body lumen that are to be denervated; and causing the air-backed ultrasound transducer to emit ultrasonic waves having a second power density for a second period of time that occurs after the first period of time, while the air-backed ultrasound transducer is positioned proximate to the nerves surrounding the body lumen that are to be denervated; wherein the second power density differs from the first power density.

A tissue treatment device is provided. The tissue treatment device comprises a catheter having a distal end an ultrasound transducer positioned at the distal end of the catheter, the ultrasound transducer comprising inner and outer surfaces, each of the inner and outer surfaces comprising an electrode; and a backing support member, wherein the ultrasound transducer is mounted to the backing support member to define an air chamber adjacent the inner surface, the air chamber being insulated to prevent entry of fluid into the air chamber during use, the backing support member having a distal end and a proximal end, wherein the backing support member comprises at least a first stand-off post at the distal end of the backing support member and at least a second stand-off post at the proximal end of the backing support member, wherein each of the at least first and second stand-off posts comprise an inner face and an outer face, the inner faces being on an interior of the air chamber, the outer faces being exterior to the air chamber, wherein the at least first and second stand-off posts are soldered to the ultrasound transducer only at the outer face of the at least first and second stand-off posts, wherein the ultrasound transducer is configured to deliver sufficient acoustic energy during sonication such as to thermally induce modulation of neural fibers surrounding a blood vessel sufficient to improve a measurable physiological parameter corresponding to a diagnosed condition of the patient.

An apparatus is provided, the apparatus comprising an ultrasound transducer configured for emitting ultrasound, wherein said ultrasound transducer comprises a hollow piezoelectric transducer body having a longitudinal axis and a radially inner surface; a tubular backing support member extending longitudinally through the piezoelectric transducer body and having a radially outer surface; a chamber defined between the radially inner surface of the piezoelectric transducer body and the radially outer surface of the tubular backing support member and between a first end and an opposite second end in an axial direction, wherein said ends are each formed by a conductive part and a gas-tight sealing layer of metal solder material in contact with the surface of the conductive part, which is opposite to the chamber; and a controller configured to cause the ultrasound transducer to emit ultrasound waves having a power density between 170 to 327 Watts per centimeter square for a period of time between 2 to 4 seconds.

### BRIEF DESCRIPTION OF THE DRAWINGS

The novel features of the present disclosure are set forth with particularity in the claims that follow. A better understanding of the features and advantages of the present disclosure will be obtained by reference to the following detailed description that sets forth illustrative embodiments, in which the principles of the invention are utilized, and the accompanying drawings.
FIG. 1A illustrates a side view of a catheter system, in accordance with many embodiments.
FIG. 1B illustrates a side view of a hub of a catheter system, in accordance with many embodiments.
FIG. 1C illustrates a section view along line 1C of the catheter shaft in FIG. 1B, in accordance with many embodiments.
FIG. 2A illustrates a backing support member for a water-backed transducer.
FIG. 2B illustrates a backing support member for an air-backed transducer, in accordance with many embodiments.
FIG. 2C illustrates a water-backed transducer assembly design.
FIG. 2D illustrates an air-backed transducer assembly design, in accordance with many embodiments.
FIG. 2E illustrates a section view of a water-backed transducer assembly design.
FIG. 2F illustrates a section view of an air-backed transducer assembly design having one step at the proximal end of the transducer, in accordance with many embodiments.
FIG. 2G illustrates a section view of an air-backed transducer assembly design having one step at the proximal end of the transducer, in accordance with many embodiments.
FIG. 2H illustrates a section view of an air-backed transducer assembly design having a step and an air-backed transducer assembly design having no steps, in accordance with many embodiments.
FIG. 2IA illustrates a side view of a water-backed backing support member.
FIG. 2IB illustrates a side view of an air-backed backing support member, in accordance with many embodiments.
FIG. 2J illustrates a side view of an air-backed backing support member with a cap at the distal end, in accordance with many embodiments.
FIG. 2K illustrates a side view of a backing support member having a stand-off post at the proximal end, in accordance with many embodiments.
FIG. 2L illustrates a side view of a backing support member having no stand-off post at the proximal end, in accordance with many embodiments.
FIG. 2M illustrates a soldering design at the proximal end of a backing support member without a stand-off post, in accordance with many embodiments.
FIG. 2N illustrates a cover (aka collar) used at the proximal end of a backing support, in accordance with many embodiments.
FIG. 20 illustrates a soldering design at the proximal end of a backing support member with a stand-off post, in accordance with many embodiments.

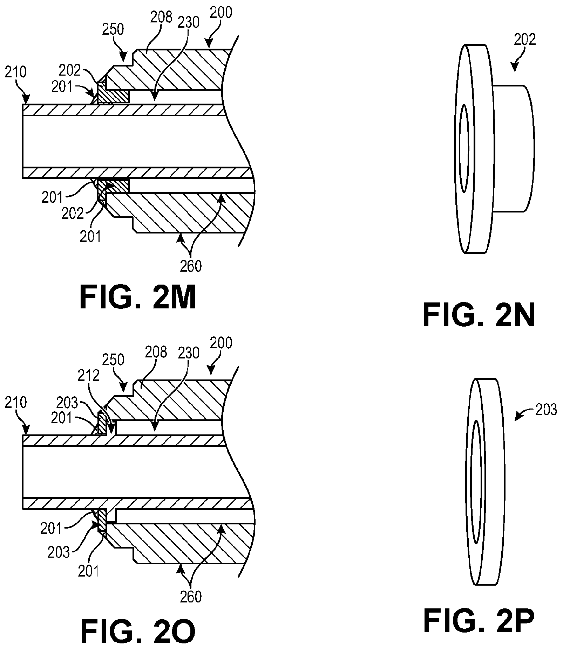

FIG. 2P illustrates a washer used at the proximal end of a backing support member, in accordance with many embodiments.

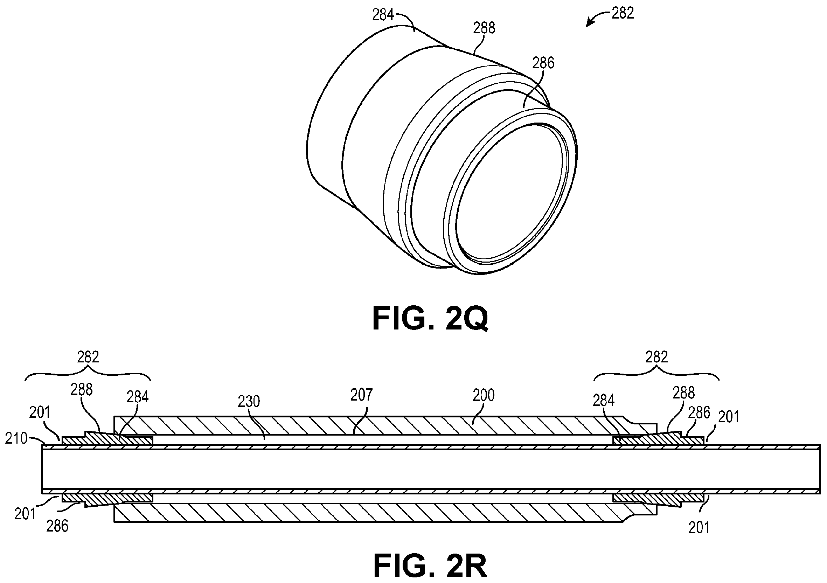

FIG. 2Q illustrates a perspective view of a ramped cover (aka ramped collar) that can be used at both the proximal end of a backing support, and the distal end of the backing support, to provide an air-backed transducer in accordance with certain embodiments.
FIG. 2R illustrates how instances of the ramped cover (aka ramped collar) that can be used at both the proximal end of a backing support, and the distal end of the backing support, to provide an air-backed transducer in accordance with certain embodiments.

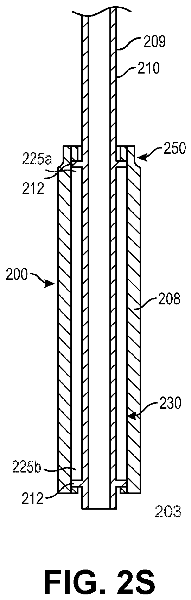

FIG. 2S illustrates a soldering design at the proximal and distal ends of a backing support member with stand-off posts, in accordance with many embodiments.

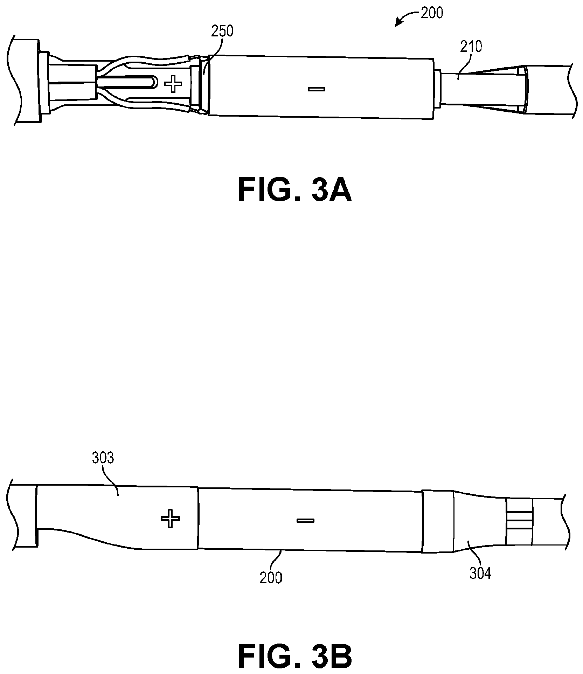

FIG. 3A illustrates a water-backed transducer, in accordance with many embodiments.
FIG. 3B illustrates a transducer where the ID is encapsulated, in accordance with many embodiments.

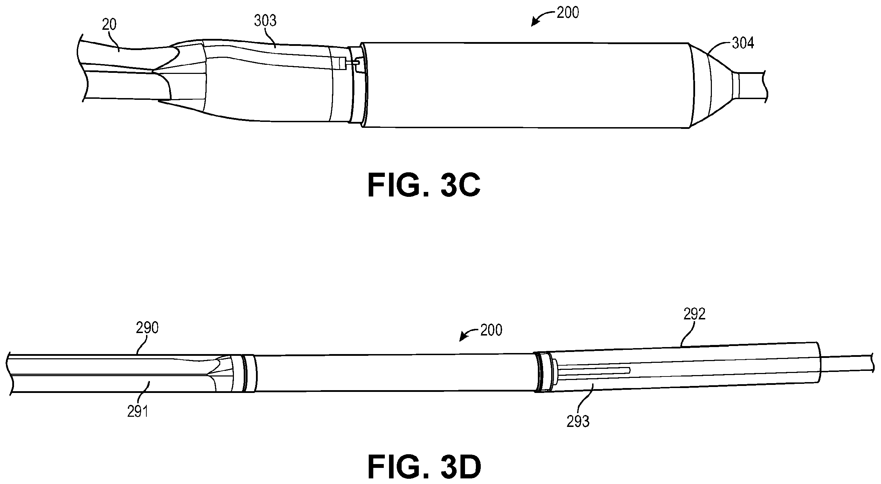

FIG. 3C illustrates an air-backed, saline compatible transducer, in accordance with many embodiments.
FIG. 3D illustrates an air-backed, saline compatible transducer, in accordance with further embodiments.

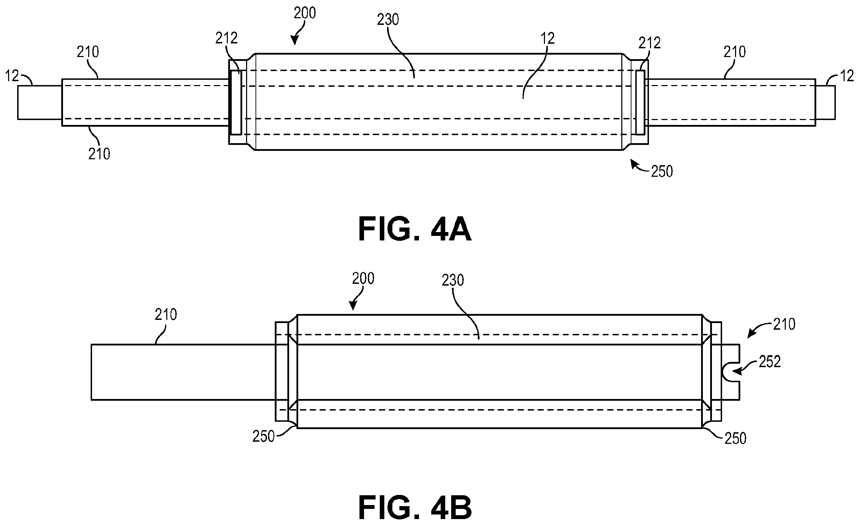

FIG. 4A illustrates a two-part backing support member comprising dual steps on the transducer, in accordance many embodiments.
FIG. 4B illustrates a continuous backing support member comprising dual steps on the transducer, in accordance with many embodiments.

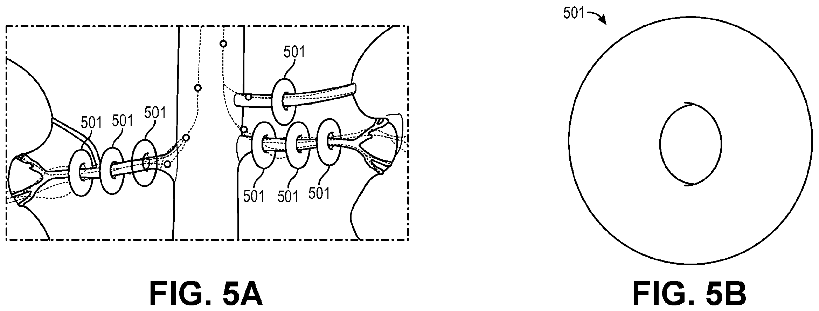

FIGS. 5A and 5B illustrate uniform and circumferential delivery of energy with an air-backed transducer, in accordance with many embodiments.

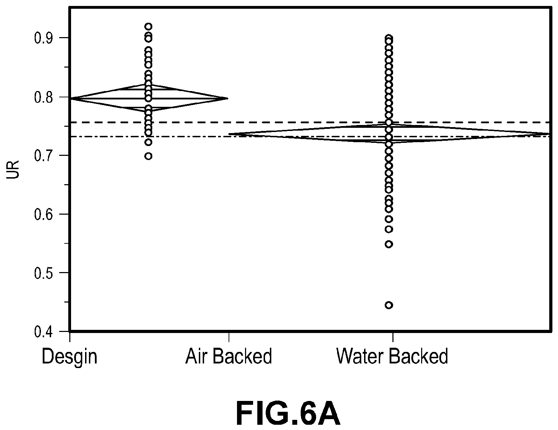

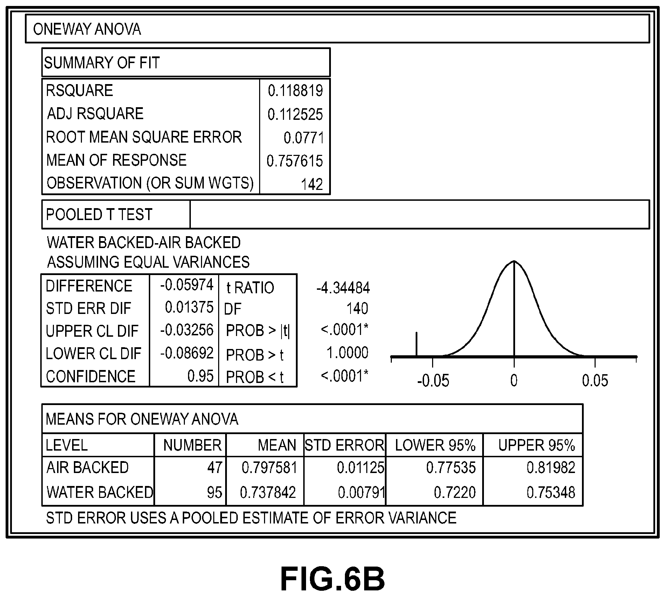

FIGS. 6A and 6B are a graph and a data table, respectively, illustrating safety and efficacy data of a high frequency, air-backed transducer, in accordance with many embodiments.

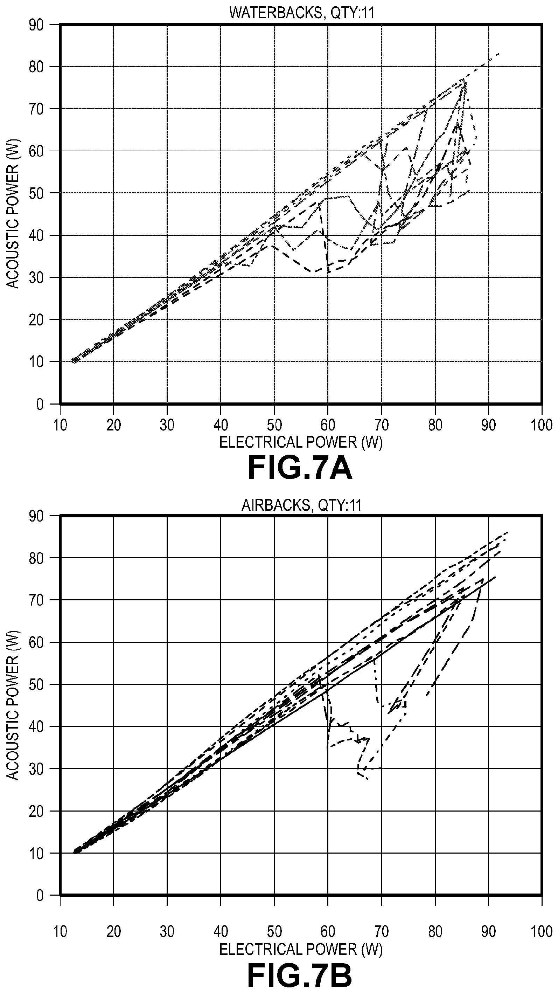

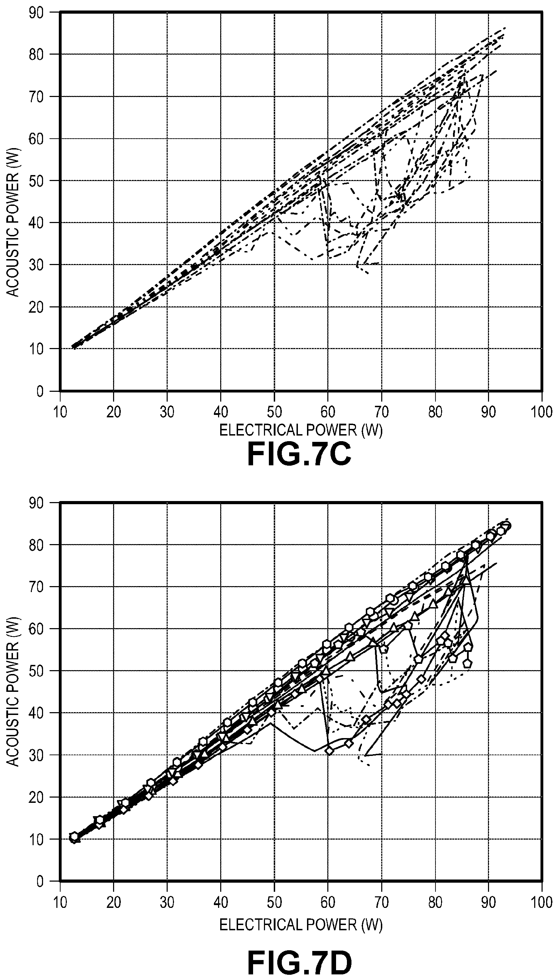

FIGS. 7A, 7B, 7C, and 7D are graphs illustrating the results of breakdown tests of water-backed and air-backed transducers, in accordance with many embodiments.

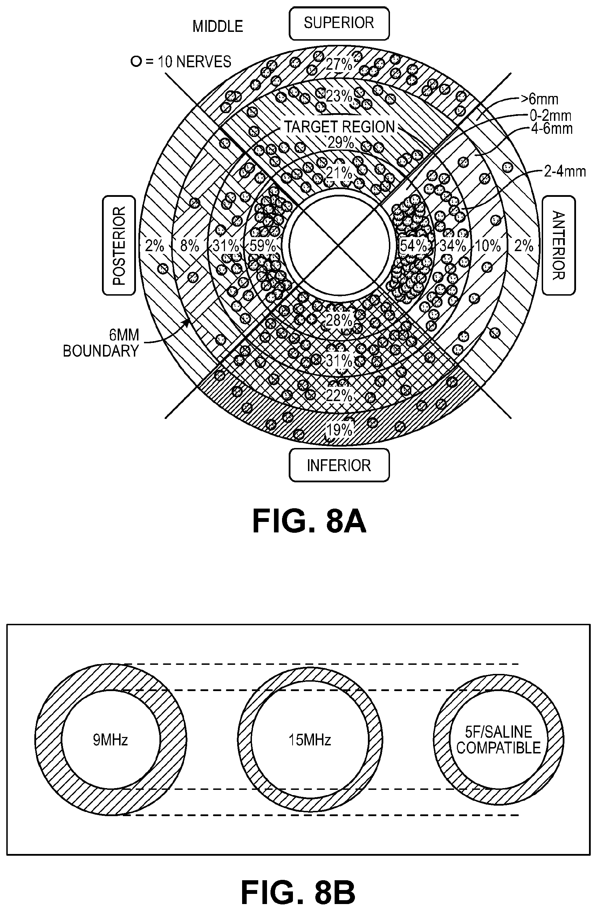

FIG. 8A illustrates a target region for nerve ablation, in accordance with many embodiments.
FIG. 8B illustrates enhanced safety by reducing the non-targeted region, in accordance with many embodiments.

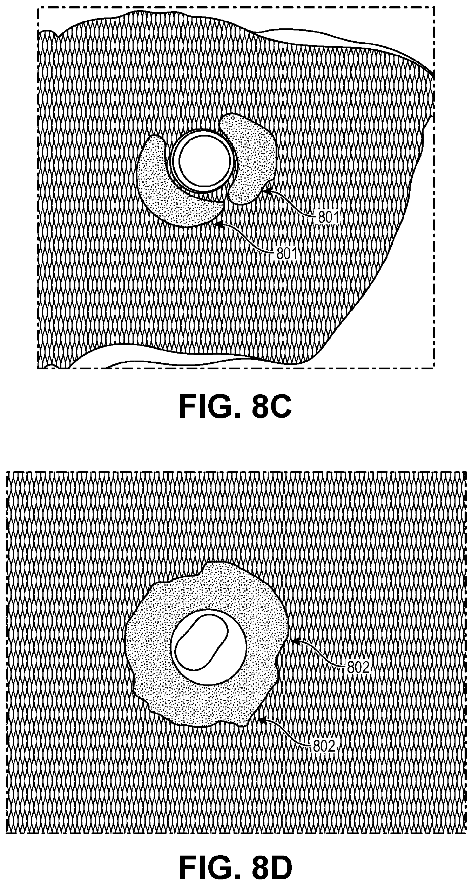

FIG. 8C illustrates partial circumferential delivery of energy.

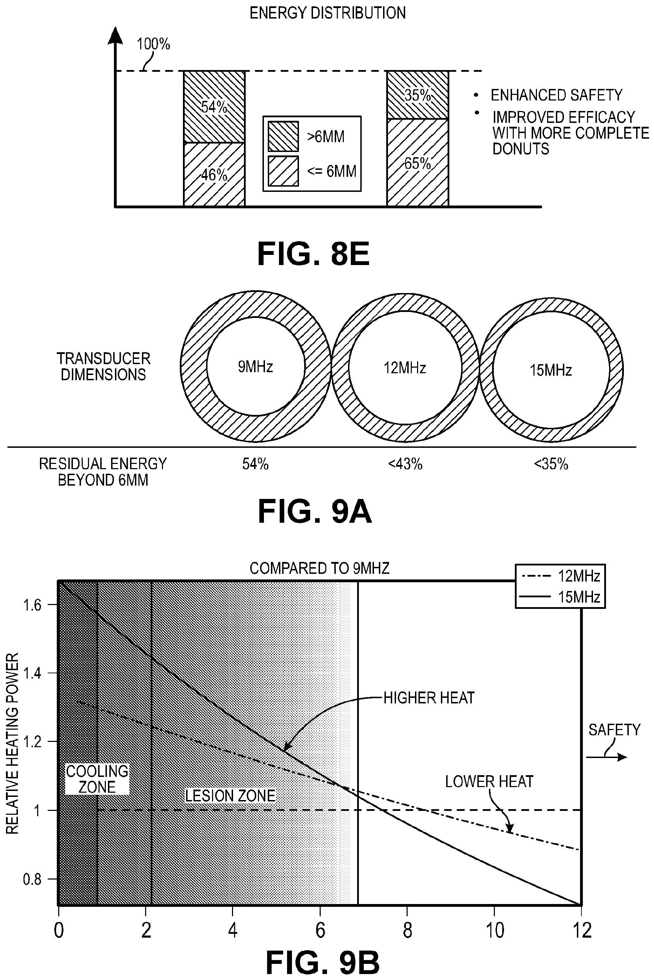

FIG. 8Dillustrates improved uniform and circumferential delivery of energy, in accordance with many embodiments.
FIG. 8E is a table illustrating energy delivery of a 9 MHz transducer versus a 15 MHz transducer, in accordance with many embodiments.
FIGS. 9A and 9B illustrate a comparison between high frequency transducers, in accordance with many embodiments.

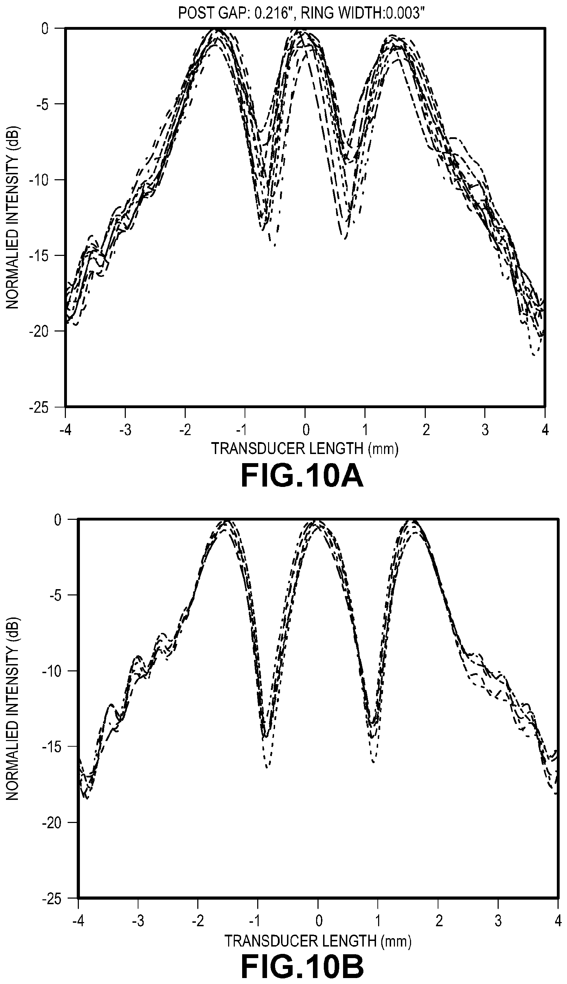

FIG. 10A is a graph illustrating asymmetry of the lobes without a dual step design, in accordance with many embodiments.
FIG. 10B is a graph illustrating symmetry of the lobes with a dual step design, in accordance with many embodiments.

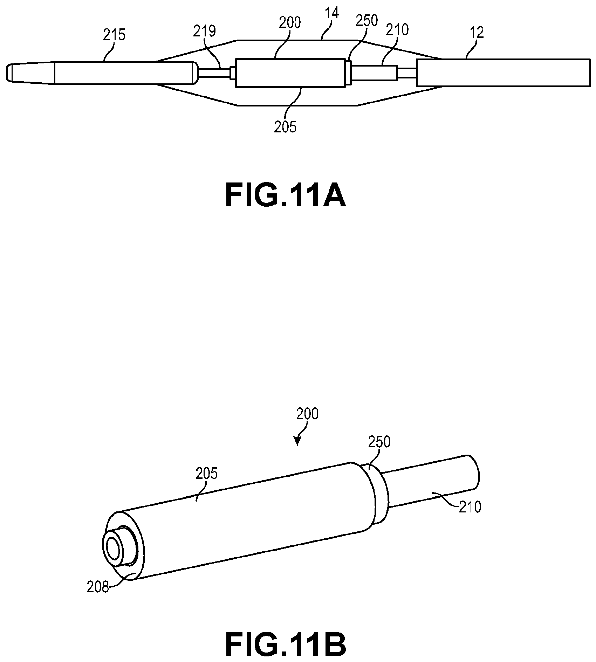

FIGS. 11A is a side view of a distal portion of a catheter of an ultrasound-based tissue treatment system including an air-backed transducer in accordance with certain embodiments of the present technology.
FIGS. 11B is a perspective view of the air-backed transducer in accordance with certain embodiments of the present technology.

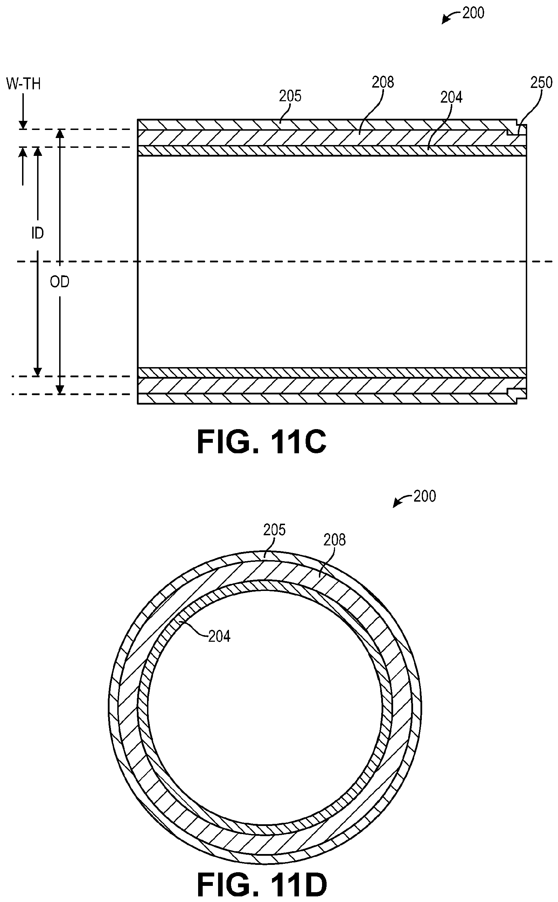

FIG. 11C illustrates a longitudinal cross-sectional view of the air-backed transducer introduced in FIGS. 11A and 11B.
FIG. 11D illustrates a radial cross-sectional view of the air-backed transducer introduced in FIGS. 11A and 11B.

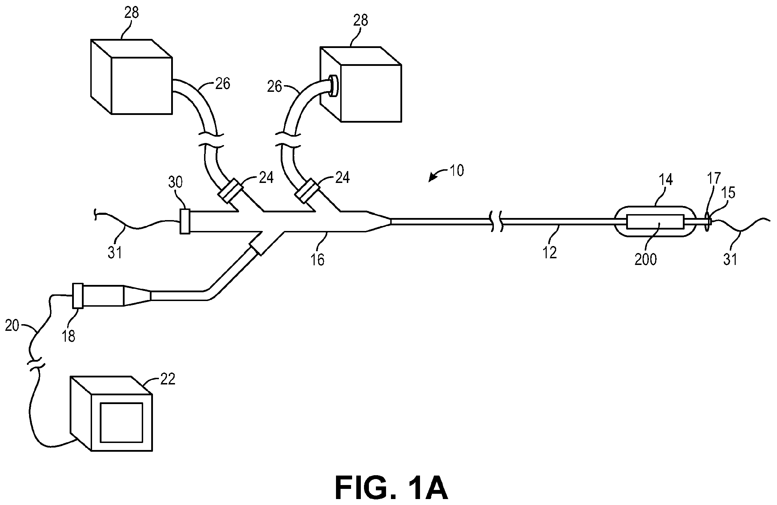

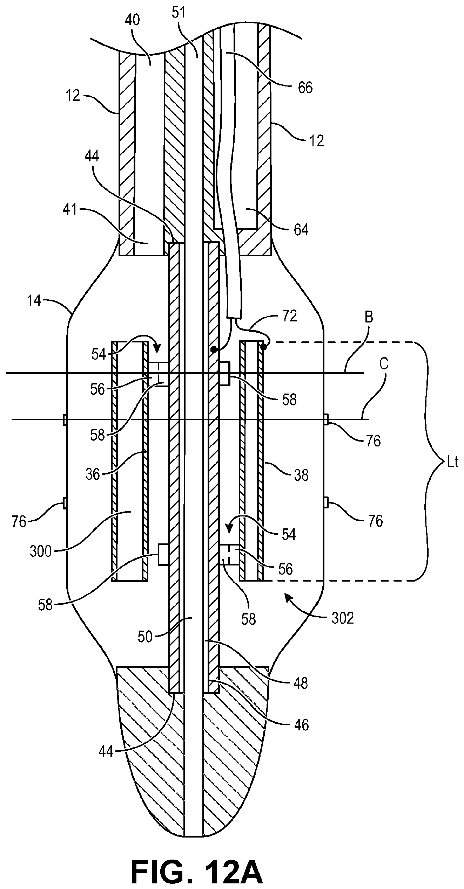

FIG. 12A through FIG. 12D illustrate a catheter suitable for use with the catheter system of FIG. 1A.
FIG. 12A is a cross section of the distal end of a catheter taken along the longitudinal axis of the catheter.

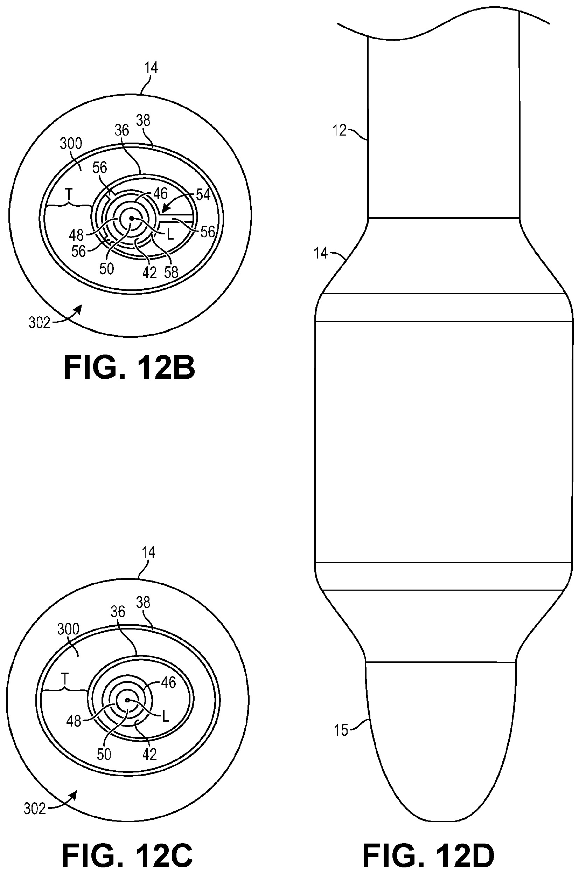

FIG. 12B is a cross section of the catheter shown in FIG. 12A taken along the line labeled B in FIG. 12A.
FIG. 12C is a cross section of the catheter shown in FIG. 12A taken along the line labeled C in FIG. 12A.
FIG. 12D is a sideview of the distal end of the catheter shown in FIG. 12A.

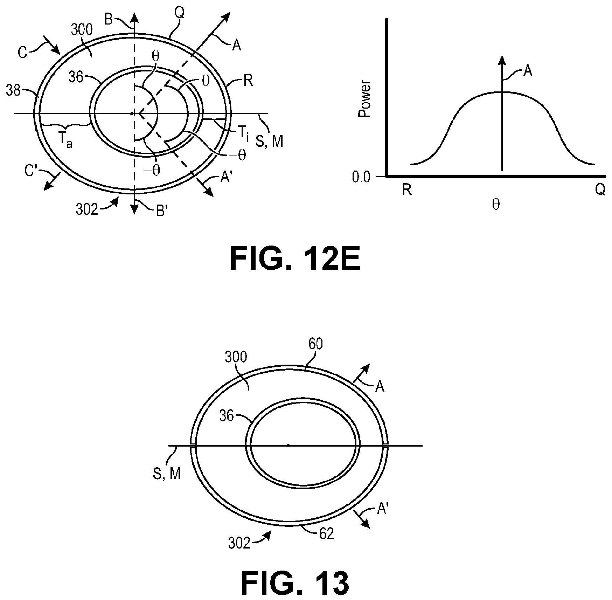

FIG. 12E includes a cross section of a transducer from the catheter of FIG. 12C.
FIG. 13 illustrates the transducer of FIG. 12E modified such that an outer electrode is divided into multiple outer electrodes.

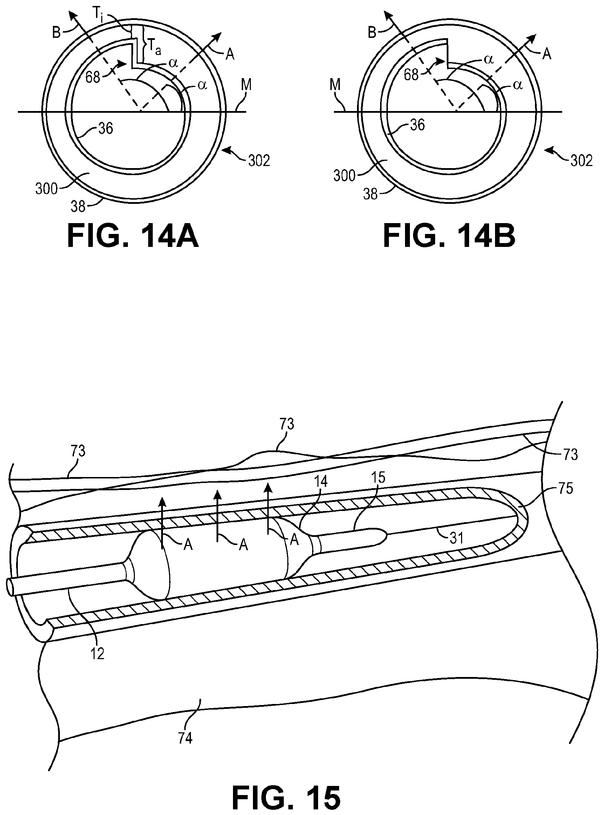

FIG. 14A shows the transducer of FIG. 12E modified such that the transducer excludes regions of the same thickness.
FIG. 14B shows the transducer of FIG. 14A modified such that an inner electrode is not positioned over and/or in contact with a transition region.
FIG. 15 illustrates the balloon of a catheter positioned at a treatment site in the body of a patient.

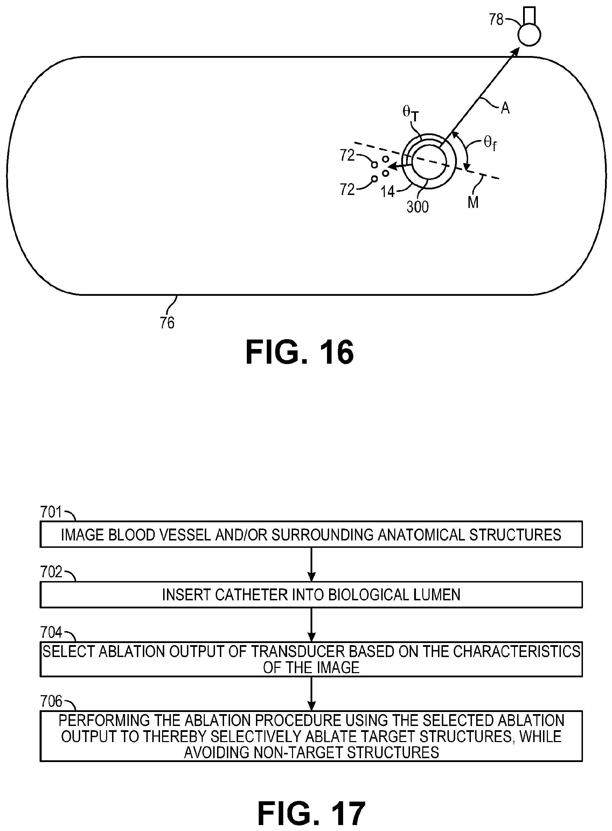

FIG. 16 illustrates a catheter positioned at a treatment site in the body of a patient.
FIG. 17 is a high-level flow diagram used to summarize methods according to certain embodiments of the present technology.

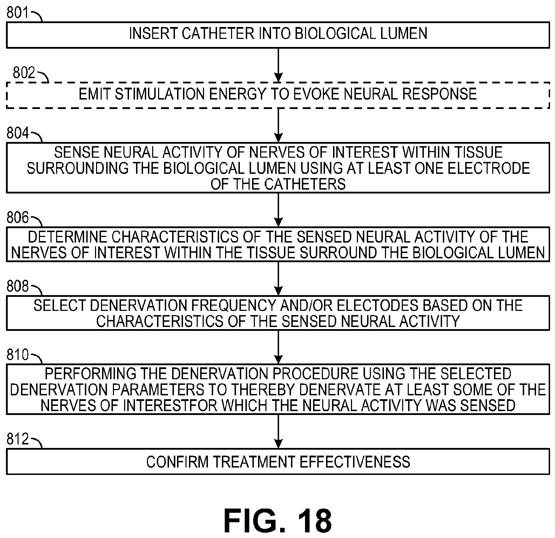

FIG. 18 is a high-level flow diagram used to summarize methods according to certain embodiments of the present technology.

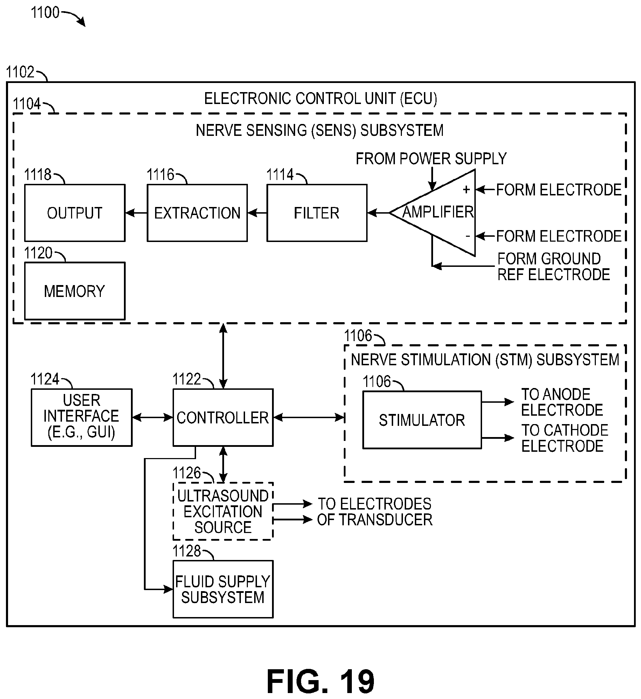

FIG. 19 is a schematic diagram of an example system, according to an embodiment of the present technology, for interfacing with a patient's blood vessels.

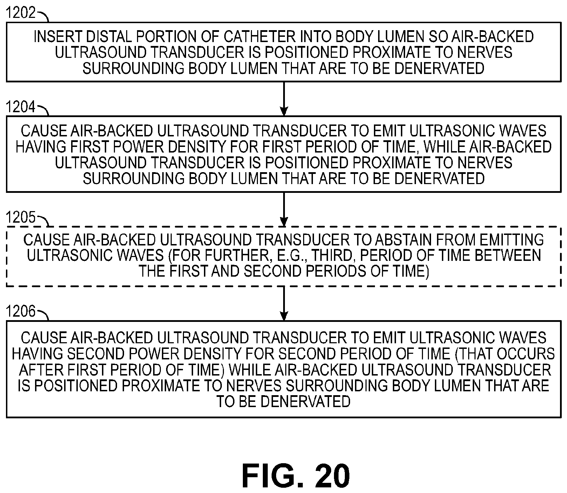

FIG. 20 is a high-level flow diagram used to summarize method according to further embodiments of the present technology.

### DETAILED DESCRIPTION

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

Certain inventive embodiments herein contemplate numerical ranges. When ranges are present, the ranges include the range endpoints. Additionally, every sub range and value within the range is present as if explicitly written out. The term "about" or "approximately" may mean within an acceptable error range for the particular value, which will depend in part on how the value is measured or determined, e.g., the limitations of the measurement system. For example, "about" may mean within 1 or more than 1 standard deviation, per the practice in the art. Alternatively, "about" may mean a range of up to 20%, up to 10%, up to 5%, or up to 1% of a given value. Where particular values are described in the application and claims, unless otherwise stated the term "about" meaning within an acceptable error range for the particular value may be assumed.

The terms rear surface, inner surface, and inner diameter of the active element refer to the same region of the active element of the air-backed transducer.

Certain embodiments described herein are directed to an apparatus that comprises an air-backed ultrasound transducer, the air-backed ultrasound transducer comprising: a piezoelectric transducer body comprises a hollow cylindrical tube of piezoelectric material having an inner surface and an outer surface; an inner electrode disposed on at least a portion of the inner surface of the piezoelectric transducer body; an outer electrode disposed on at least a portion of the outer surface of the piezoelectric transducer body; a backing support member comprising a hollow tube having an inner surface and a radially outer surface 209 and extending longitudinally through the hollow cylindrical tube of the piezoelectric material of the piezoelectric transducer body and comprising a longitudinal opening configured to accept a guide wire; and first and second stand-off members extending between and defining an air chamber between the radially outer surface 209 of the hollow tube of the backing support member 210 and the inner surface of the hollow cylindrical tube of the piezoelectric material of the piezoelectric transducer body; wherein the inner electrode is located within the air chamber and is thereby isolated from any fluid that the piezoelectric transducer body is located within, such as fluid which is used for cooling the outer electrode; and wherein the piezoelectric transducer body is configured to generate ultrasonic waves in response to application of a voltage between the inner and outer electrodes. In certain such embodiments, the hollow cylindrical tube of piezoelectric material of the piezoelectric transducer body is made by hot isostatic pressing (HIPing) a Navy Type III high density lead zirconate titanate (PZT) piezoelectric material. In other words, the piezoelectric transducer body is made of hot isostatic pressed (HIPed) Navy Type III high density PZT piezoelectric material. The pressure used for HIPing can be in a range of 10-200 MPa at a temperature of 400-2000°C. The HIPing of the Navy Type III high density PZT piezoelectric material beneficially decreases the porosity of the Navy Type III high density PZT piezoelectric material, compared to if it were not HIPed. The HIPing of the Navy Type III high density PZT piezoelectric material also provides for a dissipation factor (DF) of less than 0.006, wherein the DF is defined as the ratio of the equivalent series resistance (ESR) and the magnitude of the capacitance reactance (Xc), and wherein low DF materials make better resonators. In certain such embodiments, the air-backed ultrasound transducer is configured to generate ultrasonic waves having a power density within a range of 100 Watts per centimeter square (W/cm2) to 327 W/cm2.

Certain embodiments described herein are directed to a method for use with a catheter including an air-backed ultrasound transducer on a distal portion of the catheter, the method comprising: inserting the distal portion of the catheter into a body lumen of a patient so that the air-backed ultrasound transducer is positioned proximate to nerves surrounding the body lumen that are to be denervated; causing the air-backed ultrasound transducer to emit ultrasonic waves having a first power density for a first period of time, while the air-backed ultrasound transducer is positioned proximate to the nerves surrounding the body lumen that are to be denervated; and causing the air-backed ultrasound transducer to emit ultrasonic waves having a second power density for a second period of time that occurs after the first period of time, while the air-backed ultrasound transducer is positioned proximate to the nerves surrounding the body lumen that are to be denervated; wherein the second power density differs from the first power density. In certain such embodiments, the first period of time has a duration within a first range of 2 to 4 seconds; and the second period of time has a duration within a second range of 2 to 4 seconds. In accordance with certain embodiments, the method further comprises causing the air-backed ultrasound transducer to abstain from emitting ultrasonic waves for a third period of time between the first and second periods of time; wherein the third period of time has a duration within a third range of 2 to 4 seconds. In accordance with certain embodiments, one of the first and second power densities is within a range of 170 Watts per centimeter square (W/cm²) to 327 W/cm²; and the other one of the first and second power densities is within a range of 50 W/cm² to 169 W/cm².

In certain embodiments described herein, a tissue treatment device is provide, the tissue treatment device comprising, a catheter having a distal end; an ultrasound transducer positioned at the distal end of the catheter, the ultrasound transducer comprising inner (back) and outer (front) surfaces, each of the inner and outer surfaces comprising an electrode; and a backing support member, wherein the ultrasound transducer is mounted to the backing support member to define an air chamber adjacent the inner surface, the air chamber being insulated to prevent entry of fluid into the air chamber during use, the backing support member having a distal end and a proximal end, wherein the ultrasound transducer is configured to deliver sufficient acoustic energy during sonication such as to thermally induce modulation of neural fibers surrounding a blood vessel sufficient to improve a measurable physiological parameter corresponding to a diagnosed condition of the patient.

In certain embodiments described herein, a method of ablating a target tissue is provided, the method comprising: advancing a catheter comprising an ablation element through at least one bodily vessel to a position at or near the target tissue, the ablation element comprising a piezoelectric component, a backing support member, and an air chamber therebetween; and energizing the piezoelectric component of the ablation element to deliver energy to the target tissue, thereby ablating the target tissue, wherein the target tissue comprises one or more sympathetic nerves or nerve branches.

In certain embodiments described herein, a system for ablating target tissue is provided, the system comprising: an ultrasound energy generator; and a catheter coupled to the ultrasound energy generator, the catheter being configured to be advanceable through at least one bodily vessel to a position at or near the target tissue, and the catheter comprising a catheter shaft and an ablation element at the distal end of the catheter, wherein the ablation element comprises a piezoelectric component, a backing support member, and an air chamber therebetween, and wherein the ultrasound energy generator is operatively coupled to the ablation element to energize the piezoelectric component to deliver energy to the target tissue, ablating the target tissue, wherein the target tissue comprises one or more nerves or nerve branches.

In certain embodiments described herein, an ultrasound tissue treatment catheter comprising a cylindrical ultrasound transducer is provided, the ultrasound transducer having an outer diameter of about 1.3 mm and an operating frequency of 11 to 15 MHz, wherein the ultrasound transducer is configured to deliver sufficient acoustic energy during sonication such as to thermally induce modulation of neural fibers surrounding a blood vessel sufficient to improve a measurable physiological parameter corresponding to a diagnosed condition of the patient.In certain embodiments described herein, a tissue treatment device is provided, the tissue treatment device comprising: a catheter being configured to be advanceable through at least one bodily vessel to a position at or near the target tissue, and the catheter comprising a catheter shaft and an ablation element carried by the distal end of the catheter, wherein the ablation element comprises a piezoelectric component having a first edge and a second edge, a backing support member having a cap at the distal end, and an air chamber between the ablation element and the backing support member, the air chamber being insulated to prevent entry of fluid into the air chamber during use, wherein the piezoelectric component is configured to deliver energy to the target tissue to ablate the target tissue.

FIGS. 1A-1C show an exemplary catheter system according to many embodiments. The catheter system may include a catheter 10 having a proximal end and a distal end. The catheter 10 may include a catheter shaft 12, a balloon 14, and a tip member 15. The balloon 14 can be positioned between the catheter shaft 12 and the tip member 15. The balloon 14 can be or include a compliant, semi-compliant, or non-compliant medical balloon 14. Suitable materials for the balloon 14 may include, but are not limited to nylon, polyimide films, thermoplastic elastomers such as those marked under the trademark PEBAX^{™}, medical-grade thermoplastic polyurethane elastomers such as those marketed under the trademark PELLETHANE^{™}, pellethane, isothane, and other suitable polymers or any combination thereof.

In an embodiment, the catheter system for ablating target tissue may comprise an ultrasound energy generator (electronics) 22 and a catheter 10 coupled to the ultrasound energy generator. The catheter 10 may be configured to be advanceable through at least one bodily vessel to a position at or near the target tissue. The catheter 10 may comprise a catheter shaft 12 and an ablation element on a distal portion of the catheter 10. The ablation element may comprise a piezoelectric component, e.g., ultrasound transducer 200, a backing support member 210, and an air chamber 230 therebetween. The ultrasound energy generator may be operatively coupled to the ablation element to energize the ultrasound transducer 200 to deliver energy to the target tissue, ablating the target tissue. The target tissue may comprise one or more nerves or nerve branches. In an embodiment, the target tissue may comprise nerves or nerve branches starting within the adventitia (i.e., beyond the intima-media thickness) and ending at or less than about 10 mm from the lumen of the blood vessel, e.g., less than 6mm from the lumen. In certain embodiments, an imaging transducer is used to find the media-adventitia border and ablation is initiated a set distance, e.g., .1mm, from the media-adventitia border. In certain embodiments, the target tissue may comprise nerves or nerve branches starting within more than 0.3 mm to 10 mm from the lumen of the blood vessel, e.g., 0.5 mm to 6 mm, or 1 mm to 6 mm of the lumen of one or more blood vessels, e.g., a renal artery, hepatic artery, and/or pulmonary artery. In an embodiment, the target tissue may comprise cardiac tissue, e.g., electrically conductive cardiac tissue.

In an embodiment, the generator may be configured to energize the piezoelectric component, e.g., ultrasound transducer 200, for a time period of between 5 to 20 seconds, at a frequency of 11 to 15 MHz, or both. In an embodiment, the generator may be configured to energize the piezoelectric component for a time period of between 6 to 10 seconds, at a frequency of 12 to 14 MHz, or both. In an embodiment, the generator may be configured to energize the piezoelectric component for a time period of about 7 seconds, at a frequency of about 13 MHz, or both. Energizing the piezoelectric component by the generator may increase a temperature of the piezoelectric component by no more than 50° C. Energizing the piezoelectric component by the generator delivers energy at an average surface acoustic intensity of between 20 and 150 W/cm².

The catheter 10 can have a handle 16 at the proximal end of the catheter shaft 12. The handle 16 can include one or more electrical couplings 18 for connecting the catheter system to one or more external electrical conductors 20 that are each in electrical communication with electronics 22, e.g., a generator. Suitable external electrical conductors 20 include, but are not limited to, wires, cables, and Flexible Printed Circuits (FPC).

The electronics 22 can control the catheter 10 to sweep the operating frequency and can control the durations of the individual pulses and total time of series of pulses to control the temperature in the ablation zones and shape the lesion.

The catheter shaft 12 can include one or more electrical lumens 121. Each of the electrical lumens 121 may extend from one or more of the electrical couplings 18 along a longitudinal length of the catheter shaft 12 toward a distal end of the catheter shaft 12. The electrical lumen can each hold one or more electrical conductor carriers that each carry one or more electrical conductors. The electrical conductors can be in electrical communication with the electronics 22 through the electrical coupling 18 and one or more of the external electrical conductors 20. Suitable electrical conductors include, but are not limited to, wires, insulated wires, cables, and Flexible Printed Circuits (FPC). When an electrical conductor carrier carries multiple electrical conductors 20, a suitable electrical conductor carrier can be an electrically insulating jacket. When an electrical conductor carrier carries a single electrical conductor 20, an electrical insulator on the electrical conductor can serve as the electrical conductor carrier.

The handle 16 can include one or more fluid ports 24 for connecting the catheter to a conduit 26. Suitable conduits 26 include, but are not limited to, tubes and hoses. A conduit 26 can provide fluid communication between the fluid port 24 and a fluid source 28. Suitable fluid sources 28 include, but are not limited to, pumps, tanks, reservoirs, and vessels. The catheter shaft 12 can include one or more fluid lumens 241. Each of the fluid lumens 241 can be in fluid communication with one of the fluid ports 24 along a longitudinal length of the catheter shaft 12 toward a distal end of the catheter shaft 12.

The handle 16 can include one or more guidewire ports 30 for receiving a guidewire 31. The catheter shaft 12 can include a guidewire lumen 301. The guidewire lumen 301 can extend along a longitudinal length of the catheter shaft 12 toward a distal end of the catheter shaft 12. The guidewire lumen can be in fluid communication with the guidewire port 30 such that a guidewire 31 inserted into the guidewire port 30 can be received within the guidewire lumen.

In certain embodiment, the catheter 10 can include an imaging transducer 17, e.g., a transducer of a single element or an array, at the distal end of catheter 10. The imaging transducer 17 may have a center frequency of 15-50MHz, e.g., 20-30MHz, which can be used to identify target and nontarget structures. The imaging transducer 17 can be positioned before or after the balloon 14. The imaging transducer 17 can comprise of a ring array having a single ring or multiple rings. The imaging depth can be up to approximately 12 mm and can be used to size the vessel, image anatomy, pathology, lesion formation, temperature changes, heat sinks such as lymph nodes, vessel walls, plaques, calcification, tissue layers and nerves. The imaging frequency can be approximately 20 MHz - 35 MHz, the bandwidth can be equal or greater to 10 MHz, the array size can comprise 16 to 256 elements, however, this is not restrictive. The array element dimension can be 0.5 mm - 1.5 mm in length and 0.5 mm - 2 wavelengths in width. Multiple-row cylindrical array can help reduce the image slice thickness to achieve better contrast resolution. The elements are individually controlled to transmit and receive, for example, by an ASIC circuit, to reduce the number of cables needed.

In certain embodiments, a catheter system may additionally or alternatively include electrodes on the balloon 14, configured to sense nerve activity and/or confirm the effectiveness of the treatment, as disclosed in U.S. Patent Publication No. 20230021354, to Zhai et al.

In certain embodiments, the catheter system may additionally or alternatively include the nerve sensing and/or treatment confirmation components disclosed in U.S. Patent Application No. 63/320,103 to Barman et al. filed March 15, 2022. As disclosed in further detail in U.S. Patent Application No. 63/320,103, treatment confirmation components may be used to determine a latency of sensed electrical impulses to determine the type, size, function and/or health of the fibers whose neural response is being sensed. The delay (aka latency) may be indicative of a depth of nerves surrounding a biological lumen (e.g., a renal artery) within which a catheter used to measure the delay is located. In accordance with certain embodiments of the present technology, the above-described delay (aka latency) can be used to select the frequency used for the ablations. The electronics 22 controls the catheter 10 to sweep the operating frequency and control the durations of the individual and total time to control the temperature in the ablation zones, and shape the lesion. A portion of the transducer 200 that activates at a lower frequency is used to aim at deeper regions when it is determined that nerves are located in the deeper regions. A portion of the transducer 200 that activates at a higher frequency is used to target nearer regions when it is determined that nerves are located in the nearer regions.

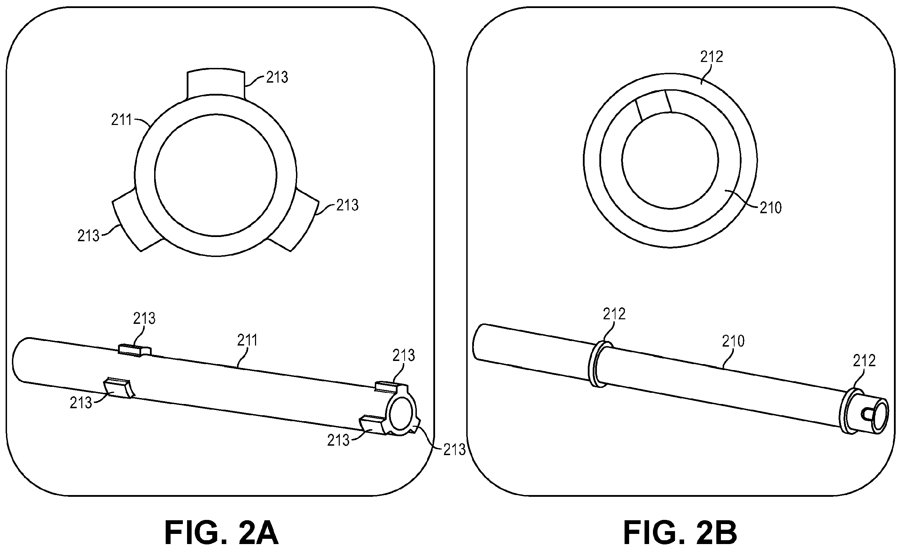

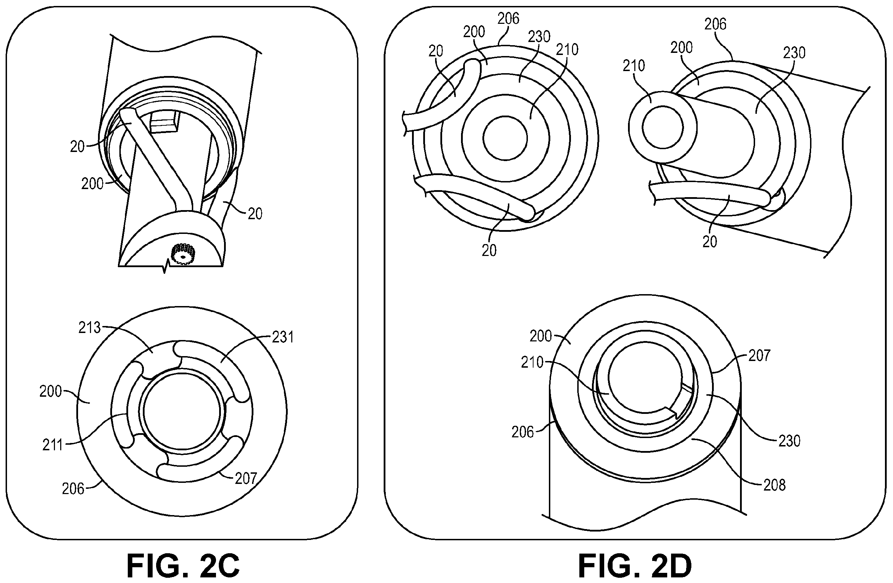

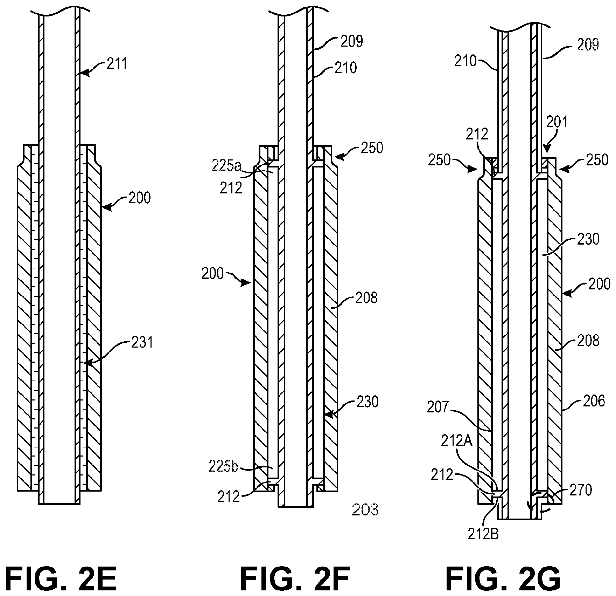

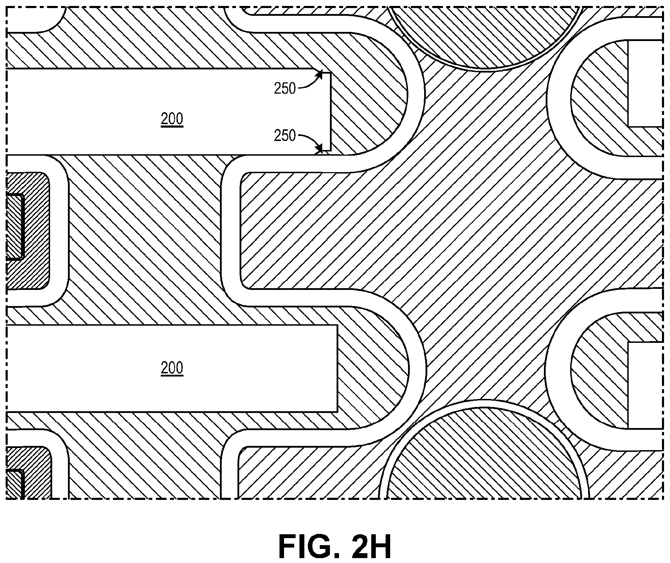

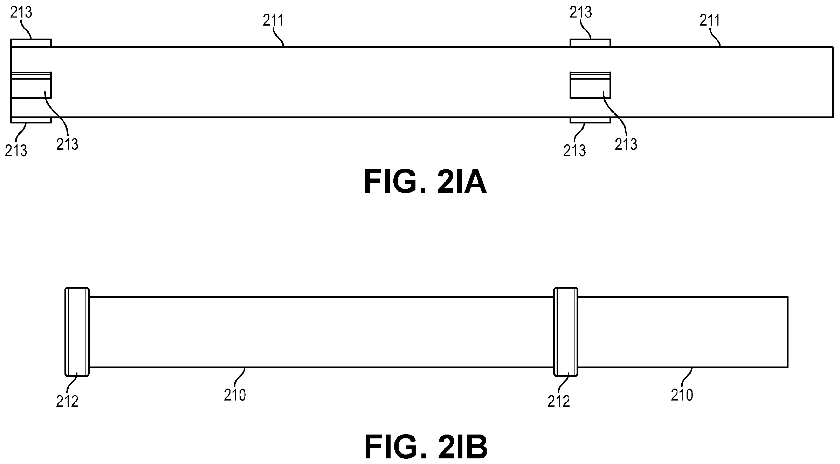

FIGS. 2A-2IB illustrate various transducers, including water-backed transducers (FIGS. 2A, 2C, 2E, 2IA) and air-backed transducers (FIGS. 2B, 2D, 2F, 2G, 2H, 2IB, 2S) and/or components thereof. Air-backed transducers have an air layer at the inner surface of the transducer or piezoelectric component. The air layer may help direct all acoustic energy transmitted through the outer surface or outer diameter of the transducer. Embodiments of the air-backed transducers herein can provide good uniformity and efficiency of energy to the treated tissue site and safer treatment. The uniformity of the lesion may rely on the concentricity of the piezoelectric transducer and less so on the support members that are separated from the PZT member by the air layer. However, the air-backed ultrasound transducer 200 can be non-concentric with the backing support member 210 and still function as intended. Accordingly, in certain embodiments the backing support member 210 is non concentrically arranged with the piezoelectric transducer body 208. As such, the dependency on the transducer assembly process can be reduced without sacrificing the uniformity of acoustic field. Air in the back of the transducer, next to the inner surface of the PZT member, can ensure that the acoustic waves are launched from the outer diameter (OD) of the transducer. Therefore, structures going through the cylindrical transducer such as a post or other assembly components, that are separated by the air layer from the PZT member has little or no impact on the acoustic efficiency. This opens up the geometrical design of the transducer and various components going through it. The OD profile can be shrunk or increased to any size as long as there is an air gap between the inner surface of the PZT member and the backing support member 210 or other assembly components. Reducing the size of the transducer enables use with a 4Fr or 5Fr catheter, for example. With a 4Fr or 5Fr catheter, the device may be advanced to target sites within a subject's or patient's vasculature, such as to the renal arteries, through their radial artery instead of larger arteries, such as the femoral artery. Radial access for advancing a catheter carrying ultrasound transducer(s) according to the present disclosure is described in: U.S. Provisional Patent Application No. 63/383,816 (Attorney Docket: PMD00091US), to Mazzone et al., filed November 15, 2022, U.S. Provisional Patent Application No. 63/375,357 (Attorney Docket: PMD00076US), to Mazzone et al., filed September 12, 2022; U.S. Provisional Patent Application No. 63/381,081 (Attorney Docket: PMD00085US), to Merino et al., filed October 26, 2022; U.S. Provisional Patent Application No. 63/429452 (Attorney Docket: PMD00093US), to Zhai, filed December 1, 2022; and U.S. Provisional Patent Application No. 63/387056 (Attorney Docket: PMD00099US), to Marino et al. The specific inner diameter (ID) and outer diameter (OD) of the transducer may highly depend on the intended frequency of operation. With an air-backed transducer operating at 9 MHz, for example, the outer diameter may be 1.52 mm (0.06") and inner diameter may be 1.02 mm (0.04").

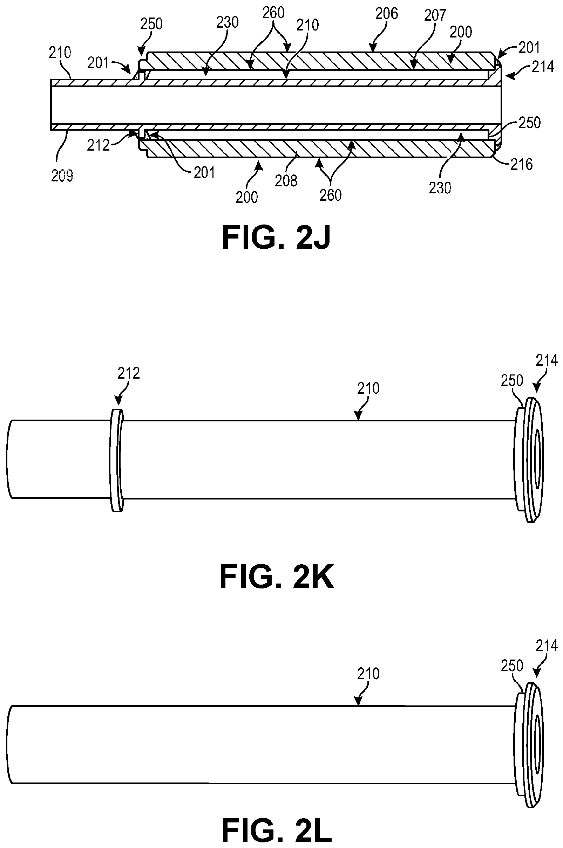

In many embodiments, the ultrasound transducers described herein are air-backed, as shown in FIGS. 2B, 2D, 2F, 2G, 2H, 2IB, 2J-4B. According to some embodiments, a tissue treatment catheter 10 comprising an ultrasound transducer 200 positioned at the distal end of the catheter 10 is provided. The ultrasound transducer 200 may comprise a piezoelectric transducer body 208 that comprises a hollow cylindrical tube of piezoelectric material having an inner surface 207 and an outer surface 206, each of the inner 207 and outer surfaces 206 comprising an electrode, a backing support member 210 may extend through the ultrasound transducer 200, the backing support member 210 may comprise at least a first conductive part 212, e.g., a first stand-off post, at the distal end of the backing support member 210 and at least a conductive part 212, e.g., a second stand-off post, at the proximal end of the backing support member 210 (FIGS. 2B, 2IB). Each stand-off post 212 comprises an inner face 212a and an outer face 212b. There may optionally be a step portion 250 at the proximal end of the ultrasound transducer 200 (FIGS. 2F, 2G). Alternatively, there is a chamfered portion instead of a step portion 250 at the proximal end of the ultrasound transducer 200. Alternatively, there is neither a stepped portion 250 nor a chamfered portion at the proximal end of the ultrasound transducer 200. The ultrasound transducer 200 may optionally be mounted to at least the first and second stand-off posts 212 of the backing support member 210 to define an air chamber 230 adjacent the inner surface 207, the air chamber 230 being insulated to prevent entry of fluid into the air chamber 230 during use, wherein the transducer 200 is configured to deliver sufficient acoustic energy during sonication such as to thermally induce modulation of neural fibers surrounding the blood vessel sufficient to improve a measurable physiological parameter corresponding to a diagnosed condition of the patient, e.g., to create an ablation zone 3 mm to 6 mm wide, e.g., 5 mm wide, and 0.5 mm to 10 mm, e.g., 1 mm to 6 mm, in depth from the lumen of the blood vessel.

The backing support member 210 may have a length of between about 0.250 inches to 0.400 inches (6.35 mm to 10.16 mm), for example, between about 0.300 inches to 0.350 inches (7.62 mm to 8.89 mm). The posts 212 may be made of an electrically conductive material such as stainless steel, for example, Electroless Nickel Immersion Gold (ENIG) plated stainless steel, or non-plated bronze, copper, brass, tungsten, to name a few. It is often difficult to attach solder to stainless steel. Accordingly, where the backing support member 210 is made of stainless steel, an outer surface of the backing support member 210 may be plated with a metal that solder readily attaches to, such as nickel and/or gold, e.g., to provide an ENIG plated stainless steel backing support member 210. Similarly, outer and inner surfaces of the transducer body 208 can be plated with a metal that solder readily attaches to, such as nickel and/or gold, and thus, the outer and inner surfaces of the transducer body 208 can be referred to as plating surfaces 260. The ultrasound transducer 200 may be mounted to at least the first and second stand-off members 212 of the backing support member 210 to define an air chamber 230 adjacent the inner surface 207, the backing support member 210 extending a first length from the outer-face 212b of the stand-off post 212 and the transducer 200 extending a second, shorter length from the outer-face 212b of the stand-off post 212, such as to define an ingress or pocket 270 establishing a circumferential solder seal (FIG. 2G) using a solder material 201. Such a pocket 270 can be created at both the proximal and distal longitudinal ends of the transducer 200 to provide a region for solder to be placed and used to provide an air-tight seal for the air chamber 230. In certain embodiments, solder nuggets or solder balls are used. For example, solder nuggets or solder balls and solder flux (e.g., liquid or cream flux) can be placed in the pocket 270 and a soldering iron is used to apply heat to the solder nuggets or solder balls to cause the solder balls and solder flux to melt and fill the pocket 270. The aforementioned solder nuggets can be made by cutting soldering wire into segments (aka the nuggets) having a thickness of about 0.25 mm to about 0.38 mm (about 0.01 inches to about 0.015 inches). In certain embodiments, once solder is used to fill in the pockets 270 on both the proximal and distal longitudinal ends of the transducer 200, the transducer 200 is placed into an ultrasonic cleaner having about 70% isopropyl alcohol (IPA) for approximately 5 minutes and dried with compressed air. Thereafter, the solder joints are visually inspected, e.g., using a visual aid apparatus. If a solder joint is determined to be unacceptable, more flux is applied, the joint is re-soldered, and the above noted cleaning and inspection steps are repeated until the solder joint is determined to be acceptable, or the transducer being manufactured is rejected.
In certain embodiments, the outer diameter of the stand-off post 212 tightly fits within the inner diameter of the transducer 200, such that solder may only be required at the outer-face 212b of the stand-off post 212 and does not migrate into the air chamber 230, e.g., transducer inner diameter (ID), during use. The solder seal can also prevent adhesive from migrating, e.g., during sonication. Using a solder preform is another option to assist in preventing the solder from infiltrating the air chamber 230. The solder preform may be a washer 203 or washer-like element, for example, such as one with a top-hat shaped side cross-section 202. Such a solder preform can be placed at its proper location on the backing support member 210, e.g., adjacent to a stand-off post 212, and then can be heated to cause the solder preform to reflow and seal any gap the exists between the stand-off post 212 (or other type of stand-off member) and an inner surface of the body of the ultrasound transducer 200 to provide an air tight seal for the air chamber 230. Alternatively, or additionally, a ring of solder spheres that are precoated with solder flux can be used to provide an air-tight seal for the air chamber 230. In certain embodiments, the solder preform comprises a tin-lead alloy, such as a 63/37 tin-lead alloy. Alternatively or additionally, the solder preform comprises a non-lead alloy, such as tin-silver-copper or tin-copper.

For at least the following reasons, it is beneficial to prevent solder and adhesive from leaking into (aka infiltrating, or migrating into) the air chamber 230 of the air-backed transducers described herein. If solder and/or adhesive (e.g., resin) leaks into the air chamber 230, the solder and/or adhesive acts as a backing material, meaning acoustic waves will go through the solder and/or adhesive layer (that leaked into the air-chamber) and bounce back. If the solder and/or adhesive is not applied in a controlled fashion, which would be the case where the solder and/or adhesive leaks into the air chamber, then the acoustic output power will be disrupted in the affected area of the transducer (i.e., in areas where solder and/or adhesive leaked into the air chamber 230). This can cause two undesirable effects, including: 1) along a longitudinal length of the air-backed transducer, it is likely that the area where solder and/or adhesive leaked into the air chamber 230 has a lower acoustic output power because of the non-optimal thickness of the transducer body, which can reduce the effective longitudinal length of the air-backed transducer; and 2) the acoustic output power uniformity would likely be adversely affected by solder and/or adhesive (e.g., resin) that leaks into the air chamber 230 because acoustic output power uniformity is highly dependent on the transducer body being highly cylindrically symmetric (i.e., having a high concentricity).

Additionally or alternatively, the backing support member 210 may comprise a first conductive part 212, e.g., first stand-off post, and a second conductive part 212, e.g., second stand-off post. The first stand-off post 212 and the second stand-off post 212 may be coupled to the piezoelectric component, e.g., ultrasound transducer 200, and together with the piezoelectric component and an area of the backing support member 210 between the first stand-off post 212 and the second stand-off post 212 define the air chamber 230. One or more of the first stand-off post 212 or the second stand-off post 212 may be coupled to the ultrasound transducer 200 by soldering. Additionally or alternatively, the ablation element may further comprise at least one spacing element positioned between the one or more of the first stand-off post 212 or the second stand-off post 212 and the ultrasound transducer 200. The at least one spacing element may be soldered together with the one or more of the first stand-off post or the second stand-off post and the piezoelectric component. The at least one spacing element may comprise at least one washer 203. The at least one spacing element may comprise a solder preform. The transducer may optionally be stepped and/or chamfered at one or more of its longitudinal ends, i.e., at its proximal and/or distal ends. The transducer may optionally be not be stepped or chamfered at one or more of its longitudinal ends.

FIG. 2J illustrates an embodiment addressing the issue of solder migration into the air chamber 230. The backing support member 210 can comprise a cap 214 at the distal end and a stand-off post 212 at the proximal end. There is a step portion 250 on the cap 214 for centering the backing support member 210 with the ultrasound transducer 200. The plating surface 260 can be removed at the chamfered edge 216 of the ultrasound transducer 200. Removing the plating surface 260 can be done, for example, by grinding. Additionally or alternatively, removing the plating surface is done before attaching the backing support member 210 to the ultrasound transducer 200. Solder material 201 can be placed at the interface between the cap 214 and the plating surface 260 of the inner surface 207. Solder material 201 can be placed at the proximal end of the backing support member where the first stand-off post meets the plating surface 260 of the inner surface 207. Easier positioning of the backing support member 210 can be achieved by inserting the backing support member 210 through the distal end of the ultrasound transducer 200.

In some embodiments, the shape of the backing support member 210 or the stand-off post 212 may be modified to inhibit the infiltration of the solder into the air chamber 230. For example, as illustrated in FIG. 2K, the backing support member 210 may be modified such that the distal end comprises a cap 214 with a step portion 250 and the proximal end comprises a stand-off post 212 to prevent solder material 201 step infiltration from the proximal end. Another embodiment is illustrated in FIG. 2L, where the backing support member 210 can comprise a cap 214 with a step portion 250 at the distal end and no stand-off post 212 at the proximal end.

There may be many embodiments at the proximal end of the transducer assembly to prevent solder material 201 from migrating into the air chamber 230. For example, as illustrated in FIG. 2M, plating surface 260 can be removed from the chamfered edge 216 of the ultrasound transducer 200. A backing support member 210 without a stand-off post 212 can be used with a cover 202 to close the space between the backing support member 210 and ultrasound transducer 200. The cover 202 can also be referred to herein as a collar, since it encircles the backing support member 210. Solder material 201 can be placed at the interface between the cover 202 and the plating surface 260 of the ultrasound transducer 200 and at the interface between the cover 202 and the backing support member 210. FIG. 2N illustrates an embodiment of the cover 202. The cover 202 (aka collar) can be made of material such as copper, brass, etc. The cover 202 in FIG. 2N includes a first portion having a first outer diameter that is configured to fit within the space between the backing support member 210 and ultrasound transducer 200, and a second portion having a second outer diameter (that is larger than the first outer diameter of the first portion of the cover 202) that is configured to placed against a longitudinal end of the transducer 200. Collectively, the first and second portions of the cover 202 cause the cover 202 to resemble a sideways top-hat, wherein the transition between the first outer diameter and the second outer diameter is provided by an abrupt step.

Another embodiment to prevent solder material 201 from migrating into the air chamber 230 is illustrated in FIG. 20. A backing support member 210 with a stand-off post 212 at the proximal end can be used with a washer 203. The washer 203 can be located on the outside surface of the stand-off post 212. Solder material 201 can be placed at the interface between the washer 203 and backing support member 210 and at the interface between the washer 203 and plating surface 260 of the ultrasound transducer 200. The plating surface 260 can be optionally removed from the chamfered edge 216 of the ultrasound transducer 200. FIG. 2P illustrates an embodiment of a washer 203. The washer 203 can be made of an electrically conductive material, such as copper, brass, gold plated polymer, etc. For the embodiments illustrated in FIGS. 2J, 2M, and 20, the plating surface 260 is removed to disconnect the outer diameter from the inner diameter of the ultrasound transducer 200, thus preventing a shortage between the inner and outer electrodes (e.g., 204 and 205 in FIGS. 11C and 11D).

The ramped cover 282 (aka ramped collar 282), shown in FIGS. 2Q and 2R, can be used in pace of the cover 202 (aka collar 202) shown in and described above with reference to FIGS. 2M and 2N. The ramped cover 282 includes a first portion 284 having a first outer diameter that is configured to fit within the space between the backing support member 210 and ultrasound transducer 200, and a ramped portion 288 having tapered or ramped outer diameter that starts out larger than the first outer diameter of the first portion 284) and eventually tapers to second potion 286 having the same outer diameter as the first portion 284, which enables the ramped cover 282 to be slid over the outer diameter of the backing support member 210 and interference fit between the outer circumferential surface of the backing support member 210 and the inner circumferential surface of the transducer 200. In a similar manner that the solder 201 is applied in the embodiment of FIGS. 2M, solder material 201 can be placed at the interface between the ramped cover 282 and the backing support member 210. In the embodiment shown in FIG. 2R, instances of the ramped cover 282 are included at both the proximal and distal ends of the transducer 200 to provide for an air chamber 230 to thereby provide for an air-backed transducer 200. The air chamber 230 can contain a low acoustic impedance medium, including, but not limited to air, helium, argon, or nitrogen. The air chamber can alternatively be evacuated, i.e., be a vacuum. Because the air chamber 230 can include other gasses besides air, or can be evacuated, the air chamber 230 may equivalently be referred to as a gas or vacuum chamber 230. For similar reasons, the air-backed transducer 200 may be equivalently referred to as a gas or vacuum backed transducer 200.

In an embodiment, the stand-off post 212 may be angled to prevent capillary action of the solder into the air chamber 230. This may advantageously allow the transducer 200 to withstand higher powers than prior air-backed transducers. The stand-off post 212 may not be necessary in an air-backed transducer design. In another embodiment, an interference fit cover (aka collar) can be used to seal the air chamber 230 at both ends of the ultrasound transducer 200 and a rod can be placed through the inner diameter of the ultrasound transducer. In other words, so long as the air chamber 230 is hermetically sealed, the design for sealing can be unlimited. An advantage of not needing the stand-off post 212 in the ultrasound transducer design is that it broadens design choices for hermetically sealing the air chamber 230.

Alternatively of additionally, a small amount of electrically conductive solder 201 may infiltrate the air chamber 230 in order to provide an electrical connection between the inner diameter of the transducer 200 and the backing support member 210, as illustrated in Fig. 2S. A solder preform 203 can be placed at its proper location on the backing support member 210, e.g., adjacent to both stand-off posts 212, and then can be heated to cause the solder preform to reflow, solder may flow into air chamber 230 sufficient to electrically connect the inner diameter of the transducer 200 and the backing support member 210 and to form a seal between the stand-off post 212 (or other type of stand-off member) and an inner surface of the body of the ultrasound transducer 200 to provide an air tight seal for the air chamber 230.

In certain embodiments, the transducer 200 may withstand the levels of power required to ablate target tissue, e.g., renal nerves, for example, up to about 150 W/cm² or more across the outer surface area of the transducer 200, such that the transducer 200 is configured to deliver sufficient acoustic energy during sonication such as to thermally induce modulation of neural fibers surrounding the blood vessel, the thermally induced modulation being sufficient to improve a measurable physiological parameter corresponding to a diagnosed condition of the patient, while being sufficiently small to fit in a renal artery and/or permit radial access using a 5F or smaller catheter.

In some embodiments, the transducer 200 comprises a step portion 250 on at least the proximal end where electrical conductors 20 may connect to the outer electrode of the transducer. Connection of the electrical conductors 20 and solder material 201 being located at the step portion 250 of the transducer 200 may also provide for an air-backed transducer design that does not interfere with the acoustic output of the transducer 200 and permits transducer 200 to attach to the cable and still maintain a smaller profile.

Referring to FIGS. 2A, 2C, 2E, and 2IA, water or liquid-backed transducers and components thereof are shown. Such water-backed transducers may have components similar to the air-backed transducers. Water or liquid transducers have a water or liquid layer at the back or region next to the inner surface of the PZT member component. In many cases, water or liquid transducers have an additional solid backing or supporting member 211 going through the water or liquid layer. The geometries are carefully designed, so that the water or liquid layer in combination with the solid backing member may induce constructive acoustic interference at the outer surface to maximize the acoustic transmission efficiency. In such cases, the distance between the inner diameter of the transducer or piezoelectric component and the outer diameter of the solid backing member (i.e., the height of the liquid or water layer) may be carefully controlled and manufactured to introduce constructive interference between the ultrasound waves directed outward from the outer diameter of the transducer or piezoelectric component and the ultrasound waves reflected off the solid backing member. By having an air layer instead, the material behind the rear surface has no or little impact on the acoustic energy transmitted from the front surface of the transducer. All energy is reflected back from the rear surface and transmitted from the front surface thus the transmission efficiency is improved. The so called "air layer" includes a low acoustic impedance medium, such as, but not limited to air, helium, argon, carbon dioxide, and/or nitrogen. The so called "air layer" can alternatively be evacuated. Accordingly, the "air layer" may equivalently be referred to as a vacuum or gas layer.

In water or liquid backed embodiments of the ultrasound transducer 200, a backing member 211 may extend through the ultrasound transducer 200, the backing member 211 may comprise a plurality of posts 213 at the distal end of the backing member 211 and a plurality of posts 213 at a proximal end of the backing member 211 (FIGS. 2A, 2C, 2AI). These posts 213 leave openings between adjacent posts such that liquid or water may still pass therebetween. The ultrasound transducer 200 may be mounted to the posts 213 to define the water or liquid backing layer 231, which is open to fluid communication in and out thereof, such that water or liquid may be circulated therethrough, for example, to cool the transducer. The transducer 200 may be configured to deliver sufficient acoustic energy during sonication such as to thermally induce modulation of neural fibers surrounding the blood vessel sufficient to improve a measurable physiological parameter corresponding to a diagnosed condition of the patient.

Ultrasound ablation uses the attenuation phenomena to generate heat. As ultrasound energy or waves propagate through soft tissue, the acoustic energy decreases while the lost acoustic energy is converted into heat. In an embodiment suitable for renal denervation, a frequency of 10 to 15 MHz may be used. More generally, in embodiments suitable for renal denervation, a frequency within the range of 1 MHz to 30 MHz may be used, which is optionally within the range of 7 MHz to 15 MHz, is optionally within the range of 8 MHz to 13 MHz, and optionally within the range of 8.5 MHz to 9.5 MHz, or 8.7 MHz to 9.3 MHz.

In an embodiment suitable for renal denervation, an air-backed transducer wall can be thinner, e.g., 0.15 mm- 0.2 mm, e.g., 0.17 mm, and have an operating frequency of between 12-15 MHz, e.g., 13 MHz. The OD of the air-backed transducer may be minimized for radial access (e.g., about 1.3 mm), while the ID is maximized to for heat dissipation (e.g., about 1mm) to deliver sufficient acoustic energy during sonication such as to thermally induce modulation of renal nerves surrounding a renal blood vessel sufficient to improve a measurable physiological parameter, e.g., blood pressure, corresponding to a diagnosed condition of the patient, e.g., hypertension.

A person of skill in the art after reading the present application will understand that the geometry of the transducer can be optimized for other applications, e.g., cardiac ablation, or other tubular anatomies.

In certain embodiments, the transducer 200 comprises a cylindrical hollow tube of piezoelectric material, which can be referred to as a piezoelectric transducer body 208. In other embodiments, the piezoelectric transducer body can have various other shapes and need not be hollow. In certain embodiments suitable, e.g., for renal denervation, the piezoelectric material may be lead zirconate titanate 8 (PZT8), which is also known as Navy III Piezo Material. Raw PZT transducers may be plated with layers of copper, nickel and/or gold to create electrodes on surfaces (e.g., the inner and outer surfaces) of the piezoelectric transducer body 208, as disclosed, e.g., in U.S. Patent No. 10,230,041 to Taylor et al**.** In certain embodiments, the PZT may be annealed or hot isostatic pressed (HIPped), as known in the art. More specifically, in certain embodiments, the piezoelectric transducer body 208 is made by hot isostatic pressing a Navy Type III high density lead zirconate titanate (PZT) piezoelectric material.. A combination of high temperatures and high pressures may be used to densify the PZT prior to machining of the PZT. This process can remove porosity in the PZT material, thereby leading to improved dielectric breakdown strength of the transducer. More specifically, by forming the HIPed piezoelectric transducer body 208 in this manner, and defining the air chamber 230 to provide for an air-backed ultrasound transducer, such an ultrasound transducer 200 is able to output sufficiently greater output power than a water-backed ultrasound transducer, as can be appreciated in part from the graphs in FIGS. 7A-7D, described below.

The water-backed design in FIG. 3A, illustrates the transducer 200 comprises a step portion 250 on at least the proximal end where electrical conductors 20 may connect to the outer electrode of the transducer. FIGS. 3B and 3C illustrate that since the inner diameter (ID) of the transducer 200 in an air-backed design is isolated from fluid (i.e., fluid within a balloon 14 or body fluid, e.g., blood, in a balloonless embodiment), the catheter can be saline compatible. The adhesive 303 and 304 that can be used to keep the seal intact may comprise a bond that adheres to the backing support member material which may be made of a metal. The adhesive may be durable because vibrations can cause the adhesive to delaminate. An example of an applicable adhesive can be epoxy.

In certain embodiments, a proximal end of the ultrasound transducer 200 can include a stepped portion 250, while distal end of the transducer 200 may or may not include a similar stepped portion, depending upon the specific implementation. In certain embodiments, the axial length of stepped portion 250 of the transducer 200 is about 0.4 mm and the axial length of a non-stepped portion of the transducer 200 is about 6 mm. Other variations are also possible and within the scope of the embodiments described herein. In certain embodiments, the non-stepped portion of the transducer 200 is the portion of the transducer that produces the bulk of the ultrasonic energy delivered from the catheter 10. In certain embodiments, the stepped portion 250 has a different outer-diameter than the non-stepped portion and behaves in a different manner than the non-stepped portion.

In accordance with certain embodiments, in order to improve the durability of an air-backed transducer 200, a pair of stand-off members 212 that are used to provide the air-chamber 230 are attached (by solder and/or adhesive) to proximal and distal portions of the piezoelectric transducer body 208 that are configured to not vibrate in response to application of a voltage between the inner and outer electrodes 204, 205 of the air-backed transducer 200. Between the proximal and distal portions of the piezoelectric transducer body 208 (that are configured to not vibrate in response to application of the voltage between the inner and outer electrodes 204, 205) is a center portion of the piezoelectric transducer body 208 that is configured to vibrate in response to application of the voltage between the inner and outer electrodes 204, 205. Solder and/or adhesive is included between the piezoelectric transducer body 208 and the pair of stand-off members 212 to provide air-tight seals that prevent any fluid (that the piezoelectric transducer body 208 can be located within) from leaking into the air chamber 230. By having the stand-off members 212 attached, respectively, to the proximal and distal portions of the piezoelectric transducer body that are configured to not vibrate in response to application of the voltage between the inner and outer electrodes, this reduces a probability that the air-tight seals provided by the solder and/or adhesive will fail and allow fluid to leak into the air chamber 230, compared to if at least one of the stand-off members 230 were instead attached to the central portion of the piezoelectric transducer body that is configured to vibrate in response to application of the voltage between the inner and outer electrodes.

The generator 22 may be electrically connected to the transducer 200 by coaxial cable(s), parellel pair with single/stranded conductors, shielded parallel pair, twisted pair, or a flex circuit.

In certain embodiments, two separate coaxial cables are electrically connected to a proximal portion of the ultrasound transducer 200. The two separate coaxial cables can collectively comprise electrical cabling. Each such coaxial cable includes an inner conductor surrounded by a dielectric, which is surrounded by an outer tubular conducting shield, all of which are surrounded by an insulating jacket. In certain embodiments, the distal ends of the inner conductors of the coaxial cables can be electrically coupled to the outer electrode (e.g., 205 in FIGS. 11C and 11D) on the proximal end of the transducer 200 approximately 180 degrees apart. More specifically, each of the inner conductors of the coaxial cables may be soldered on the stepped portion 250 on the proximal end of the transducer 200 about 180 degrees apart, such that current output from the electronics 22 flows from the inner conductors of the coaxial cables to the outer electrode 205 of the transducer 200. The proximal ends of the inner conductors of the coaxial cables can be electrically coupled to one another by soldering, or the like. When located within a cable lumen of the catheter shaft 12, the pair of coaxial cables will extend through the cable lumen parallel to one another.

Each of the outer conductors of the coaxial cable may comprise multiple wires that are bundled together and soldered to the backing support member 218, so as to electrically couple the inner electrode (e.g., 204 in FIGS. 11C and 11D) of the transducer 200 to the coaxial cables via the backing support member 210 (in embodiments where the backing member is electrically conductive and electrically coupled to the inner electrode 204). The proximal ends of the outer tubular conducting shields of the coaxial cables can be similarly bundled together and/or soldered together.

Wires of the electrical cabling that are soldered to the proximal end of the transducer 200, and more specifically, to the inner and outer electrodes 204, 205 of the transducer 200, or even more specifically to the stepped portion 250 and to the backing support member 210, may include sharp edges that could potentially damage a balloon 14 within which the transducer 200 is located. In balloonless embodiments, such wires could potentially damage a body lumen within which a distal portion of the catheter 10 and the transducer 200 thereof are positioned.

Referring to FIG. 3D, in accordance with certain embodiments, a dielectric tube 290 is placed over a distal end of the wires of the electrical cabling that is soldered to the proximal end of the transducer 200 (e.g., to the stepped portion 250 thereof) and over the distal end of the backing support member 210, so as to cover the sharp edges of the wires. Such a dielectric tube 290 can be made of polyimide, but is not limited thereto. A liquid resin 291 or other type of adhesive with a high dielectric breakdown threshold can be poured into and fill at least a portion of the dielectric tube and then either UV cured or heat cured to function as a potting compound for the wires. Similarly, a further dielectric tube 292 can be placed over a distal portion of the transducer 200 and a distal portion of the backing support member 210, and secured using a UV cured or heat cured liquid resin 293 or other type of adhesive with a high dielectric breakdown threshold. The dielectric strength of the dielectric tubes 290, 292 and the resin or other type of adhesive 291, 293 should be at least 1.57 kV/mm (40 volts per mil, where a mil is 1/1000 of an inch), and can be within the range of 1.57 to 15.75 kV/mm (40 to 400 volts per mil), is not limited thereto. In certain embodiments, an adhesive lined heat shrink tube can be used to provide the dielectric tube 290, 292 and the resin or other type of adhesive 291, 293.

In certain embodiments, rather than including a stepped portion 250 on the proximal end of the transducer 200, a flat section is instead located on the proximal end of the transducer 200, wherein the flat section is used in place of the stepped portion 250 as being the location where the inner conductors of the coaxial cables are soldered to the transducer 200. The solder would make the flat section of the transducer 200 a dead section. In certain embodiments, the axial length of flat section of the transducer 200 is about 0.4 mm and the axial length of a non-flat portion of the transducer 200 is about 6 mm.

Air-backed transducers have a structure which provides an air layer at the inner surface of the active element. The interface between the air and the element is highly reflective, because air has an acoustic impedance far lower than that of the ceramic. This interface can serve as a backing interface and can help to direct acoustic vibrations through the outer surface of the tubular element, which serves as the front or emitting surface of the transducer.

Air-backed transducers can provide good efficiency and can be compact. However, the emitting power of such a transducer may be limited by thermal considerations. Air and other gasses can provide only a limited cooling effect at the inner surface of the active element. The power of the applied drive signal may be limited to avoid overheating the transducer. This problem may be particularly severe in the case of small transducers for applications such as ablation. In some embodiments, the fluid within the balloon surrounding the transducer contributes to transducer cooling and temperature regulation. In some embodiments, the transducer may be cooled and/or thermally regulated by direct contact with the blood (in balloon-less embodiments).

According to some embodiments, one challenge in designing transducers that deliver significant power (approximately 10 acoustic watts per cm² at the transducer surface, or greater) is preventing the degradation of adhesives and other heat/vibration sensitive materials in proximity to the transducer. If degradation occurs, materials under or over the transducer can delaminate and cause voids that may negatively affect the acoustic coupling and impedance of the transducer. In cases where air backing of the transducer is used, material degradation can lead to fluid infiltration into the air space that will compromise transducer performance.

Using adhesives/epoxies in the current design offers no control of the amount of adhesive dispensed on the unit. This could lead to seepage of the adhesive in the rear section of the transducer, which could result in over heating of the transducer and degradation in transducer performance. Embodiments herein also provide for efficiencies in manufacturability. Using adhesives for air backing may be difficult to control or prevent from degradation. The embodiments disclosed herein provide an improved method of sealing the transducer to prevent electrical shorting.

### Enhanced uniformity

With air backing, the uniformity of the device relies highly on the concentricity of the piezoelectric transducer and not on transducer-backing support member interaction. So, the ablations can be more uniform. The dependence of device performance on the transducer assembly process can be greatly reduced. Mean uniformity has improved indicating a more uniform circumferential delivery of energy is provided.

### Enhanced efficiency

Air in the rear of the transducer can ensure that the acoustic waves are launched primarily from the outer diameter (OD) of the transducer.

### Saline Compatibility

Since the inner diameter (ID) of the transducer is isolated from the cooling fluid, more options for saline compatibility may be available.

The adhesive seal can be another barrier layer for the air backing if the soldering process is incomplete, preventing liquid entry into the ID space. Since ID is now isolated completely, selective insulation may be provided which can enable saline compatibility. Parylene coating all of or substantially all of the exposed surface with or without the adhesive seal can also enable saline compatibility. The sealing may be provided to withstand a voltage of >80 VDC per minute

### Compactness

The transducer OD profile can be reduced to enable 4 F/ 5 F catheter implementations. As used herein, a 4 F catheter is a catheter compatible with a 4 F guide catheter and a 5 F catheter is a catheter that is compatible with a 5 F guide catheter. In contrast to liquid or water-backed transducers, air-backed transducers may not need to be manufactured with strict tolerances between the transducer and backing support member, for instance, a quarter wavelength design. Improvement to durability might be possible with annealing post machining to reduce residual stress.

Being air-backed and electrically insulated may allow the OD of the device to be reduced to accommodate a smaller catheter size. An array of transducers may be provided with various thicknesses and/or operating frequencies on the same backing support member. For example, in a 4F/5 F device, the ID and OD of the transducer can be shrunk because of air backing. There can also be asymmetry, for example, different OD rings for the backing support member (e.g., the distal end may be smaller than proximal end for mounting ease and less scrapping of plating on manufacturing and/or use).

FIG. 4A illustrates a two-part backing support member 210, with backing support member elements positioned largely distal and proximal to the transducer 200, in accordance with some embodiments. Each backing support member element may include a stand-off post 212 to couple to the transducer 200. The main body of the transducer 200 may be disposed in-between the proximal and distal stand-off posts 212 and over the air chamber 230 and over the catheter shaft 12 and/or a hypotube. A two-part backing member 210 may allow the backing support member 210 to be configured about the transducer 200 without scraping the inner diameter (ID) of the transducer 200.

FIG. 4B illustrates a continuous backing support member 210, in accordance with some embodiments. Alternatively or in combination with having a step portion 250 as shown in FIG. 4B, the transducer 200 can be chamfered at the ends of the transducer 200. A chamfered end can be another way of providing a non-vibrating section of the transducer 200. The chamfered ends of the transducer 200 can be non-vibrating while the rest of the transducer 200 is vibrating freely. With this embodiment, there may be better symmetry of the acoustic energy distribution as illustrated in FIG. 10B.

The transducers 200 in FIGS. 4A and 4B have a dual step design wherein, at the proximal and distal portion of the transducer 200, there is a step portion 250. Symmetry can be conferred within the acoustic energy distribution by creating a transducer with a symmetric geometry. Having a dual step design can constrain the transducer 200 at the step portion 250 from vibrating, thus allowing the main body of the transducer 200 to vibrate. Coatings or adhesives bonded to the non-vibrating step portion 250 of the transducer 200 can provide improved isolation. In some embodiments, the transducer 200 can have either one step, two steps, or no step. In some embodiments, there can also be a slot 252 for alignment of the transducer 200. Additionally or alternatively, the acoustic performance of the ultrasound transducer 200 can be retained even if it is constrained in the distal/non-step end by approximately 0.25 mm (0.01").

In some embodiments, more than one transducer assembly can be positioned in one or more balloons on a catheter.

Electrical conductors may be connected to the transducer assembly. A first electrical conductor may connect to the backing support member 210. A second electrical conductor may connect to the outer electrode of the most proximal transducer assembly and may connect the outer electrode to a generator through a cabling system that runs through the shaft of the catheter. A third electrical conductor may extend from the outer electrode of the distal transducer assembly through an opening into the inner lumen of the backing support member 210 into the shaft of the catheter. Electrical conductors may connect the outer electrode of the transducer assembly to a generator through a cabling system that runs through the shaft of the catheter.

During operation of the transducer assemblies, the backing support member 210 can serve as a common return for the electrical energy applied to the one or more transducer assemblies. As a result, the electronics can independently operate multiple transducer assemblies by applying a voltage between the outer electrode of a selected transducer assembly and the backing support member 210. Alternate arrangements of the electrical conductors can be used to permit independent operation of all or different selections the transducer assemblies.

The electrical conductors may be wires though other electrical conductors can be employed. For instance, electrically conductive adhesives can serve as one or more of the electrical conductors. An electrically conductive adhesive can be used in conjunction with a substrate. For instance, the electrically conductive adhesive can have one or more features selected from a group consisting of being on one side of the substrate, both sides of the substrate, supported by the substrate, or positioned with the substrate as by absorption or other mechanism such as impregnation. In one example, the electrically conductive adhesive may be included in a layer on a tape that replaces the third electrical conductor. Suitable electrically conductive adhesives include, but are not limited to, conductive epoxies, adhesive metallic films, and blends that include epoxy and acrylates impregnated with silver-coated glass beads. Suitable substrates include, but are not limited to, plastics such as Polyethylene Terephthalate (PET).

At least a portion of a bridge portion of the backing support member extending between adjacent transducer assemblies can include one or more enhanced flexibility regions. The one or more enhanced flexibility regions can be selected to enhance the flexibility of the backing support member 210 and accordingly the catheter. For instance, the portion of the backing support member 210 with an enhanced flexibility region can be more flexible than one or more portions of the backing support member 210 without an enhanced flexibility region and/or more.

Suitable enhanced flexibility regions include, but are not limited to, openings through the wall of the backing member 210 arranged in patterns, the backing member 210 cut into lattice patterns, etc.

The opening(s) may spiral around the longitudinal axis of the backing support member 210 for the portion of the backing support member 210 located between adjacent transducer assemblies. As a result, at least one flexibility enhanced portion of the backing member 210 can have a helical or substantially helical configuration for a portion of the longitudinal length of the backing support member 210. In some instances, the enhanced flexibility region does not extend into any of the transducer assemblies in the balloon.

The spiral rate can measure the number of degrees that the helical configuration turns around the longitudinal axis of the backing support member per length of the longitudinal axis. The spiral rate can determine the degree of flexibility of the flexibility enhanced portion of the backing support member 210. For instance, increasing the spiral rate can provide a more flexible backing support member 210 while decreasing the spiral rate can provide a more rigid backing support member 210. Suitable spiral rates (pitch counts) include, but are not limited to, rates greater than or equal to 0°/mm and can extend over more than 360° or more than 720°.

Multiple transducer assemblies within a single balloon can be used to increase the flexibility of the catheter. The increased flexibility may provide access to smaller diameter body lumens, such as accessory renal arteries and renal arterial branches (e.g., blood vessels having a diameter that is less than 3 mm), may help maneuver the catheter through tortuous anatomies, and/or may reduce de-centering of a transducer assembly due to placement of the transducer assembly at a curve in a body lumen. When it becomes more desirable to treat smaller and/or more tortuous spaces, the transducer assemblies disclosed above can be broken into multiple smaller transducer assemblies.

In some embodiments, electrical connections may be arranged to provide the electronics the ability to operate the transducer assemblies independently. In some embodiments, the electrical connections can be arranged for concurrent operation of the transducer assemblies as well. For instance, the transducer assemblies can be connected in parallel or in series.

In some embodiments, two transducer assemblies are provided, though a catheter may include more transducer assemblies, e.g., without limitation, three, four, five, six, or more, all connected in series by electrical conductors that connect the outer electrodes of each pair of transducer assemblies to each other and then finally connect the proximal most outer electrode of the proximal most transducer assembly to the generator via a cabling system that runs through the catheter shaft to the generator.

Referring to FIGS. 5A and 5B, catheter systems of the present disclosure may be used to deliver energy with an air-backed transducer in a uniform and circumferential manner. For example, multiple toroidal ablation zones or lesions 501 may be generated around the renal arteries, accessory arteries, or proximal side branches of a patient as shown in FIG. 5A.

In certain embodiments described herein, a method of ablating a target tissue is provided. A catheter 10 comprising an ablation element may be advanced through at least one bodily vessel to a position at or near the target tissue. The ablation element may comprise a piezoelectric component, e.g., ultrasound transducer 200, a backing support member 210, and an air chamber 230 therebetween. The piezoelectric component of the ablation element may be energized to deliver energy to the target tissue, thereby ablating the target tissue. The target tissue may comprise one or more nerves or nerve branches. The catheter 10 may be advanced through at least a radial artery. In some embodiments, the target tissue comprises one or more renal nerves or renal nerve branches, and the catheter 10 is advanced into a one or more renal arteries from at least the radial artery. The catheter 10 may have a size of 5 Fr or less. The catheter 10 may be advanced through an introducer having a size of 5 Fr or less to access the bodily vessel. The catheter 10 may be sized to be advanceable through at least a radial artery. The target tissue may comprise one or more renal sympathetic nerves or renal sympathetic nerve branches, and the catheter 10 is sized to be advanceable into a one or more renal arteries from the radial artery. The catheter 10 may have a size of 5 Fr or less. The catheter 10 may be configured to be advanced through an introducer having a size of 5 Fr or less to access the bodily vessel.

In an embodiment, the piezoelectric component, e.g., ultrasound transducer 200, may be energized for a time period of between 5 to 20 seconds, at a frequency of 11 to 15 MHz, or both. In an embodiment, the piezoelectric component may be energized for a time period of between 6 to 10 seconds, at a frequency of 12 to 14 MHz, or both. In an embodiment, the piezoelectric component may be energized for a time period of about 7 seconds, at a frequency of about 13 MHz, or both. In an embodiment, energizing the piezoelectric component may increase a temperature of the piezoelectric component by no more than 50° C. In an embodiment, energizing the piezoelectric component may deliver energy at an average surface acoustic intensity of between 20 and 150 W/cm².

### EXPERIMENTS AND TESTING

Referring to FIGS. 6A and 6B, tests were conducted to evaluate the uniformity of the ablation zones or lesions generated with air-backed ultrasound transducers versus water-backed ultrasound transducers. FIG. 6A shows a graph of the uniformity ratios or UR (maximum energy emission over minimum energy emission over the energy emitting surface of the transducer) for various ablation zones or lesions generated with the air-backed transducers and water-backed transducers. As shown in the FIG. 6A graph, mean uniformity is improved with air-backed transducers, indicating a more uniform, circumferential delivery of energy. Mean uniformity ratio for all 147 transducers tested was 0.757615, mean uniformity ratio for air-backed transducers was 0.797581 for the 47 air-backed transducers tested, and mean uniformity ratio for water-backed transducers was 0.737842 for the 95 water-backed transducers tested. Further analysis data is provided by the table of FIG. 6B. As a greater percentage of air-backed ultrasound transducers manufactured would have higher energy delivery uniformity, manufacturing yield for ultrasound transducers delivering energy at least at a particular uniformity issue (for example, 0.74) can be increased.

Referring to FIGS. 7A-7D, breakdown tests were performed for various air-backed transducers and water-backed transducers. Electrical power was provided from 10 to 100 W for the transducers and acoustic power (in W) was measured. Acoustic power smoothly increases with increases in electrical power until the transducer breaks down, at which point acoustic power sharply dives and energy conversion efficiency is lost. As shown in FIG. 7A, eleven water-backed transducers were tested and the first instance of break-down occur at around 45 W of electrical power, with further instances of break-down between 50-85 W and only a single transducer not breaking down at 90 W. As shown in FIG. 7B, eleven air-backed transducers were tested and break-down didn't start to occur until around 60 W for these, with further instances of break-down between 70-90 W, including three instances of break-down between 80-90 W and five instances of no break-down at 90W. These results indicate that air-backed transducers are more durable than water-backed transducers. FIGS. 7C and 7D show combined graphs of the data from the break-down tests of both transducer types.

Power density, in Watts per centimeter square (W/cm²), can be calculated using the question: Power Density (W/cm^2) = Acoustic Power(W)/(2*pi*r*h), where r is the radius of the transducer, and h is the active length of the transducer. Where radius of transducer is 0.0762 cm and active length of transducer is 0.575 cm, for those air-backed transducers that have no break-down at 90 W (i.e., do not break down at an acoustic power of 90 W), they are able to provide a power density of about 327 W/cm² since Power Density (W/cm²) = Acoustic Power(W)/(2*pi*r*h) = 90 W / 2*pi*0.0762 cm*0.575 cm = 372 W/cm².

FIG. 8A is an illustration of the nerve distribution around a renal artery. Most of the nerves are within 6 mm of the inner wall of the renal artery. Hence, it may be desirable and more energy efficient to direct most ablation energy within this 6 mm boundary. FIG. 8B shows a comparison of the cross-sections of an ultrasound transducer operating at 9 MHz, a smaller and thinner ultrasound transducer operating at 15 MHz, and a smaller ultrasound transducer configured for delivery through a 5 Fr or smaller introducer as described herein. FIG. 8C shows an image of the ablation zones or lesions 801 made on test chicken breasts tissue with the 9 MHz transducer. FIG. 8D shows an image of the ablation zones or lesions 802 made on test chicken breasts tissue with the 15 MHz transducer. With a high operating frequency, the ablation zones or lesions generated can be more uniform and better controlled. Further, as shown in FIG. 8E, at 15 MHz, more of the energy delivered is delivered at a distance of 6 mm or less (65% versus 46% for 9 MHz), improving energy targeting specificity and safety.

FIGS. 9A and 9B illustrate a comparison between high frequency transducers. FIG. 9A shows a comparison of ultrasound transducers set to operate at 9 MHz, 12 MHz, and 15 Mhz. At 12 MHz, less than 43% of residual energy is delivered beyond 6 mm. At 15 MHz, less than 35% of residual energy is delivered beyond 6 mm. FIG. 9B is a graph of relative heating power with distance from the transducer surface in a theoretical tissue model. From 0-2 mm, tissue may be cooled by the inflated balloon that surrounds the transducer (i.e., a "cooling zone"). From 2-6 mm, lesions may be generated by the delivered energy (i.e., a "lesions zone"). As shown by FIG. 9B, comparing the heating power profiles of transducers operating at 12 MHz versus 15 MHz, the 12 MHz transducer has greater heating power less at distances of less than about 6 mm and lesser heating power at distances of greater than about 6 mm.

FIGS. 10A and 10B illustrate how acoustic energy intensity can appear in three-dimensional space. FIGS. 10A and 10B show the normalized acoustic energy across the axial length of a transducer, as measured from an axial center of the transducer. FIG. 10A illustrates asymmetry of the lobes (e.g., acoustic energy distribution) without a dual step design, in accordance with an embodiment. Instead, the transducer 200 has a step portion 250 only at the proximal end of the transducer 200. FIG. 10B illustrates symmetry of the lobes with a dual step design, in accordance with an embodiment. The step portion 250 is located at the proximal and distal end of the transducer 200 causing the transducer 200 to be more symmetric. This symmetry of the transducer 200 provides symmetry of acoustic energy distribution. Alternatively, rather than including a stepped portion 250 on the proximal and distal end of the transducer 200, the inner conductors of the coaxial cables are soldered to the transducer 200 at an inactive region of the transducer created by an air-backing soldering seal. This soldering seal may be located in a nonvibrating portion of the transducer. The soldering seal would make the section a dead section but because it located in a nonvibrating portion of the transducer it does not significantly affect the acoustic output and the symmetry of the lobes is similar in this design as with a dual step design depicted in Fig. 10B, without adding to the length of the transducer. In certain embodiments, the axial length of soldering seal section of the transducer 200 is about 0.4 mm or less and the axial length of a non- soldering seal section portion of the transducer 200 is about 6 mm or less.

When acoustic energy distribution is more symmetrical, there can be more uniform and circumferential delivery of acoustic energy at the ablation site.

FIGS. 11A is a side view of a distal portion of a catheter (e.g., 10) of an ultrasound-based tissue treatment system including an air-backed transducer 200 in accordance with certain embodiments of the present technology. The air-backed transducer 200 can be referred to more succinctly as a transducer 200. FIG. 11B is a perspective view of the transducer 200 in accordance with certain embodiments of the present technology. FIGS. 11C and 11D illustrate, respectively, a longitudinal cross-sectional view and a radial cross-sectional view of the transducer 200.

In the embodiments of FIGS. 11A-11D, the piezoelectric transducer body 208 comprises a hollow tube of piezoelectric material having an inner surface and an outer surface, with an inner electrode 204 disposed on the inner surface of the hollow tube of piezoelectric material, and an outer electrode 205 disposed on the outer surface of the hollow tube of piezoelectric material. In such embodiments, the hollow tube of piezoelectric material is an example of the piezoelectric transducer body 208. In FIGS. 11A-11D, the hollow tube of piezoelectric material, or more generally the piezoelectric transducer body 208, is cylindrically shaped and has a circular radial cross-section, as can be appreciated from FIG. 11D. However, in alternative embodiments the hollow tube of piezoelectric material can have other shapes besides being cylindrical with a circular radial cross-section. Other cross-sectional shapes for the hollow tube of piezoelectric material, and more generally the piezoelectric transducer body 208, include, but are not limited to, an oval or elliptical cross-section, a square or rectangular cross-section, pentagonal cross-section, a hexagonal cross-section, a heptagonal cross-section, an octagonal cross-section, and/or the like.

The hollow tube of piezoelectric material, and more generally the piezoelectric transducer body 208, can be made from various different types of piezoelectric material, such as, but not limited to, lead zirconate titanate (PZT), polyvinylidene fluoride (PVDF), or other presently available or future developed piezoelectric ceramic materials. As depicted in FIGS. 11A-11C, the transducer 200 may include a stepped portion 250, as described in U.S. Patent No. 10,454,605. In certain embodiments, the stepped portion 250 on the proximal end of the transducer 200 allows for attachment of the electrical conductors 20 (of electrical cabling) that delivers energy to the transducer 200. In certain embodiments, such electrical cabling comprises parallel coaxial cables having a combined impedance of about 50 ohms. Such a stepped portion 250 can be incorporated into any of the transducers described herein. It would also be possible for both the proximal and distal ends of a transducer to include a stepped portion (the same as or similar to 250), which embodiments can be referred to as dual stepped embodiments.

Moreover, as illustrated in FIG. 11A, the backing support member 210 may have an isolation tube 219 disposed along its interior surface so as to prevent or reduce the likelihood of electrical conduction between a guidewire and the backing support member 210, for use in embodiments where such an electrical conduction is not desired. The isolation tube 219 can be formed of a non-electrically conductive material (e.g., a polymer, such as polyimide), which can also be referred to as an electrical insulator. The isolation tube 219 may extend from the catheter shaft 12 through the lumen of the backing support member 210 within the transducer 200 to a tip 215.

Referring to FIG. 11C, in accordance with certain embodiments, which are suitable for a renal denervation procedure, an outer diameter (OD) of the piezoelectric transducer body 208 is within the range of about 1.3 mm to 1.7 mm, and an inner diameter (ID) of the piezoelectric transducer body 208 is within the range of about 0.8 mm to 1.2 mm. In specific embodiments, the OD is about 1.5 mm and the ID is about 1 mm. In accordance with certain embodiments, a wall thickness (W-TH) of the piezoelectric material of the piezoelectric transducer body 208, between its inner diameter (ID) and its outer diameter (OD), is within the range of 0.2 mm and 1.0 mm. More specifically, the wall thickness (W-TH) can be in the range of 0.2 mm and 0.5 mm. Even more specifically, the wall thickness (W-TH) can be in the range of 0.24 mm and 0.26 mm (and even more specifically, can be 0.25 mm +/- 0.01 mm), which can provide for an ultrasound transducer that produces acoustical energy have a frequency of approximately 9 MHz. In certain embodiments, the ultrasound transducers (e.g., 200, 300, etc.) described herein are configured to deliver acoustic energy in the frequency range of 8.5 to 9.5 MHz. In certain embodiments, such transducers are configured to deliver acoustic energy in the frequency range of 8.7-9.3 MHz or 8.695-9.304 MHz. Transducers delivering acoustic energy in the frequency range of 8.7-9.3 MHz have been shown to produce ablation up to mean depths of 6 mm. The piezoelectric transducer body 208 and the inner and outer electrodes 204, 205 may be formed using any suitable method, such as the methods described in U.S. Patent No. 10,140,041 to Taylor. The above described dimensions and thicknesses, while described with reference to the embodiments shown in FIGS. 11A-11D, also apply to the other embodiments described herein, including the embodiments described below.

The piezoelectric transducer body 208 is configured to generate ultrasonic waves in response to a voltage being applied between the inner and outer electrodes 204, 205. Since the inner electrode is within the air chamber 230, a short circuit is inhibited from occurring between the inner and outer electrodes 204, 205 when the ultrasound transducer 200 is placed within the electrically conductive fluid and a voltage is applied between the inner and outer electrodes 204, 205. More specifically, the electronics 22 may be electrically coupled to inner and outer electrodes 204, 205 via electrical cabling, and may actuate the transducer 200 (or any of the other transducers described herein) by applying a voltage between the inner and outer electrodes 204, 205 (or any other pair of electrodes described herein), so as to cause the piezoelectric material of the piezoelectric transducer body 208 to generate an unfocused ultrasonic wave that radiates radially outwardly.

In certain embodiments, the ultrasound transducer 200 is placed within a balloon (e.g., 14) that is at least partially filled with cooling fluid, which is used to cool a portion of a body lumen BL within which the ultrasound transducer 200 may be positioned. In certain embodiments, the cooling fluid is an electrically conductive fluid, e.g., saline, non-pure water, or sodium lactate solution, or a combination thereof, but is not limited thereto. In alternative embodiments, which can be referred to as balloonless embodiments, the ultrasound transducer 200 is directly exposed to blood flowing through a body lumen BL within which the ultrasound transducer may be positioned, in which case the electrically conductive fluid comprises the blood. In still other embodiments, an air-backed ultrasound transducer 200 is located within a balloon, but the cooling fluid is not circulated, but rather, there is no flow through the balloon. Bench tests have shown that no flow (of a cooling fluid) in a balloon can adversely impact the durability of a water-backed ultrasound transducer and may reduce the acoustic output power output of the water-back transducer. Beneficially, bench tests have shown that air-backed transducers described herein can successfully operate under no flow conditions. The use of no flow can reduce the system footprint be eliminating the need for cooling fluid cartridge, and may also help to bring in the near field lesion.

In the embodiments described above, with reference to the above described FIGS., the transducers 200 were shown as generally having a uniform thickness along the longitudinal length of the transducers 200, with the exception of there being a step at one or both of the longitudinal ends of the transducers 200. In accordance with specific embodiments, described below, the transducer has a thickness that is different at different locations on the transducer, wherein the change in the thickness of the transducer allows the direction that the acoustic signal that travels away from the transducer to be controlled and/or tuned. Accordingly, the acoustic signal can be steered at a treatment site. The ability to steer the acoustic signal allows the acoustic signal to be directed toward desired targets such as nerves, cancer cells, cardiac tissue, and/or features such as calcification and/or plaques. Additionally, the ability to steer the acoustic signal allows the acoustic signal to be directed away from non-targeted tissues, e.g., lymph nodes, hypaxial skeletal muscle with fibrous sheaths, peritoneum, periadventitial vessels, calcification, organs, e.g., ureter, intestines, liver, pancreas, urethra, renal pelvis, bladder, liver, pancreas, spleen, and kidneys, and/or features such as calcification and/or plaques. In addition, the change in the thickness of the transducer allows the catheter to more selectively ablate far-field and/or near-field targets. A portion of the transducer that activates at a lower frequency may be used to aim at deeper regions. A portion of the transducer that activates at a higher frequency may be used to target nearer regions. The transducers described below, that have a varying thickness, are labeled as transducers 300. It is noted that the transducers 300, which have a varying thickness, can be used in any of the embodiments described above with reference to FIGS. 1A-11, in place of the transducers 200 generally having a uniform thickness along the longitudinal length of the transducers 200

FIG. 12A is a cross section of the distal end of the catheter taken along the longitudinal axis of the catheter. FIG. 12B is a cross section of the catheter shown in FIG. 12A taken along the line labeled B in FIG. 12A. FIG. 12C is a cross section of the catheter shown in FIG. 12A taken along the line labeled C in FIG. 12A. FIG. 12D is a sideview of the distal end of the catheter shown in FIG. 12A. The balloon 14 can be positioned between the catheter shaft 12 and the tip member 15. The balloon 14 can be secured along an exterior of the catheter shaft 12 and/or an exterior of the tip member 15. Suitable mechanisms for securing the balloon 14 to the exterior of the catheter shaft 12 and/or an exterior of the tip member 15 include, but are not limited to, friction fits, adhesion mechanisms such as a glues, adhesives, and epoxies; a mechanical attachment mechanism such as a retainer, lock ring, or clamp such as a ring clamp or hose clamp; and welds such as laser welds and thermal welds, and combinations thereof.

In an embodiment, a transducer assembly 302 is positioned in an interior of the balloon 14. The transducer assembly 302 can include a transducer 300. The transducer can have an interior surface and an exterior surface. An inner electrode 36 contacts the interior surface and can extend along a length of the transducer 300. An outer electrode 38 contacts the exterior surface and can extend along a length of the transducer 300. Suitable materials for the transducer 300 include, but are not limited to, piezoelectric materials including piezoelectric ceramics, crystalline and polymers, acoustic micro-electromechanical systems (MEMS) transducers such as piezoelectric micromachined ultrasonic transducers (PMUT) and capacitive micromachined ultrasonic transducer (CMUT). Examples of suitable piezoelectric materials include, but are not limited to, lead zirconate titanate (PZT), CMUT, and PMUT. In certain embodiments, suitable for use in renal denervation, the materials for the transducer 300 includes or consists of lead zirconate titanate 8 (PZT8), which is also known as Navy III Piezo Material. In certain embodiments, the PZT8 is HIPped. Raw PZT transducers may be plated with layers of copper, nickel and/or gold to create the inner electrode 36 and the outer electrode 38. Additionally or alternatively, transducer 300 may be an air-backed transducer according to an embodiment described herein. Advantageously, using an air-backed directional, nonconcentric transducer enables a smaller and/or more efficient targeting of tissue because the backing member 42 can be nonconcentric within the transducer assembly 302 without impacting the acoustic efficiency.

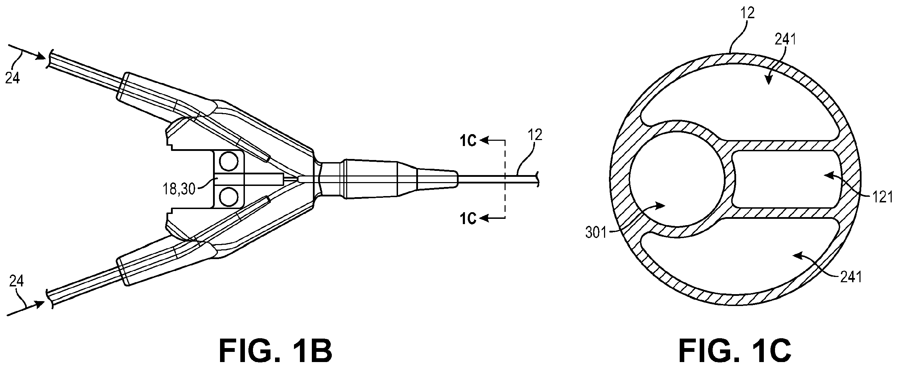

FIG. 12A shows a fluid lumen 40 in the catheter shaft 12. The lumen 40 includes a fluid port 41 through which the fluid lumen 40 and the interior of the balloon 14 can exchange fluid. As a result, the fluid lumen 40 and the interior of the balloon 14 are in fluid communication. Accordingly, the fluid lumen 40 provides fluid communication between an interior of the balloon 14 and one of the conduits 26 disclosed in the context of Figure 1. The catheter system can be configured to drive fluid through the fluid lumen 40 into the interior of the balloon 14 and/or to withdraw fluid from the interior of the balloon 14 through the fluid lumen 40. As a result, the fluid can be used to inflate or deflate the balloon 14. Alternately, the catheter shaft 12 can include multiple fluid lumens 40 that are each open to an interior of the balloon 14. The catheter system can be configured to drive fluid through a first selection of the fluid lumens 40 into the interior of the balloon 14 and to withdraw fluid from the interior of the balloon 14 through a second selection of the fluid lumens 40. The relative flow of fluid into the balloon 14 and out of the balloon 14 can be varied so as to inflate the balloon 14, deflate the balloon 14, or keep the balloon 14 inflation level at steady state.

In some instances, the fluid is a liquid. A fluid in the interior of the balloon 14 can be in contact with the transducer assembly 302. For instance, the fluid can contact the transducer 300, the inner electrode 36 and/or the outer electrode 38. As a result, the fluid can provide cooling of the transducer assembly 302. In some instances, the fluid source 28 disclosed in the context of Figure 1 is configured to pre-cool the fluid and/or to store pre-cooled fluid. As a result, the fluid is cooled before entering the fluid lumen 40 and/or the interior of the balloon 14. Suitable temperatures for a pre-cooled fluid include, but are not limited to, temperatures from the freezing point of the fluid to room temperature, and/or temperatures greater than or equal to 0 °C, 15°C, or 25°C and/or less than 20 °C, 25°C, or 37 °C. Examples of suitable fluids include, but is not limited to, sterilized water, dextrose, saline, or other suitable cooling fluid.

A backing member 42 can be positioned in an interior of the acoustic transducer 300. In some instances, the backing member 42 is also positioned in an interior of the inner electrode 36. The inner electrode 36 and/or the transducer 300 can surround and/or define an opening or void in which the backing member 42 is positioned. As is evident from FIG. 12A, the backing member 42 can extend beyond the ends of the transducer 300. The backing member 42 extends from the distal end of the catheter shaft 12 to the tip member 15. An end of the backing member 42 is received in a recess 44 at a distal end of the catheter shaft 12 and the opposing end of the backing member 42 is received in a recess 44 on the tip member 15.

A backing member lumen 46 extends longitudinally through the backing member 42. An electrical insulator 48 can be positioned in an interior of the backing member lumen 46. A second guidewire lumen 50 extends longitudinally through the backing member 42 and can be defined by the electrical insulator 48. The second guidewire lumen 50 is aligned with a guidewire lumen 51 that extends longitudinally through the catheter shaft 12. The guidewire lumen 51 and the second guidewire lumen 50 are sized to receive a guidewire (not shown). The electrical insulator 48 is positioned so as to electrically insulate the backing member 42 from a guidewire received in the second guidewire lumen 50. Suitable materials for the electrical insulator 48 include, but are not limited to, polyimide, other polymeric or elastomeric material, and other natural or synthetic materials. The backing member 42 can be constructed of an electrically conducting. Suitable materials for the backing member 42 include, but are not limited to, tungsten, steel, and aluminum.

The second guidewire lumen 50 is aligned with a tip member 15 lumen. The tip member 15 lumen is sized to receive the guidewire (not shown). As a result, the catheter can be moved along a guidewire positioned in the guidewire lumen, the second guidewire lumen 50 and the tip member 15 lumen.

One or more spacing components 54 can be positioned between the backing member 42 and the transducer assembly 302. The spacing components 54 can be configured to maintain a space between the backing member 42 and the transducer assembly 302. The spacing components 54 can include multiple spacers 56 that extend away from a spacer body 58. An opening can extend through the spacer body 58. The opening can be sized to receive the backing member 42. Accordingly, the spacer body 58 can surround the backing member 42.

The spacers 56 can contact the interior of the transducer assembly at one or more contact locations. For instance, the spacers 56 can contact the inner electrode 36 at the one or more contact locations. The contact locations can be selected to preserve the position of the transducer 300 and the backing member 42 relative to one another while also permitting the fluid within the balloon 14 to flow into contact with the interior of the transducer assembly 302 and with the exterior of the backing member 42. In some instances, the contact locations are selected to keep the backing member 42 concentric within the transducer assembly 302 and also configured to permit the fluid within the balloon 14 to flow past the backing member 42 into contact with the interior of transducer assembly 302 and/or to flow from contact with the interior of transducer assembly 302 past the backing member 42.

FIG. 12B shows a first group of contact locations at the same location along the length of the longitudinal axis (labeled L). The contact locations in FIG. 12B are spaced apart from each other and are positioned around the longitudinal axis of the backing member 42. The spacing between the contact locations is a result of openings between the spacers 56 that allow fluid to flow into the interior of the transducer assembly 302. FIG. 12B illustrates three spacers 56 that are generally equally spaced apart from one another at an angle of 120° where the angle is measured from the longitudinal axis of the backing member 42. However, the quantity, shape, size, orientation, spacing and/or other details of the spacers 56 can vary, as desired or required by a particular design or application.

One or more of the spacing components 54 can be electrically conducting. Suitable materials for an electrically conducting spacing component 54 includes, but is not limited to, steel, copper, and aluminum.

In the embodiment illustrated in FIG. 12A, the catheter system includes a second spacing component 54 spaced apart from the spacing component 54 of FIG. 12B along the longitudinal axis of the backing member 42. The spacing components 54 can be positioned at or near opposing ends of the transducer assembly 302.

The catheter shaft 12 includes an electrical lumen 64. The illustrated electrical lumen 64 includes a conductor carrier 66. The conductor carrier 66 extends through a wall of the catheter shaft 12 into the interior of the balloon 14. The conductor carrier 66 includes a first electrical conductor 70 that can be connected to the backing member 42. An electrically conducting backing member 42 and an electrically conducting spacing component 54 provide electrical communication between the inner electrode 36 and the first electrical conductor 70. Additionally, the first electrical conductor 70 is in electrical communication with the electronics 22, e.g., a generator, through the electrical coupling 18 and one of the external electrical conductors 20.

The conductor carrier 66 includes a second electrical conductor 72 that can be connected to an outer electrode 38 of the transducer assembly 302. Additionally, the second electrical conductor 72 is in electrical communication with the electronics 22 through the electrical coupling 18 and one of the external electrical conductors 20. As a result, the outer electrode 38 of the transducer assembly 302 is in electrical communication with the electronics 22 through the second electrical conductor 72, the electrical coupling 18, and one of the external electrical conductors 20.

Since the electronics 22 are in electrical communication with the inner electrode 36 and the outer electrode 38, application of a voltage and alternating current across the inner electrode 36 and the outer electrode 38 causes the transducer 300 to vibrate transverse to the longitudinal axis of the transducer 300 and radially emit an acoustic signal. In some instances, the transducer 300 is operated such that the acoustic signal has a desired frequency level.

The thickness of the transducer 300 is labeled T in FIG. 12B and FIG. 12C. The thickness changes such that the thickness of transducer 300 is different at different locations around the transducer. For instance, the thickness of the transducer 300 in FIG. 12B and FIG. 12C changes smoothly as the location on the transducer moves around the inner surface of the transducer 300. The change in the thickness of the transducer 300 can be a result of an opening defined by the inner surface of the transducer 300 being nonconcentric with an opening defined by the outer surface of the transducer 300.

FIG. 12E illustrates the transducer 300 of FIG. 12C outside of the balloon. As a result of the changing thickness of the transducer 300, the direction that the acoustic signal travels away from the transducer 300 changes in response to changes in the frequency of the applied alternating current. The change in direction results from the efficiency of the transducer 300 being different for different applied alternating current frequencies. In general, the thickness of the portion of the transducer 300 that produces the acoustic signal is inversely proportional to the frequency of the alternating current applied to the transducer 300. As a result, the frequency of the applied alternating current can be tuned so as to tune the portion of the transducer 300 that produces the acoustic signal and accordingly tunes the direction that the acoustic signal travels away from the transducer 300. In certain embodiments, a portion of the transducer 300 that activates at a lower frequency (a thicker portion) is used to aim at deeper regions (e.g., 4mm to 10mm from the lumen of a blood vessel). A portion of the transducer 300 that activates at a higher frequency (a thinner portion) is used to target nearer regions (e.g., .5mm to 4mm from the lumen of a blood vessel).

FIG. 12E has an arrow labeled A that represents a direction that an acoustic signal travels away from the transducer 300. The direction that the acoustic signal travels away from the transducer 300 can be represented by an angle labeled Θ where Θ is measured relative to a measurement line such as the measurement line labeled S in FIG. 12E. Examples of suitable measurement lines include, but are not limited to, a line of symmetry for the outer surface of the transducer. In some instances, the measurement line extends through the center of the outer surface of the transducer, the center of gravity of the outer surface of the transducer and/or the center of gravity of the transducer.

FIG. 12E also includes a graph of an exemplary power distribution for the acoustic signal labeled A. The power distribution shows the power level of the acoustic signal labeled A from the angle Θ labeled R to the angle Θ labeled Q. The angle Θ associated with the acoustic signal labeled A (the representative angle) can be chosen to be representative of the direction that the acoustic signal labeled A travels away from the transducer. For instance, the representative angle associated with the acoustic signal labeled A can be located at the maximum in the power distribution, the average of the power distribution over a range of angles Θ, the average of the power distribution weighted by power and taken over a range of angles 30°, 90°, or 180°.

In order to illustrate the steerable nature of the acoustic signal, FIG. 12E also includes arrows labeled B and C. Each of the arrows labeled A, B, and C represents a direction that a different acoustic signal travels away from the transducer 300. The acoustic signal represented by the arrow labeled A occurs when a higher alternative current frequency ("a first frequency") is applied to the transducer 300 than the acoustic signal represented by the arrow labeled B. Additionally, the acoustic signal represented by the arrow labeled B occurs when a higher alternative current frequency ("a second frequency") is applied to the transducer 300 than the acoustic signal represented by the arrow labeled C. And the acoustic signal represented by the arrow labeled C occurs when a lower alternative current frequency ("a third frequency") is applied to the transducer 300 than the acoustic signal represented by the arrows labeled B or A. Accordingly, different angles are associated with different alternating current frequency levels.

The measurement line labeled S in FIG. 12E is a line of symmetry. In an embodiment, a first region of the transducer 300 on one side of the line of symmetry has a thickness that is the same as a second region of the transducer on the opposite side of the line of symmetry. As a result, the transducer 300 can concurrently output the same acoustic signals from opposite regions of the transducer 300. For instance, the transducer of FIG. 12E can concurrently output an acoustic signal labeled A and an acoustic signal labeled A' equal in power at the first frequency, or can concurrently output the acoustic signal labeled B and an acoustic signal labeled B' equal in power at the second frequency, or can concurrently output the acoustic signal labeled C and an acoustic signal labeled C' equal in power at the third frequency. As a result, a single alternating current frequency level can be associated with more than one angle Θ.

The transducer 300 of FIG. 12E can be modified such that when the transducer 300 has multiple different regions of the same thickness, the region from which the acoustic signal is output can be selected. For instance, FIG. 13 illustrates the transducer 300 of FIG. 12E modified such that the outer electrode 38 is divided into a first outer electrode 60 and a second outer electrode 62. The first outer electrode 60 and a second outer electrode 62 are positioned on opposing sides of the line of symmetry (labeled S). The conductor carrier 66 (FIG. 12A) can include three different electrical conductors including the first electrical conductor 70, the second electrical conductor 72, and a third electrical conductor (not shown). The electronics 22 can be in electrical communication with the inner electrode 36 through the first electrical conductor 70 as described above. The electronics 22 can be in electrical communication with the first outer electrode 60 through the second electrical conductor 72. The electronics 22 can be in electrical communication with the second outer electrode 62 through the third electrical conductor.

The acoustic signal that is output from the transducer 300 can be selected by selecting the combination of the electrodes to which the alternating current is applied. For instance, the acoustic signal labeled A in FIG. 13 can be selected by applying the alternating current between the inner electrode 36 and the first outer electrode 60. The direction of the acoustic signal labeled A in FIG. 13 can be tuned by tuning the frequency of the applied alternating current. The acoustic signal labeled A' in FIG. 13 can be selected by applying the alternating current between the inner electrode 36 and the second outer electrode 62. The direction of the acoustic signal labeled A' in FIG. 13 can be tuned by tuning the frequency of the applied alternating current.

Rather than modifying the transducer 300 of FIG. 12E such that the outer electrode 38 is divided into a first outer electrode 60 and a second outer electrode 62, the inner electrode 36 can be divided into a first inner electrode (not shown) and a second inner electrode (not shown). The transducer assembly 302 can then be configured such that the electronics are in electrical communication with each of the electrodes through a different one of the conductors selected from the group consisting of the first electrical conductor 70, the second electrical conductor 72, and the third electrical conductor (not shown). As a result, the acoustic signal that is output from the transducer 300 can be selected by selecting the combination of the electrodes to which the alternating current is applied.

The transducer 300 of FIG. 12E can also be modified so as to eliminate or reduce the presence of transducer 300 regions having the same thickness. As an example, FIG. 14A shows the transducer 300 of FIG. 12E modified such that the transducer excludes regions of the same thickness. For instance, the thickness of the transducer 300 increases from a minimum thickness (Tᵢ) to a maximum thickness (Tₐ) until a transition region 68 is reached where the thickness returns to the minimum thickness (Tᵢ). The change from the minimum thickness (Tᵢ) to the maximum thickness (Tₐ) can be smooth and extend over an angular range Θ greater than 340° or 355° and less than or equal to 360°. The transition region 68 can extend over an angular range Θ greater than or equal to 0.0°, 30°, 90°, or 180° and less than or equal to 90°, 180°, or 270°. The angle Θ can be measured relative to a measurement line (labeled M) that extends through the center of the outer surface of the transducer, the center of gravity of the outer surface of the transducer and/or the center of gravity of the transducer.

Although FIG. 14A illustrates the inner electrode 36 over and/or in contacting with the transition region 68, the inner electrode 36 need not be positioned over and/or in contacting with the transition region 68. As an example, FIG. 14B shows the transducer 300 of FIG. 14A modified such that the inner electrode 36 is not be positioned over and/or in contacting with the transition region 68.

Although FIG. 14A and FIG. 14B illustrate the transducer assembly 302 and outer electrode 38 as having a circular cross section, the transducer assembly 302 and/or outer electrode 38 need not be circular. Although FIG. 12A through FIG. 13 illustrate the transducer assembly 302 and outer electrode 38 as having an oval or substantially oval cross section, the transducer assembly 302 and/or outer electrode 38 need not be circular. Suitable geometries for the cross sections of one or more components selected from the group consisting of the transducer assembly 302, the transducer 300, the outer electrode 38 and the inner electrode 36 include, but are not limited to, triangular, or any shape
The above transducers 300 have a thickness that extends from a minimum thickness (Tᵢ) to a maximum thickness (Tₐ) as is labeled in FIG. 12E and FIG. 14A. The minimum thickness (Tᵢ) can be associated with a minimum thickness frequency and the maximum thickness (Tₐ) can be associated with a maximum thickness frequency. The minimum thickness frequency can represent an alternative current frequency that is applied to the transducer 300 and causes the acoustic signal to be output from the region of the transducer 300 with the minimum thickness (Tᵢ). For instance, the minimum thickness frequency can represent an alternative current frequency that is applied to the transducer 300 and causes the acoustic signal to travel away from the transducer 300 in a direction that extends from the region of the transducer 300 having the minimum thickness (Tᵢ). The maximum thickness frequency can represent an alternative current frequency that is applied to the transducer 300 and causes the acoustic signal to be output from the region of the transducer 300 with the maximum thickness (Tₐ). The maximum thickness frequency can represent an alternative current frequency that is applied to the transducer 300 and causes the acoustic signal to travel away from the transducer 300 in a direction that extends from the region of the transducer 300 having the minimum thickness (Tᵢ).

A suitable minimum thickness (Tᵢ) includes, but it not limited to, a thickness greater than 0.1mm, 0.5mm, or 2mm and/or less than 5mm, 3mm, or 1mm. Additionally or alternately, a suitable maximum thickness (Tₐ) includes, but it not limited to, a thickness greater than 0.1mm, 0.5mm, or 2mm and/or less than 5mm, 3mm, or 1mm. Additionally or alternately, suitable frequencies for the alternating current applied to the transducer 300 include, but are not limited to, frequencies in a range that extends from a frequency greater than or equal to 1MHz, 5MHz, 9MHz, 12-15MHz, or 20MHz to a frequency less than 20MHz or 10MHz. In one example, the frequency of the alternating current applied to the transducer 300 is in a range that extends from a frequency greater than or equal to 8MHz to a frequency less than or equal to 15MHz.

Referring to the transducer cross sections of FIG. 14B and FIG. 12E, an angle Θ (not shown) can be measured relative to a measurement line labeled M. The measurement line can extend through an angle vertex at the center of the outer surface of the transducer, the center of gravity of the outer surface of the transducer and/or the center of gravity of the transducer. The angle Θ can be determined between the measurement line and a line that extends from the outer surface of the transducer to the angle vertex. Although the above transducers show the thickness of the transducer changing continuously over the angle Θ that is equal or substantially equal to 360°, the transducers can have one or more regions where the thickness of the transducer does not change. In some instances, the thickness of the transducer changes continuously over the angle Θ between 0° and 360°.

The transducer assemblies 302 and/or the transducer 300 has a length (labeled Lt in FIG. 12A). In some instances, the above transducer cross-sections can represent the cross-section of the transducer for the full length of the transducer 300. In some instances, the transducer assemblies 302 and/or the transducer 300 has a length greater than or equal to 0.5 mm and/or less than or equal to 12 mm and/or a diameter greater than or equal to 3 French and/or less than or equal to 10 French. In one example suitable for renal denervation and other applications, the transducer assemblies 302 in a balloon and/or the transducers in the balloon each has a length greater than or equal to 2 mm and/or less than or equal to 8 mm and a diameter greater than or equal to 3 French and less than or equal to 6 French.

Although the transducer is illustrated as having an inner surface and/or an outer surface that are elliptical or circular, the inner surface and/or outer surface can have other geometries.

The distal end of the catheter is configured to be inserted into a body lumen of a subject. Examples of suitable body lumens include, but are not limited to, veins and/or arteries such as a renal artery. As an example, FIG. 15 is a cutaway view of a body lumen 75 that has a plurality of nerves 73 in an outer layer. For instance, the body lumen 75 of FIG. 15 can represent a blood vessel such as a renal artery having nerves 73 in the adventitia layer. As illustrated in FIG. 15, the balloon 14, the distal portion of the catheter shaft 12, the tip member 15 are received in the body lumen 75. A guidewire 31 can be used to aid placement of the catheter in the body lumen 75 as shown FIG. 15.

The nerves 73 shown in FIG. 15 are arranged in a bundle above the body lumen 75. When it is desired to direct an acoustic signal to the nerves 73, the frequency of the alternating current applied to the transducer 300 can be selected so the transducer 300 outputs an acoustic signal that travels away from the transducer 300 in a direction that is toward the nerves 73. For instance, the alternating current applied to the transducer 300 can be selected so the acoustic signal that travels in the direction of the arrows labeled A in FIG. 15. The ability to control the direction of the acoustic signal allows for targeting of the nerves 73.

In some instances, the treatment site within the body lumen 75 is positioned near a biological body 74 that could be harmed by exposure to an acoustic signal. As a result, FIG. 15 illustrates a biological body 74 that is sufficiently close to the transducer 300 to be exposed to an acoustic signal from the transducer 300. The ability to control the direction of the acoustic signal can prevent or reduce damage to the biological body 74. Examples of biological bodies include, but are not limited to, organs such as bowels, kidneys, ureters, renal pelvis, bladder, urethra, liver, pancreas, spleen, and tissues such as lymph nodes and muscle.

In some instances, it is desirable for the acoustic signal to be uniformly applied to around the treatment site. In these instances, the alternating current frequency applied to the transducer can be swept from the minimum thickness frequency to the maximum thickness frequency. This frequency sweep can be continuous or repeated one or more times. The alternating current frequency applied to the transducer can be swept from the minimum thickness frequency to the maximum thickness frequency and then from the maximum thickness frequency back to the minimum thickness frequency. This frequency sweep sequence can be repeated one or more times.

In some instances, the orientation of the transducer 300 within a body lumen 75 may be unknown at the time of placement of the transducer 300 at the treatment site. It may be desirable to know the orientation of the transducer 300 in order to direct the acoustic signal to a desired target. One or more receivers can be used to identify the orientation of the transducer 300. As an example, FIG. 16 illustrates the balloon 14 of a catheter positioned at a treatment site in the body 76 of a patient. The treatment site includes a bundle of nerves or ganglia to which an acoustic signal is to be delivered. A receiver is positioned outside of the body 76 so as to receive the acoustic signal output from the transducer 300. Suitable receivers include, but are not limited to, an ultrasound transducer, external magnetic detector.

The alternating current frequency applied to the transducer can be swept through a frequency range. In some instances, the frequency range extends from the minimum thickness frequency to the maximum thickness frequency. The alternating frequency that provides the largest acoustic signal power to a particular one of the receivers 78 during the sweep can be identified. For instance, the alternating frequency provides the largest acoustic signal power to the receiver 78 labeled A during the sweep can be identified.

The angle associated with the identified frequency (θ_{f}) can also be identified and can indicate the angular orientation of the receiver 78 relative to a measurement line (labeled M). The angular orientation of the receiver 78 relative to the nerves 73 (labeled θ_{T}) can be known as a result of measurements taken by an imaging device. Suitable imaging devices include, but are not limited to, an ultrasound imaging system.

The value of the angle θ_{T} between the nerves 73 and the measurement line can be determined from by adding the angle associated with the identified frequency (θ_{f} ) and the angular orientation of the receiver 78 relative to the nerves 73 (θ_{T}). In order to apply an acoustic signal to the nerves 73, an alternating current can be applied to the transducer 300 at the frequency associated with the angle θ_{T}.

Although FIG. 16 illustrates a single receiver 78, more than one receiver 78 can be used in order to confirm the accuracy of the identified frequency and/or the accuracy of the angular orientation of the receiver 78 relative to the measurement line (θ_{f}). As is evident from the description of FIG. 16, the measurement line can serve as a reference line against which other measurements are taken.

Figure 8 is a sideview of an alternative catheter system. A catheter system may include all the components for FIG. 16, as well as an imaging transducer 17, e.g., a transducer of a single element or an array, at the distal end of catheter 10. The imaging transducer 17 may have a center frequency of 15-50MHz, e.g., 20-30MHz, which can be used to identify target and nontarget structures.

In certain embodiments, a catheter system may additionally or alternatively include electrodes on the balloon 14, configured to sense nerve activity and/or confirm the effectiveness of the treatment, as disclosed in U.S. Patent Application No. 17/813,311, to Zhai et al., filed July 19, 2022, which claims priority to U.S. Provisional Patent Application No. 63/306 496**.**

In certain embodiments, the catheter system may additionally or alternatively include the nerve sensing and/or treatment confirmation components disclosed in U.S. Patent Application No. 18/182,821 filed March 13, 2023 (PMD00104US), which claims priority to U.S. Patent Application No. 63/320 103**.** As disclosed in further detail in U.S. Patent Application No. 18/182,821 and U.S. Patent Application No. 63/320,103, treatment confirmation components may be used to determine a latency of sensed electrical impulses to determine the type, size, function and/or health of the fibers whose neural response is being sensed. The delay (aka latency) may be indicative of a depth of nerves surrounding a biological lumen (e.g., a renal artery) within which a catheter used to measure the delay is located. In accordance with certain embodiments of the present technology, the above-described delay (aka latency) can be used to select the frequency used for the ablations. The electronics 22 controls the catheter 10 to sweep the operating frequency and control the durations of the individual and total time to control the temperature in the ablation zones, and shape the lesion. A portion of the transducer 300 that activates at a lower frequency is used to aim at deeper regions when it is determined that nerves are located in the deeper regions. A portion of the transducer 300 that activates at a higher frequency is used to target nearer regions when it is determined that nerves are located in the nearer regions. The imaging transducer 17 can be positioned before or after the balloon 14. The imaging transducer 17 can comprise of a ring array having a single ring or multiple rings. The imaging depth can be up to approximately 12 mm and can be used to size the vessel, image anatomy, pathology, vessel walls, plaques, calcification, tissue layers and nerves. The imaging frequency can be approximately 20 MHz - 35 MHz, the bandwidth can be equal or greater to 10 MHz, the array size can comprise 16 to 256 elements, however, this is not restrictive. The array element dimension can be 0.5 mm - 1.5 mm in length and 0.5 mm - 2 wavelengths in width. Multiple-row cylindrical array can help reduce the image slice thickness to achieve better contrast resolution. The elements are individually controlled to transmit and receive, for example, by an ASIC circuit, to reduce the number of cables needed.

In some instances, a catheter constructed as disclosed in the context of Figure 1 through Figure 8 has a catheter shaft 12 with diameter greater than or equal to 3 French and/or less than or equal to 9 French and/or a catheter length greater than or equal to 75cm and/or less than or equal to 175cm. In one example suitable for renal denervation, the catheter has a catheter shaft with a diameter greater than or equal to 3 French and less than or equal to 6 French and/or a catheter length greater than or equal to 85cm and less than or equal to 155cm.

Although the transducer 300 is disclosed as being positioned in a balloon 14, the transducer 300 can be positioned outside of a balloon 14 and/or the catheter can exclude the balloon 14. For instance, a catheter can be constructed as disclosed in FIG. 12A without the balloon 14. Accordingly, the transducer 300 can be coupled to the catheter shaft without the balloon 14 being present on the catheter.

The balloon 14 can be a compliant balloon 14 or non-compliant balloon 14 configured to be inflated to a diameter between a first inflation diameter and a second inflation diameter using an inflation pressure between a first inflation pressure and a second inflation pressure such that the outer diameter is directly correlated with the pressure of the balloon 14. In some instances, the balloon 14 is configured to inflate from the first inflation diameter to the second inflation diameter using a constant inflation pressure. The relationship between the inflation diameter and the inflation pressure may change over time in response to multiple inflation and deflation cycles of the balloon. For instance, as the number of inflation and deflation cycles increases, less pressure may be needed to maintain a balloon at the same diameter.

Suitable first inflation diameters include, but are not limited to, first inflation diameters greater than or equal to 1.5mm, 2.0mm, or 3.0mm and/or less than or equal to 3.5mm, 5.0mm, or 8.0mm. Suitable second inflation diameters include, but are not limited to, first inflation diameters greater than or equal to 3.5mm, 4.0mm, or 5.0mm and/or less than or equal to 6.0mm, 8.0mm, or 10mm. Suitable first inflation pressures include, but are not limited to, first inflation pressures greater than or equal to 13.79 kPa (2 psi), 34.47 kPa (5 psi), or 55.16 kPa (8 psi) and/or less than or equal to 82.74 kPa (12 psi), 103.42 kPa (15 psi), or 206.84 kPa (30 psi). Suitable second inflation pressures include, but are not limited to, first inflation pressures greater than or equal to 13.79 kPa (2 psi), 34.47 kPa (5 psi), or 55.16 kPa (8 psi) and/or less than or equal to 82.74 kPa (12 psi), 103.42 kPa (15 psi), or 206.84 kPa (30 psi). A suitable constant inflation pressure can be between or equal to the first inflation pressure and the second inflation pressure. In one example, the first inflation diameter is greater than 1.5 mm and less than 5.0 mm; the second inflation diameter is greater than or equal to 3.5 mm and less than 8.0 mm; the first inflation pressures is greater than 34.47 kPa (5 psi) and less than 172.37 kPa (25 psi); and the second inflation pressures is greater than 34.47 kPa (5 psi) and less than 172.37 kPa (25 psi).

The inflation of the balloon 14 to the second inflation diameter and/or the second inflation pressure can cause the balloon 14 to contact an interior surface of the body lumen. For instance, the inflation of the balloon 14 to the second inflation diameter and/or the second inflation pressure can cause the balloon 14 to contact an interior surface of the renal artery. The portion of the balloon 14 surface that is configured to come into contact with an interior surface of a body lumen (the contact portion of the balloon 14 surface) has a length labeled L in FIG. 12A. In some instances, the length of the contact portion of the balloon 14 surface is greater than 1 mm, or 18 mm and/or is less than 30 mm, or 100 mm. In one example, the length of the contact portion of the balloon 14 surface is greater than 1 mm and less than 90 mm. Increasing the length of the contact portion of the balloon 14 surface can provide room for one or more outer electrodes on the contact portion of the balloon 14 surface.

The electronics 22 can operate the transducer assembly 302 such that the transducer outputs the acoustic signal before, during, and/or after the inflation of the balloon 14 to the second inflation diameter and/or the second inflation pressure. In some instances, the electronics 22 can operate the transducer assembly 302 such that the transducer outputs an acoustic signal having a frequency in a range of from 1 to 20 MHz. For example, the transducer can be configured to output an acoustic signal having a frequency of about 9 MHz. In other instances, the transducer outputs the acoustic signal with a frequency of 12-15 MHz. In other instances, the transducer outputs the acoustic signal with a frequency below 1 MHz. For instance, the transducer can output the acoustic signal with a frequency greater than or equal to 0.1 MHz and less than MHz. The frequency of the acoustic signal can be a function of the particular application, function, or use of the catheter.

The power supplied to the transducer to generate the acoustic signal can vary, as desired or required. In some instances, the power supplied to the transducer to generate the acoustic signal is 5 to 80 Watts. The duration that the acoustic signal is applied to the body lumen can vary for a variety of factors including the treatment procedure, the power level at the transducer, the frequency of acoustic signal emitted, the size of the body lumen or type of tissue being treated, the age of the patient, weight of the patient, and gender of the patient. However, in some instances, the acoustic signal is applied to the body lumen for a duration greater than or equal to 0.1 seconds and less than or equal to 20 minutes. In an embodiment, the acoustic signal is applied to the body lumen for a duration of 5 to 10 seconds. In an embodiment, the acoustic signal is applied to the body lumen for a duration of 7 seconds. In certain embodiments, the acoustic signal is applied for a duration of 3.5 to 9 seconds, and more specifically for a duration from 4 to 5 seconds.

The above descriptions of the operation of a catheter and/or interior catheter describe one or more movements of the catheter within the body lumen. These movements include movements from one location to another location within the body lumen, and/or an adjustment of the catheter position within the body lumen. A movement of the catheter within the body lumen can include a deflation of any balloons at a first location within the body lumen, followed by physical rotation and/or translation of the catheter, followed by a re-inflation of one or more balloons on the catheter at a second location.

Suitable electronics 22 can include one or more components selected from the group consisting of analog electrical circuits, digital electrical circuits, processors, microprocessors, digital signal processors (DSPs), Application Specific Integrated Circuits (ASICs), computers, microcomputers, or combinations suitable for performing the operation, monitoring and control functions described above. In some instances, the electronics 22 include an RF electrosurgical generator such as electrosurgical unit or ESU for generating the energy in the electromagnetic signal output from all or a portion of the one or more electrode 76 selections. In some instances, the electronics 22 include a user interface that allows an operator to provide input to the electronics 22 and/or to extract information and/or data from the electronics 22. In some instances, the electronics 22 include a memory that carries instructions to be executed by the electronics 22 during performance of the operation, control and monitoring functions. Although the electronics 22 are illustrated as a single component in a single location, the electronics 22 can include multiple different components that are independent of one another and/or placed in different locations.

The application of the acoustic signal to the renal nerves can be effective for the treatment of hypertension. However, the catheter can be used for a variety of other applications such as applying the acoustic signal to renal arteries in order to treat chronic kidney disease, atrial fibrillation, arrhythmia, heart failure, chronic kidney disease, end stage renal disease, myocardial infarction, anxiety, contrast nephropathy, diabetes, metabolic disorder and insulin resistance, etc., to pulmonary arteries in order to treat pulmonary hypertension, or to hepatic artery in order to treat diabetes, to a splenic artery, celiac trunk, superior or inferior mesenteric artery in order to treat an autoimmune and/or inflammatory condition, such as rheumatoid arthritis, sepsis, Crohn's disease, ulcerative colitis, and/or gastrointestinal motility disorders, in the cardiovascular system for treatment of atrial fibrillation.

The high-level flow diagram of FIG. 17 will initially be used to summarize methods according to certain embodiments of the present technology.

Referring to FIG. 17, step 701 involves imaging the blood vessel and/or surrounding anatomical structures, using, e.g., computerized tomography (CT), magnetic resonance imaging, positron emission tomography, ultrasound (US), radiological machines, etc. Target (e.g., nerves, cancer cells, cardiac tissue, calcification and/or plaques) and non-target (e.g., lymph nodes, hypaxial skeletal muscle with fibrous sheaths, peritoneum, periadventitial vessels, organs, ureter, intestines, liver, pancreas, urethra, renal pelvis, bladder, liver, pancreas, spleen, kidneys, calcification, and/or plaques) structures and related locations are determined using the imaging. Step 702 involves inserting a catheter into a biological lumen (e.g., a renal artery, hepatic artery, etc.). In certain embodiments, imaging is performed by an imaging transducer located on the catheter 10, in which case step 702 could be performed prior to step 701.

Still referring to FIG. 17, step 704 involves selecting an ablation output based on the characteristics of the image. In an embodiment, an ablation output of the transducer 300 could be selected by selecting a frequency of the applied alternating current generated by the electronics 22 that selectively activates one or more areas of the transducer to direct an acoustic signal to a target and/or away from a non-target. In an embodiment, the electronics 22 determines an ablation output based on the imaging by selecting a combination of the electrodes to which the alternating current is to be applied that selectively activates one or more areas of the transducer to direct an acoustic signal towards a target and/or away from a non-target.

In an embodiment, the selecting is performed using the electronics 22 which comprises one or more processors, e.g., of an electrical control unit (ECU). In certain embodiments, ablation outputs are selected using one or more tables that are stored in memory (e.g., 1120 in FIG. 19) and accessed by at least one processor (e.g., of a controller 1122 in FIG. 19). In other embodiments, ablation parameters are selected using a machine learning model implemented by at least one of the one or more processors, or more generally, using artificial intelligence.

Step 706 involves performing the ablation procedure using the selected ablation output to thereby selectively ablate target structures, while avoiding non-target structures.

In accordance with certain embodiments, the ablation procedure that is performed at step 706 uses an ultrasound transducer of a catheter inserted into the biological lumen to emit ultrasonic energy. In certain such embodiments, the ablation parameters that are selected at step 704, and used at step 706, can specify a vector, amplitude, power, duration, frequency, and/or duty cycle of the ultrasound energy. In certain embodiments, the ultrasound transducer is located within a balloon that is at least partially filled with a cooling fluid that is circulated through the balloon in order to cool at least a portion of the tissue surrounding the biological lumen proximate the balloon. In certain such embodiments, the ablation parameters selected at step 704, and used at step 706, can specify a flow rate and/or a temperature associated with the cooling fluid.

Referring to FIG. 18, step 801 involves inserting a catheter into a biological lumen (e.g., a renal artery), and step 804 involves sensing neural activity of nerves within tissue surrounding a biological lumen, wherein the sensing is performed using at least one electrode of the catheter inserted into the biological lumen. Electrodes that are used to sense neural activity can be referred to herein as sensing electrodes. The neural activity that is sensed at step 804 can be native (aka spontaneous) neural activity. Alternatively, or additionally, the neural activity that is sensed at step 804 can be evoked neural activity that is evoked by electrical stimulation delivered using the same catheter used to sense the neural activity at step 804. More specifically, in certain embodiments, between steps 802 and 804 one or more electrodes of the catheter is/are used to emit stimulation energy (in step 802 shown in dashed line) that causes an evoked neural response that is sensed at step 804. An example of a catheter that includes electrodes and can be used to perform steps 802 and 804, as well as to selectively emit stimulation energy to evoke a neural response at step 802, is described with reference to FIGS. 3A and 3B of U.S. Patent Application No. 18/182,821, to Barman et al., filed March 13, 2023 (PMD00104US), which claims priority to U.S. Patent Application No. 63/320 103**.** However, it should be noted that embodiments of the present technology are not limited to being used with the example catheter described with reference to FIG. 3A and 3B of Barman et al.

Still referring to FIG. 18, step 806 involves determining characteristics of the sensed neural activity of the nerves within the tissue surround the biological lumen, wherein the characteristics are indicative of one or more of the size, type, function or health of the nerves for which neural activity is sensed, indicative of proximity of the nerves relative to the sensing electrode(s) of the catheter, and/or the nerve distribution/location around the renal artery, but not limited thereto.

Step 808 involves selecting a frequency at which an alternating current is to be applied to the transducer 300 and/or selecting denervation electrodes of the transducer 300 based on the characteristics of the sensed neural activity in order to selectively target nerves according to their distribution around a body lumen, wherein the denervation parameters are for use in performing a denervation procedure intended to denervate at least some of the nerves for which the neural activity was sensed, and wherein the selecting is performed using one or more processors, e.g., of an electrical control unit (ECU), an example of which is described below with reference to FIG. 19. In certain embodiments, a denervation frequency and/or electrodes are selected using one or more tables that are stored in memory (e.g., 1120 in FIG. 19) and accessed by at least one processor (e.g., of a controller 1122 in FIG. 19). In other embodiments, a denervation frequency and/or electrodes are selected using a machine learning model implemented by at least one of the one or more processors, or more generally, using artificial intelligence. A higher frequency may be used to activate a thinner portion of the transducer that is located proximal the nerves to selectively direct the acoustic signal toward the nerves. A lower frequency may be used to activate a thicker portion of the transducer that is located proximal the nerves to selectively direct the acoustic signal toward the nerves. In addition, or alternatively, electrodes located proximal the nerves may be selected in order to direct the acoustic signal toward the nerves.

In addition to selecting a frequency at which an alternating current is to be applied to the transducer 300 and/or selecting denervation electrodes of the transducer 300 based on the characteristics of the sensed neural activity, the selection of frequency and/or electrodes may also be based on imaging of the blood vessel, as described in FIG. 17. A processor (e.g., a controller 1122 in FIG. 19) may take into account both the nerve distribution and the location of structures such as lymph nodes, hypaxial skeletal muscle with fibrous sheaths, peritoneum, periadventitial vessels, organs, e.g., ureter, intestines, liver, pancreas, urethra, renal pelvis, bladder, liver, pancreas, spleen, and kidneys, and/or features such as calcification and/or plaques, in selecting the frequency at which an alternating current is to be applied to the transducer 300 and/or selecting denervation electrodes of the transducer 300. The processor may use characteristics of the image and characteristics of the sensed neural activity to more selectively target nerves, while avoiding non-target structures.

Step 810 involves performing the denervation procedure using the selected denervation frequency to thereby denervate at least some of the nerves for which the neural activity was sensed. Depending upon the specific implementation, the denervation procedure can be performed at step 810 using the same catheter used to sense the neural activity at step 804, or using a separate catheter inserted into the biological lumen after the catheter used to sense the neural activity at step 804 has been removed. In other words, in certain embodiments, one catheter may be swapped for another catheter during a time period between when step 804 is performed and when step 810 is performed. The catheter described in U.S. Patent Application No. 18/182,821, to Barman et al., filed March 13, 2023 (PMD00104US), which claims priority to U.S. Patent Application No. 63/320,103, may be used, except that the transducer 300 having a thickness that is different at different locations on the transducer and/or multiple electrode pairs that can be selectively chosen to change the depth and/or direction of the acoustic signal traveling away from the transducer 300, as described herein, may be used.

In accordance with certain embodiments, the biological lumen referred to in the flow diagram of FIG. 18 is a renal artery and the nerves comprise renal nerves innervating a kidney. The method can also include removing any catheter that had been inserted into a biological lumen, such as a renal artery.

In accordance with certain embodiments, the denervation procedure that is performed at step 810 using an ultrasound transducer 300 having a thickness that is different at different locations on the transducer and/or multiple electrode pairs that can be selectively chosen to change the depth and/or direction of the acoustic signal traveling away from the transducer 300, as described above, is inserted into the biological lumen to emit ultrasonic energy. In certain such embodiments, the denervation frequency and/or electrodes selected at step 808, and used at step 810 depends on the distribution of the nerves around the artery and/or whether they are located near field and/or far field. In certain embodiments, the ultrasound transducer is located within a balloon that is at least partially filled with a cooling fluid that is circulated through the balloon in order to cool at least a portion of the tissue surrounding the biological lumen proximate the balloon, e.g., as described above with reference to Figures 1 through 8. In certain such embodiments, further denervation parameters may be selected at step 808 based on characteristics of the sensed neural activity, and used at step 810, including amplitude, power, duration, and/or duty cycle of the ultrasound energy and a flow rate and/or a temperature associated with the cooling fluid.

Step 804 in FIG. 18 can be performed by using a pair of electrodes, at least one of which is on the catheter inserted at step 801, to sense a signal indicative of the neural activity, wherein the signal indicative of the neural activity including multiple peaks temporally spaced apart from one another. The electrodes that are used for sensing neural activity (whether spontaneous or evoked) can be referred to herein as sensing electrodes, and electrodes that are used to deliver stimulation energy to evoke a neural response can be referred to herein as stimulation electrodes. It is noted that switches within a catheter and/or within an electronic control unit (ECU) to which the catheter is electrically connected can be used to selectively change certain electrodes from sensing electrodes to stimulation electrodes, and/or vice versa, at different points in time. In accordance with certain embodiments, both electrodes of a pair of sensing electrodes are located on the catheter. In other embodiments, one sensing electrode of a pair of sensing electrodes is located on the catheter, while another sensing electrode of the pair is located in the distal end of an introducer sheath (that is used to insert the catheter into the biological lumen), or on the distal end of a guidewire (that is used to guide the catheter into the biological lumen), or alternatively is an external skin electrode located on the skin of a patient.

In certain embodiments, characteristics of the sensed neural activity are determined at step 806 based on amplitudes of the multiple peaks and/or based on temporal spacings between the multiple peaks. For example, an average amplitude of the multiple peaks can be determined and denervation parameters can be selected based on the average amplitude of the multiple peaks. Alternatively, or additionally, a median amplitude of the multiple peaks can be determined and denervation parameters can be selected based on the median amplitude of the multiple peaks. Alternatively, or additionally, determining characteristics of the sensed neural activity at step 806 can involve fitting a curve to a portion of the signal indicative of the neural activity, and determining an area under the curve, and step 808 can involve the selecting denervation parameters based on the area under the curve.

As noted above, in certain embodiments stimulation is emitted (at step 802) in order to evoke a neural response to the simulation. This can be achieved be emitting the stimulation using a first electrode (e.g., of a first pair of electrodes) on the shaft of a catheter and sensing the evoked response at a second electrode (e.g., of a second pair of electrodes) of the catheter that is a known distance from the first electrode. The electrodes that are used for stimulating nerves to evoke a neural response can be referred to herein as stimulation electrodes, as was noted above. As also noted above, switches within a catheter and/or within an electronic control unit (ECU) to which the catheter is electrically connected can be used to selectively change certain electrodes from sensing electrodes to stimulation electrodes, and/or vice versa, at different points in time. In accordance with certain embodiments, both electrodes of a pair of stimulation electrodes are located on the catheter. In other embodiments, one stimulation electrode of a pair of stimulation electrodes is located on the catheter, while another stimulation electrode of the pair is located in the distal end of an introducer sheath (that is used to insert the catheter into the biological lumen), or on the distal end of a guidewire (that is used to guide the catheter into the biological lumen), or alternatively is an external skin electrode located on the skin of a patient.

A delay (aka latency) between when the stimulation is delivered and when an evoked neural response (to the stimulation) is detected can be determined, which delay (aka latency) is indicative of a conduction velocity of the nerves that responded to the stimulation. Different nerve fibers can have different conduction velocities. Some electrical impulses travel faster than others. Larger neural fibers tend to have very fast conduction velocities. By contrast, smaller neural fibers tend to have relatively slower conduction velocities compared to the larger neural fibers. Accordingly, the latency of the sensed electrical impulses can be used to determine the type, size, function and/or health of the fibers whose neural response is being sensed. The delay (aka latency) may be indicative of a depth of nerves surrounding a biological lumen (e.g., a renal artery) within which a catheter used to measure the delay is located. In accordance with certain embodiments of the present technology, the above-described delay (aka latency) is another example of a characteristic of the sensed neural activity that can be determined at an instance of step 806 in FIG. 18, and which can be used to select denervation parameters at step 808 in FIG. 18.

When a nerve fiber is being ablated during a denervation procedure, such that the health of the nerve fibers has been diminished or the nerve has been destroyed, the amplitude and shape of a sensed signal indicative of the neural activity, its latency, and its synchrony will change, compared to such characteristics prior to the denervation procedure. In certain embodiments, one or more of these various characteristics, such as amplitude, shape, latency, and synchrony, are used to quantify a neural fiber's health.

**In** accordance with certain embodiments, convolution is used to determine how far nerve fibers are from a sensing electrode, or more generally from a sensing site (which can also be referred to as a recording site). Typically, the larger the amplitude of the sensed neural activity the more likely nerve fibers are close to the sensing site, and the smaller the amplitude of the sensed neural activity the more likely the nerve fibers are far from the sensing site. Assume for example the neural activity of two different nerve fibers are sensed using a sensing electrode on a catheter, and that one of the nerve fibers is relatively far away from the sensing site, while another one of the nerve fibers is relatively close to the sensing site. Now also assume that both the relative far and the relatively close nerve fibers fire at the same time in response to stimulation energy that is intended to evoke a neural response. When a catheter is used to sense the above described neural activity, characteristics of both the relatively far and the relatively near neural fibers can be distinguished from one another in a sensed signal, which can enable a system and/or physician to approximate how far the different neural fibers are from the sensing site, as well as the size of such fibers, which are examples of characteristics of sensed neural activity that can be determined at step 806 in FIG. 18.

In accordance with certain embodiments, the characteristics of the sensed neural activity that are identified at step 806, and used to select denervation parameters at step 108, include a frequency and/or selection of electrode pairs to selectively activate a portion of the transducer 300 to effect the direction and/or depth of an acoustic signal traveling away from the transducer 300, a minimum amount of energy delivered with a specific waveform to get an evoked response and/or the amount of energy delivered with the specific waveform that achieves saturation of the evoked neural response. The evoked neural response can be considered saturated when increases to the neural stimulation no longer increase the evoked neural response. These characteristics can be used to select a target area for therapy and/or an amount of therapy to be delivered to the nerve fiber to get full nerve capture and a full effect. More generally, these characteristics can be used to select denervation parameters at step 808 in FIG. 18.

In an embodiment, instead of, or in addition to, performing stimulation using electrical energy to evoke a neural response, the neural response is evoked by heating nerves at a temperature lower than a temperature at which ablation occurs to evoke a neural response and sense a baseline neural response prior to ablation, and/or to sense a post-ablation neural response to determine whether denervation was successful or if additional denervation needs to be performed. The same transducer that is used for ablation can also be used to evoke a neural response prior to ablation by using the transducer to heat an artery to a temperature sufficient to evoke a neural response but not high enough to cause ablation. This could make a catheter less complex by eliminating the need for stimulation electrodes to evoke the neural response.

In an embodiment, at step 802, a frequency and/or pairs of electrodes of the transducer 300 is/are used to activate a selected portion of the transducer in order to output an acoustic signal with sufficient power to heat nerves at a temperature lower than a temperature at which ablation occurs, such as to evoke a neural response and sense a baseline neural response prior to ablation. At step 804, neural activity is sensed using at least one nerve sensing electrode of the catheter. In an embodiment, steps 802 and 804 are repeated 360° around the blood vessel. At step 806, characteristics of the sensed neural activity at particular vectors may be determined based on the characteristics of the sensed neural activity. In embodiment wherein steps 802 and 804 are repeated 360° around the blood vessel, the nerve profile around the circumference may be determined. At step 808, the electronics 22 can determine the optimal vector(s) of ablation and select the corresponding denervation frequency(ies) and/or pairs(2) of electrodes of the transducer 300, as well as other denervation parameters. The vectors may also be determined based on imaging described herein with reference to FIG. 17. At step 810, the denervation procedure may be performed using the selected denervation parameters to thereby denervate at least some of the nerves of interest for which the neural activity was sensed.

In an embodiment, after ablation, at step 812, the effectiveness of the ablation procedure may be confirmed. A frequency and/or pairs of electrodes of the transducer 300 may be selected to activate a specific portion of the transducer in order to again output an acoustic signal at a particular vector with sufficient power to heat nerves along the vector at a temperature lower than a temperature at which ablation occurs, such as to evoke a neural response and sense a post-ablation neural response. If it is determined that ablation was not effective, ablation may then again be applied, e.g., to a particular vector around the blood vessel, and/or the catheter may be moved to another location along the blood vessel.

### Example Electrical Control Unit (ECU)

FIG. 19 is a high-level block diagram of an electrical control unit (ECU) 1102 that is configured to be in electrical communication with a catheter, such as the catheter 10 described above. The ECU 1102, and the catheter (e.g., 10) to which the ECU 1102 is electrically coupled via a cable, can be referred to more generally as a system 1100. The ECU 1102 can process a received signal to produce an output signal, and present information including information about the output signal, the received signal, or processing information. Such a system can be used, for example, in diagnostic procedures for determining the location of target and non-target structures and/or assessing the status of a patient's nervous activity proximate a biological lumen, such as a vein or an artery, e.g., a renal artery, or another type of blood vessel. Such a system can be additionally, or alternatively, used to select preferred ablation parameters, e.g., the frequency of the alternating current output by electronics 22 for use in an ablation procedure. A same catheter that is used to assess the status of a patient's nervous activity proximate and/or select preferred denervation parameters can also be used to perform a denervation procedure, as detailed in U.S. Patent Application No. 18/182,821, to Barman et al., filed March 13, 2023 (PMD00104US), which claims priority to U.S. Patent Application No. 63/320103. Alternatively, it is possible that the catheter that is used to perform a denervation procedure differs from the catheter that is used to assess that status of a patient's nervous activity proximate a biological lumen, in which case different catheters can be swapped in and out of a biological lumen during a procedure.

Still referring to FIG. 11, the ECU 1102 includes a stimulator 1106 electrically coupled to a selected pair of nerve stimulation electrodes of the catheter 10. The stimulator 1106 which is part of a STIM circuit or subsystem 1105, can selectively emit electrical signals (including stimulation pulses) having a specific voltage, amperage, duration, duty cycle and/or frequency of application that will cause nerve cell activation. For an example, one nerve stimulation electrode can be connected as a stimulation anode and another nerve stimulation electrode can be connected as the stimulation cathode, or vice versa. Switches, which are not specifically shown, can be used to selectively control how various nerve stimulation electrodes are coupled to various nodes of the ECU 1102, such as to the input terminals of the amplifier 1112, or to the output terminals of the stimulator 1106. In this manner, the switches can be used to control which electrodes are configured as stimulation electrodes and which electrodes are configured as sensing electrodes.

Upon receiving the stimulation signal produced by the stimulator 1106, the electrodes of the catheter 10 that are connected as nerve stimulation electrodes can apply electrical energy to a patient's nerves through the biological lumen wall based on the received signal. Such stimulus can have any of a variety of known waveforms, such as a sinusoid, a square wave form or a triangular wave form, but is not limited thereto. In various examples, the stimulation can be applied for durations between approximately 0.05 milliseconds (msec) and approximately 8 msec.

The stimulation of nerves can be performed to evoke an elicited potential, which can cause such a potential to propagate in every direction along the nerve fibers. More generally, the STIM subsystem 1105 can be used to deliver electrical stimulation via a selected pair of electrodes in order to evoke a neural response, and the SENS subsystem 1104 can be used to sense the evoked neural response.

In some embodiments, the ECU 1102 can digitally sample the signal sensed using a pair of electrodes to receive the electrical signal from the catheter 10. In alternate embodiments, the signal can be recorded as an analog signal. When receiving an electrical signal from the electrodes on the catheter 10, the ECU 1102 can perform filtering and/or other processing steps on the signal. Generally, such steps can be performed to discriminate the signal sensed by the catheter from any background noise within the patient's vasculature such that the resulting output is predominantly the signal from nerve cell activation. In some instances, the ECU 1102 can modulate the electrical impedance of the signal receiving portion in order to accommodate the electrical properties and spatial separation of the electrodes mounted on the catheter in a manner to achieve the highest fidelity, selectively and resolution for the signal received. For example, electrode size, separation, and conductivity properties can impact the field strength at the electrode/tissue interface.

Additionally, or alternatively, the ECU 1102 can comprise a headstage and/or an amplifier to perform any of offsetting, filtering, and/or amplifying the signal received from the catheter. In some examples, a headstage applies a DC offset to the signal and performs a filtering step. In some such systems, the filtering can comprise applying notch and/or band-pass filters to suppress particular undesired signals having a particular frequency content or to let pass desired signals having a particular frequency content. An amplifier can be used to amplify the entire signal uniformly or can be used to amplify certain portions of the signal more than others. For example, in some configurations, the amplifier can be configured to provide an adjustable capacitance of the recording electrode, changing the frequency dependence of signal pick-up and amplification. In some embodiments, properties of the amplifier, such as capacitance, can be adjusted to change amplification properties, such as the resonant frequency, of the amplifier.

In the illustrated embodiment of FIG. 19, the ECU 1102 includes an amplifier 1112 including a non-inverting (+) input terminal, an inverting (-) input terminal, a power supply input terminal, and a ground or reference terminal. As can be appreciated from FIG. 19, the non-inverting (+) input terminal can be coupled to the electrode 326, the inverting (-) input terminal can be electrically coupled to the electrode 327, the power supply input terminal is electrically coupled to a voltage source (e.g., a reference voltage generator), and the ground or reference terminal is electrically coupled to a ground reference electrode, which can be located on the catheter 10, can be located on a distal end of an introducer sheath, or can be located on the skin of the patient, but is not limited thereto.

In some embodiments, the ECU 1102 can include a switching network configured to interchange which of electrodes of a catheter (e.g., 10) are coupled to which portions of the ECU. In some such embodiments, a user can manually switch which inputs receive connections to which electrodes of the catheter 10. Such configurability allows for a system operator to adjust the direction of propagation of the elicited potential as desired. For example, the switching network, or more generally switches, can be used to connect the nerve stimulation electrodes to the stimulator 1106 during a period of time during which stimulation pulses are to be emitted by the catheter 10, and the switches can be used to connect the electrodes 326 and 326 to the amplifier 312 to sense the elicited response to the stimulation pulses. Additionally, or alternatively, a controller (e.g., 1122) can autonomously control such a switching network.

The amplifier 1112 can include any appropriate amplifier for amplifying desired signals or attenuating undesired signals. In some examples, the amplifier has a high common-mode rejection ratio (CMRR) for eliminating or substantially attenuating undesired signals present in each of at each of the sensing electrodes. In some embodiments, the amplifier 1112 can be adjusted, for example, via an adjustable capacitance or via other attributes of the amplifier.

In the example system 1100 of FIG. 19, the ECU 1102 further includes an amplifier 1114 for enhancing the desired signal in the signal received via a pair of the electrodes. The amplifier 1114 can include a band-pass filter, a notch filter, or any other appropriate filter to isolate desired signals from noise artifacts within the received signals. In some embodiments, various properties of the amplifier 1114 can be adjusted to manipulate its filtering characteristics. For example, the filter may include an adjustable capacitance or other parameter to adjust its frequency response.

At least one of amplification and filtering of a sensed signal (e.g., received at the nerve stimulation electrodes) can allow for extraction of the desired signal at 1116. In some embodiments, extraction 1116 comprises at least one additional processing step to isolate desired signals from the signal sensed using electrodes such as preparing the signal for output at 1118. In some embodiments, the functionalities of any combination of amplifier 1112, amplifier 1114, and extraction 1116 may be combined into a single entity. For instance, the amplifier 1112 may act to filter undesired frequency content from the signal without requiring additional filtering at a separate filter.

In some embodiments, the ECU 1102 can record emitted stimuli and/or received signals. Such data can be subsequently stored in permanent or temporary memory 1120. The ECU 1102 can comprise such memory 1120 or can otherwise be in communication with external memory (not shown). Thus, the ECU 1102 can be configured to emit stimulus pulses to electrodes of the catheter, record such pulses in a memory, receive signals from the catheter, and also record such received signal data. The memory 1120 in or associated with the ECU 1102 can be internal or external to any part of the ECU 1102 or the ECU 1102 itself.

The ECU 1102 or separate external processor can further perform calculations on the stored data to determine characteristics of signals either emitted or received via the catheter. For example, in various embodiments, the ECU 1102 can determine any of the amplitude, duration, or timing of occurrence of the received or emitted signals. The ECU 1102 can further determine the relationship between the received signal and the emitted stimulus signal, such as a temporal relationship therebetween. In some embodiments, the ECU 1102 performs signal averaging on the signal data received from the catheter. Such averaging can act to reduce random temporal noise in the data while strengthening the data corresponding to any elicited potentials received by the catheter.

Averaging as such can result in a signal in which temporally random noise is generally averaged out and the signal present in each recorded data set, such as elicited potentials, will remain high. In some embodiments, each iteration of the process can include a synchronization step so that each acquired data set can be temporally registered to facilitate averaging the data. That is, events that occur consistently at the same time during each iteration may be detected, while temporally random artifacts (e.g., noise) can be reduced. In general, the signal to noise ratio (SNR) resulting in such averaging will improve by the square root of the number of samples averaged in order to create the averaged data set.

The ECU 1102 can further present information regarding any or all of the applied stimulus, the signal, and the results of any calculations to a user of the system, e.g., via output 1118. For example, the ECU 1102 can generate a graphical display providing one or more graphs of signal strength vs. time representing the stimulus and/or the received signal.

In some embodiments, the ECU 1102 can include a controller 1122 in communication with one or both of stimulator 1106 and SENS subsystem 1104. The controller 1122 can be configured to cause stimulator 1106 to apply a stimulation signal to a catheter, e.g., the catheter 10. Additionally or alternatively, the controller 1122 can be configured to analyze signals received and/or output by the SENS subsystem 1104. In some embodiments, the controller 1122 can act to control the timing of applying the stimulation signal from stimulator 1106 and the timing of receiving signals by the SENS subsystem 1104. The controller 1122 can be implemented, e.g., using one or more processors, field programmable gate arrays (FPGAs), state machines, and/or application specific integrated circuits (ASICs), but is not limited thereto.

Example electrical control units have been described. In various embodiments, the ECU 1102 can emit stimulus pulses to the catheter 10, receive signals from the catheter 10, perform calculations on the emitted and/or received signals, and present the signals and/or results of such calculations to a user. In some embodiments, the ECU 1102 can comprise separate modules for emitting, receiving, calculating, and providing results of calculations. Additionally or alternatively, the functionality of controller 1122 can be integrated into the ECU 1102 as shown, or can be separate from and in communication with the ECU.

The controller 1122 can also control a fluid supply subsystem 1128, which can include a cartridge and a reservoir, which are described below with reference to FIG. 1, but can include alternative types of fluid pumps, and/or the like. The fluid supply subsystem 1128 is fluidically coupled to one or more fluid lumens (not shown) within the catheter shaft 12 which in turn are fluidically coupled to the balloon 14. The fluid supply subsystem 1128 can be configured to circulate a cooling liquid through the catheter 10 to the transducer 200 (or 300) in the balloon 14 to cool the transducer and protect the body lumen. The fluid supply subsystem 1128 is an example of the fluid source 28 discussed above with reference to FIG. 1A.

An ultrasound excitation source 1126 may be electrically coupled to inner and outer electrodes of the transducer 300 through electrical conductor 72 (FIG. 12A), and may actuate the transducer 300 by applying a voltage between the inner and outer electrodes (or any other pair of electrodes), so as to cause the piezoelectric material of the piezoelectric transducer body 208 to generate an unfocused ultrasonic wave that radiates radially outwardly.

Controller 1122 can be configured to cause ultrasound excitation source 1126 to apply a specific frequency to the transducer 300 to direct the acoustic signal toward one or more targets and/or away from one or more non-target structures.

In some embodiments, the controller 1122 can act to cause ultrasound excitation source 1126 to apply an alternating current between a selected group of electrodes of the transducer 300 (e.g., between electrodes 36 and 60 or between electrodes 36 and 62 of FIG. 13) to direct the acoustic signal towards one or more targets and/or away from one or more non-target structures.

Additionally or alternatively, the controller 1122 can be configured to analyze signals received and/or output by the SENS subsystem 1104. In certain embodiments, the controller 1122 can act to cause ultrasound excitation source 1126 to apply an alternating current at a specific frequency to the transducer 300 to direct the acoustic signal towards near field nerves. In some embodiments, the controller 1122 can act to cause ultrasound excitation source 1126 to apply an alternating current at a specific frequency to the transducer 300 to direct the acoustic signal towards far field nerves.

### Multidose Treatments

Because the air-backed ultrasound transducers described herein are able to provide both low and high power density treatments, without the transducer breaking down, such an air-backed ultrasound transducer can advantageously be used to provide both low power density and high power density ultrasound treatments from a same longitudinal location within a body lumen (e.g., a renal artery). This can involve, for example, delivering ultrasound treatment having a low power density for a first period of time (aka a first duration) while an air-backed ultrasound transducer is positioned proximate to a nerves (surrounding a body lumen) that are to be denervated, and then delivering ultrasound treatment having a high power density for a second period of time (aka a second duration) while the same air-backed ultrasound transducer is positioned proximate to the same nerves (surrounding the body lumen) that are to be denervated. In other words, the low power density and high power density treatments can be delivered from the same air-backed transducer without moving the transducer. It would also be possible for the high power density treatment to be delivered prior to the low power density treatment. In accordance with certain embodiments, the first duration is within the first range of 2 to 4 seconds, and the second duration is within a second range of 2 to 4 seconds. In certain embodiments, the air-backed ultrasound transducer is controlled to abstain from emitting ultrasonic waves for a third period of time (aka a third duration) between the first and second periods of time (between the first and second durations). This third period of time can similarly have a duration within a third range of 2 to 4 seconds. In certain embodiments, where the air-backed transducer is within a balloon through which a cooling fluid is circulated, the cooling fluid is circulated through the balloon during the third period of time, between the first and second periods of time, to thereby cool the transducer and the tissue surrounding the transducer between the two sonication periods. Such embodiments are described below with reference to FIG. 20, which is a high-level flow diagram describing methods for use with a catheter including an air-backed ultrasound transducer on a distal portion of the catheter, according to certain embodiments of the present technology.

Referring to FIG. 20, step 1202 involves inserting the distal portion of the catheter into a body lumen of a patient so that the air-backed ultrasound transducer is positioned proximate to nerves surrounding the body lumen that are to be denervated. Step 1204 involves causing the air-backed ultrasound transducer to emit ultrasonic waves having a first power density for a first period of time, while the air-backed ultrasound transducer is positioned proximate to the nerves surrounding the body lumen that are to be denervated. Step 1206 involves causing the air-backed ultrasound transducer to emit ultrasonic waves having a second power density for a second period of time that occurs after the first period of time, while the air-backed ultrasound transducer is positioned proximate to the nerves surrounding the body lumen that are to be denervated, wherein the second power density differs from the first power density. In accordance with certain embodiments, the first period of time has a duration within a first range of 2 to 4 seconds, and the second period of time has a duration within a second range of 2 to 4 seconds. In accordance with certain embodiments, the method also includes, at step 1205 (which occurs between steps 1204 and 1206) causing the air-backed ultrasound transducer to abstain from emitting ultrasonic waves for a third period of time between the first and second periods of time. In certain embodiments, the third period of time has a duration within a third range of 2 to 4 seconds. As noted above, during this third period of time, a cooling fluid may be circulated through a balloon, if the air-backed ultrasound transducer is located within a balloon.

A benefit of using the two different ultrasound power densities (one low and one high) for delivering ultrasound energy at the same treatment site using the embodiments summarized with reference to FIG. 20 is that it could help with not heating deeper while trying to bring an inner lesion extent closer to a body lumen surface (e.g., the renal artery surface). It can also help the elongate the lesion created axially, increasing the probability of nerve denervation.

Still referring to the embodiments described with reference to FIG. 20, in certain embodiments, one of the first and second power densities is within a range of 170 Watts per centimeter square (W/cm2) to 327 W/cm2, and the other one of the first and second power densities is within a range of 50 W/cm2 to 169 W/cm2.5). Other variations are also possible and within the scope of the embodiments described herein.

### Reducing Active Treatment Duration

When a catheter (e.g., 10) with an ultrasound transducer (e.g., 200, 300) is used to perform a denervation procedure (e.g., a renal denervation procedure), the longer the amount of time (i.e., the active treatment duration) that it takes to perform the denervation procedure, the longer the patient on which the procedure is being performed may experience pain and/or discomfort. Certain embodiments of the present technology, which will now be described below, can be used to reduce patient pain/discomfort through maximizing the peak treatment temperature and minimizing the active treatment duration (aka active ablation duration).

Tissue coagulation is achieved by thermal dose accumulation. Beyond 43 degrees Celsius (C), thermal dose doubles in any given temporal interval for every one degree of Celsius increase in temperature. Beyond 65°C, tissue necrose occurs instantaneously. Thus, increasing the treatment temperature faster to the target temperature, or a high temperature, can dramatically reduce the active treatment time (aka the active ablation duration) while maintaining efficacy. Thus, a maximum peak temperature method, according to certain embodiments of the present technology, can be applied to deliver effective treatment with minimum active treatment time (aka with minimum active ablation duration). In certain such embodiments, the total treatment power can be predetermined such that it is optimized based on the target tissue, transducer geometry, and acoustic parameters such as frequency and attenuation.

In some embodiments, the power delivered to the patient is programmed to increase the temperature continuously until the end of the ablation at one target site. In other embodiments, the power is programmed to raise the temperature as fast as possible until the targeted peak temperature, then is lowered to maintain that temperature until the denervation treatment is complete.

The peak temperature location for acoustic thermal ablation depends on various factors, such as transducer geometry, operating frequency, acoustic intensity, treatment duration, tissue properties, anatomy, and so on. For a given denervation application, the peak temperature position relative to the transducer face can be pre-determined or characterized on bench models or thermal simulations. With this knowledge, acoustic power can be programmed precisely to achieve the target maximum temperature to enable effective treatment and to achieve the shortest treatment time, thus minimizing the patient discomfort/pain. For example, in a renal denervation (RDN) application, first a target peak temperature is determined, in an embodiment such that the temperature is just below the boiling point (e.g., 99°C) of the tissue being treated. Next, patient entry power is programmed according to a size of the body lumen (e.g., renal artery) to achieve the target temperature in the ablation space around the body lumen (e.g., renal artery). In such embodiments, given the short active ablation duration, typically of only a few seconds, thermal conduction has minimum impact. Therefore, a strategy of constant delivered acoustic energy can be used to trade power versus time. For example, for an 8mm diameter vessel, unfocused ultrasound therapy may be delivered at about 235 Joules (J) of acoustic energy for 7 seconds with a maximum peak temperature of about 70°C. If the strategy of this invention is applied, and the target temperature is instead 99°C (instead of 70°C), then a 70W entry power for the same treatment frequency (assuming a constant power during the treatment) can be applied to shorten the treatment time to around 3.5 seconds, which is half of the 7 seconds duration used when the target temperature is 70°C.

Alternatively, or additionally, the active treatment duration can be reduced by increasing an acoustic operating frequency. Only a portion of the total transmitted acoustic energy is converted into heat in targeted tissue. This portion increases with increases in the acoustic frequency. Thus, at the same total acoustic power, using higher frequency ultrasound generates more heat, and thus a faster temperature rise. For example, a 15 MHz unfocused ultrasound signal generates about 40% more heat within the first 6mm in tissue being treated compared to a 9 MHz unfocused ultrasound signal, when the transducer surface geometry and total power are the same, which can help reduce the active treatment duration to less than 5 seconds.

In some embodiment, acoustic power is set constant to increase the tissue temperature continuously until the end of the treatment. In some other embodiment, a higher acoustic power is delivered at the beginning to raise the temperature rapidly until the target temperature is achieved. Then the generator switches to a lower power to maintain the temperature until the end of the treatment. In certain such embodiments, pulsing with a predetermined duty cycle factor (on/off duration ratio) can be used to achieve the same effect of maintaining the temperature.

## Claims

1. A tissue treatment device comprising:
a catheter (10) having a distal end;
an ultrasound transducer (200) positioned at the distal end of the catheter (10), the ultrasound transducer (200) comprising an inner surface (207) comprising an inner electrode (204) and an outer surface (206) comprising an outer electrode (205); and
a backing support member (210), wherein the ultrasound transducer (200) is mounted to the backing support member (210) to define a gas or vacuum chamber (230), the gas or vacuum chamber (230) being insulated to prevent entry of fluid into the gas or vacuum chamber (230) during use, the backing support member (210) having a distal end and a proximal end, wherein the backing support member (210) comprises at least a first stand-off post (212) at the distal end of the backing support member (210) and at least a second stand-off post (212) at the proximal end of the backing support member (210), wherein each of the at least first and second stand-off posts (212) comprise an inner face (212a) and an outer face (212b), the inner faces (212a) being on an interior of the gas or vacuum chamber (230), the outer faces (212b) being exterior to the gas or vacuum chamber (230), **characterized in that**
the at least first and second stand-off posts (212) are soldered to the ultrasound transducer (200) only at the outer face (212b) of the at least first and second stand-off posts (212); and
the gas or vacuum chamber (230) is adjacent the inner surface (207).

2. The tissue treatment device of claim 1, wherein the at least first and second stand-off posts (212) are soldered using a solder preform.

3. The tissue treatment device of claim 1 or 2, wherein the backing support member (210) is one piece.

4. The tissue treatment device of claim 1 or 2, wherein the backing support member (210) is at least two separate pieces.

5. The tissue treatment device of any one of claims 1 through 4, wherein the ultrasound transducer (200) comprises a hollow piezoelectric transducer body (208) comprising a tube of piezoelectric material, wherein said tube has a longitudinal axis, a radially outer surface (206) and a radially inner surface (207), and wherein the inner electrode (204) is disposed on at least a portion of the radially inner surface (207) and the outer electrode (205) is disposed on at least a portion of the radially outer surface (206).

6. The tissue treatment device of claim 5, wherein a central portion of the piezoelectric transducer body (208) is configured to vibrate and thereby generate ultrasonic waves in response to application of a voltage between the inner and outer electrodes (204, 205),
proximal and distal portions of the piezoelectric transducer body (208), between which the central portion of the piezoelectric transducer body (208) is located, are configured to not vibrate in response to application of the voltage between the inner and outer electrodes (204, 205), and
the first and second stand-off posts (212) are attached, respectively, to the proximal and distal portions of the piezoelectric transducer body (208) that are configured to not vibrate in response to application of the voltage between the inner and outer electrodes (204, 205).

7. The tissue treatment device of claim 6, wherein the first and second stand-off posts (212) are attached, respectively, to the proximal and distal portions of the piezoelectric transducer body (208) by solder and/or adhesive.

8. The tissue treatment device of any one of claims 1 through 7, further comprising:
at least a second transducer (200), wherein the backing support member (210) comprises a bridge portion extending between the first and second transducers (200), the bridge portion including one or more enhanced flexibility regions.

9. The tissue treatment device of claim 8, wherein the one or more enhanced flexibility regions of the bridge portion include openings through a wall of the backing member (210).

10. The tissue treatment device of claim 9, wherein the openings spiral around a longitudinal axis of the bridge portion of the backing support member (210).

11. The tissue treatment device of any one of claims 8 through 10, wherein the one or more enhanced flexibility regions of the bridge portion is cut into a lattice pattern.

12. The tissue treatment device of any one of claims 1 through 11, wherein the backing support member (210) comprises electroless nickel immersion gold plated stainless steel.

13. The tissue treatment device of any one of claims 1 through 12, further comprising:
a first electrical conductor (20) soldered to a proximal portion (250) of the backing support member (210); and
a second electrical conductor (20) soldered to a proximal portion of the outer electrode (206), wherein:
the first stand-off post (212) is attached to the inner electrode (204),
each of the backing support member (210) and the first stand-off post (212) are electrically conductive such that the first electrical conductor (20), that is soldered to the proximal portion (250) of backing support member (210), is electrically coupled to the inner electrode (204), and
the proximal portion (250) of the backing support member (210), to which the first electrical conductor (20) is soldered, is covered by a dielectric to inhibit a short circuit from occurring between the inner and outer electrodes (204, 206) when the ultrasound transducer (200) is placed within electrically conductive fluid and a voltage is applied between the inner and outer electrodes (204, 206).

14. The tissue treatment device of claim 13, wherein the dielectric, which covers the proximal portion (250) of the backing support member (210), to which the first electrical conductor (20) is soldered, comprises a conformal coating including at least one of parylene or polyimide.

15. The tissue treatment device of claim 13, wherein the dielectric, which covers the proximal portion (250) of the backing support member (210), to which the first electrical conductor (20) is soldered, comprises a dielectric tube (290).

16. The tissue treatment device of claim 15, further comprising a dielectric adhesive within at least a portion of an interior of the dielectric tube (290).

## Patentansprüche

1. Gewebebehandlungsvorrichtung, umfassend:
einen Katheter (10), der ein distales Ende aufweist;
einen Ultraschallwandler (200), der an dem distalen Ende des Katheters (10) positioniert ist, wobei der Ultraschallwandler (200) eine Innenfläche (207), die eine innere Elektrode (204) umfasst, und eine Außenfläche (206) umfasst, die eine äußere Elektrode (205) umfasst; und
ein Rückstützelement (210), wobei der Ultraschallwandler (200) an dem Rückstützelement (210) montiert ist, um eine Gas- oder Vakuumkammer (230) zu definieren, wobei die Gas- oder Vakuumkammer (230) isoliert ist, um einen Eintritt von Fluid in die Gas- oder Vakuumkammer (230) während des Gebrauchs zu verhindern, wobei das Rückstützelement (210) ein distales Ende und ein proximales Ende aufweist, wobei das Rückstützelement (210) mindestens einen ersten Abstandshalterstift (212) an dem distalen Ende des Rückstützelements (210) und mindestens einen zweiten Abstandshalterstift (212) an dem proximalen Ende des Rückstützelements (210) umfasst, wobei jeder des mindestens ersten und zweiten Abstandshalterstifts (212) eine Innenfläche (212a) und eine Außenfläche (212b) umfasst, wobei sich die Innenflächen (212a) in einem Inneren der Gas- oder Vakuumkammer (230) befinden, wobei sich die Außenflächen (212b) außerhalb der Gas- oder Vakuumkammer (230) befinden, **dadurch gekennzeichnet, dass**
der mindestens erste und zweite Abstandshalterstift (212) nur an der Außenfläche (212b) des mindestens ersten und zweiten Abstandshalterstifts (212) an den Ultraschallwandler (200) gelötet sind; und
die Gas- oder Vakuumkammer (230) benachbart zu der Innenfläche (207) ist.

2. Gewebebehandlungsvorrichtung nach Anspruch 1, wobei der mindestens erste und zweite Abstandshalterstift (212) unter Verwendung einer Lötvorform verlötet sind.

3. Gewebebehandlungsvorrichtung nach Anspruch 1 oder 2, wobei das Rückstützelement (210) einstückig ist.

4. Gewebebehandlungsvorrichtung nach Anspruch 1 oder 2, wobei das Rückstützelement (210) mindestens zwei separate Stücke ist.

5. Gewebebehandlungsvorrichtung nach einem der Ansprüche 1 bis 4, wobei der Ultraschallwandler (200) einen hohlen piezoelektrischen Wandlerkörper (208) umfasst, der ein Rohr aus piezoelektrischem Material umfasst, wobei das Rohr eine Längsachse, eine radial äußere Oberfläche (206) und eine radial innere Oberfläche (207) aufweist und wobei die innere Elektrode (204) auf mindestens einem Abschnitt der radial inneren Oberfläche (207) angeordnet ist und die äußere Elektrode (205) auf mindestens einem Abschnitt der radial äußeren Oberfläche (206) angeordnet ist.

6. Gewebebehandlungsvorrichtung nach Anspruch 5, wobei ein mittlerer Abschnitt des piezoelektrischen Wandlerkörpers (208) dazu konfiguriert ist, zu vibrieren und dadurch Ultraschallwellen als Reaktion auf ein Anlegen einer Spannung zwischen der inneren Elektrode und der äußeren Elektrode (204, 205) zu erzeugen;
ein proximaler und ein distaler Abschnitt des piezoelektrischen Wandlerkörpers (208), zwischen denen sich der mittlere Abschnitt des piezoelektrischen Wandlerkörpers (208) befindet, dazu konfiguriert sind, als Reaktion auf ein Anlegen der Spannung zwischen der inneren Elektrode und der äußeren Elektrode (204, 205) nicht zu vibrieren; und
der erste und der zweite Abstandshalterstift (212) an dem proximalen Abschnitt bzw. an dem distalen Abschnitt des piezoelektrischen Wandlerkörpers (208) angebracht sind, die dazu konfiguriert sind, als Reaktion auf ein Anlegen der Spannung zwischen der inneren Elektrode und der äußeren Elektrode (204, 205) nicht zu vibrieren.

7. Gewebebehandlungsvorrichtung nach Anspruch 6, wobei der erste Abstandshalterstift und der zweite Abstandshalterstift (212) durch Lötmittel und/oder Klebstoff an dem proximalen Abschnitt bzw. an dem distalen Abschnitt des piezoelektrischen Wandlerkörpers (208) angebracht sind.

8. Gewebebehandlungsvorrichtung nach einem der Ansprüche 1 bis 7, ferner umfassend:
mindestens einen zweiten Wandler (200), wobei das Rückstützelement (210) einen Brückenabschnitt umfasst, der sich zwischen dem ersten und dem zweiten Wandler (200) erstreckt, wobei der Brückenabschnitt eine oder mehrere Regionen mit verbesserter Flexibilität beinhaltet.

9. Gewebebehandlungsvorrichtung nach Anspruch 8, wobei die eine oder die mehreren Regionen mit verbesserter Flexibilität des Brückenabschnitts Öffnungen durch eine Wand des Rückstützelements (210) beinhalten.

10. Gewebebehandlungsvorrichtung nach Anspruch 9, wobei die Öffnungen spiralförmig um eine Längsachse des Brückenabschnitts des Rückstützelements (210) angeordnet sind.

11. Gewebebehandlungsvorrichtung nach einem der Ansprüche 8 bis 10, wobei die eine oder die mehreren Regionen mit verbesserter Flexibilität des Brückenabschnitts in ein Gittermuster geschnitten werden.

12. Gewebebehandlungsvorrichtung nach einem der Ansprüche 1 bis 11, wobei das Rückstützelement (210) mittels Electroless Nickel/Immersion Gold plattierten Edelstahl umfasst.

13. Gewebebehandlungsvorrichtung nach einem der Ansprüche 1 bis 12, ferner umfassend:
einen ersten elektrischen Leiter (20), der an einen proximalen Abschnitt (250) des Rückstützelements (210) gelötet ist; und
einen zweiten elektrischen Leiter (20), der an einen proximalen Abschnitt der äußeren Elektrode (206) gelötet ist, wobei:
wobei der erste Abstandshaltestift (212) an der inneren Elektrode (204) angebracht ist;
das Rückstützelement (210) und auch der erste Abstandshalter stift (212) jeweils elektrisch leitfähig sind, sodass der erste elektrische Leiter (20), der an den proximalen Abschnitt (250) des Rückstützelements (210) gelötet ist, elektrisch mit der inneren Elektrode (204) verbunden ist; und
der proximale Abschnitt (250) des Rückstützelements (210), an den der erste elektrische Leiter (20) gelötet ist, durch ein Dielektrikum bedeckt ist, um einen Kurzschluss zwischen der inneren Elektrode und der äußeren Elektrode (204, 206) zu verhindern, wenn der Ultraschallwandler (200) in einer elektrisch leitfähigen Flüssigkeit platziert wird und eine Spannung zwischen der inneren Elektrode und der äußeren Elektrode (204, 206) angelegt wird.

14. Gewebebehandlungsvorrichtung nach Anspruch 13, wobei das Dielektrikum, das den proximalen Abschnitt (250) des Rückstützelements (210) abdeckt, an den der erste elektrische Leiter (20) gelötet ist, eine konforme Beschichtung umfasst, die mindestens eines von Parylen oder Polyimid beinhaltet.

15. Gewebebehandlungsvorrichtung nach Anspruch 13, wobei das Dielektrikum, das den proximalen Abschnitt (250) des Rückstützelements (210) abdeckt, an den der erste elektrische Leiter (20) gelötet ist, ein dielektrisches Rohr (290) umfasst.

16. Gewebebehandlungsvorrichtung nach Anspruch 15, ferner umfassend einen dielektrischen Klebstoff innerhalb mindestens eines Abschnitts eines Inneren des dielektrischen Rohrs (290).

## Revendications

1. Dispositif de traitement tissulaire comprenant :
un cathéter (10) ayant une extrémité distale ;
un transducteur à ultrasons (200) positionné au niveau de l'extrémité distale du cathéter (10), le transducteur à ultrasons (200) comprenant une surface interne (207) comprenant une électrode interne (204) et une surface externe (206) comprenant une électrode externe (205) ; et
un élément de support arrière (210), dans lequel le transducteur à ultrasons (200) est monté sur l'élément de support arrière (210) pour définir une chambre à gaz ou à vide (230), la chambre à gaz ou à vide (230) étant isolée pour empêcher l'entrée de fluide dans la chambre à gaz ou à vide (230) pendant l'utilisation, l'élément de support arrière (210) ayant une extrémité distale et une extrémité proximale, dans lequel l'élément de support arrière (210) comprend au moins un premier montant d'écartement (212) au niveau de l'extrémité distale de l'élément de support arrière (210) et au moins un second montant d'écartement (212) au niveau de l'extrémité proximale de l'élément de support arrière (210), dans lequel chacun des au moins premier et second montants d'écartement (212) comprend une face interne (212a) et une face externe (212b), les faces internes (212a) étant sur un intérieur de la chambre à gaz ou à vide (230), les faces externes (212b) étant extérieures à la chambre à gaz ou à vide (230), **caractérisé en ce que**
les au moins premier et second montants d'écartement (212) sont soudés au transducteur à ultrasons (200) uniquement au niveau de la face externe (212b) des au moins premier et second montants d'écartement (212) ; et
la chambre à gaz ou à vide (230) est adjacente à la surface interne (207).

2. Dispositif de traitement tissulaire selon la revendication 1, dans lequel les au moins premier et second montants d'écartement (212) sont soudés en utilisant une préforme de soudure.

3. Dispositif de traitement tissulaire selon la revendication 1 ou 2, dans lequel l'élément de support arrière (210) est constitué d'une seule pièce.

4. Dispositif de traitement tissulaire selon la revendication 1 ou 2, dans lequel l'élément de support arrière (210) est constitué d'au moins deux pièces séparées.

5. Dispositif de traitement tissulaire selon l'une quelconque des revendications 1 à 4, dans lequel le transducteur à ultrasons (200) comprend un corps de transducteur piézoélectrique creux (208) comprenant un tube de matériau piézoélectrique, dans lequel ledit tube a un axe longitudinal, une surface radialement externe (206) et une surface radialement interne (207), et dans lequel l'électrode interne (204) est disposée sur au moins une portion de la surface radialement interne (207) et l'électrode externe (205) est disposée sur au moins une portion de la surface radialement externe (206).

6. Dispositif de traitement tissulaire selon la revendication 5, dans lequel une portion centrale du corps de transducteur piézoélectrique (208) est configurée pour vibrer et générer ainsi des ondes ultrasonores en réponse à l'application d'une tension entre les électrodes interne et externe (204, 205),
les portions proximale et distale du corps de transducteur piézoélectrique (208), entre lesquelles la portion centrale du corps de transducteur piézoélectrique (208) est située, sont configurées pour ne pas vibrer en réponse à l'application de la tension entre les électrodes interne et externe (204, 205), et
les premier et second montants d'écartement (212) sont fixés, respectivement, aux portions proximale et distale du corps de transducteur piézoélectrique (208) qui sont configurées pour ne pas vibrer en réponse à l'application de la tension entre les électrodes interne et externe (204, 205).

7. Dispositif de traitement tissulaire selon la revendication 6, dans lequel les premier et second montants d'écartement (212) sont fixés, respectivement, aux portions proximale et distale du corps de transducteur piézoélectrique (208) par de la soudure et/ou de l'adhésif.

8. Dispositif de traitement tissulaire selon l'une quelconque des revendications 1 à 7, comprenant en outre :
au moins un second transducteur (200), dans lequel l'élément de support arrière (210) comprend une portion de pont s'étendant entre les premier et second transducteurs (200), la portion de pont incluant une ou plusieurs régions à flexibilité améliorée.

9. Dispositif de traitement tissulaire selon la revendication 8, dans lequel l'une ou plusieurs régions à flexibilité améliorée de la portion de pont incluent des ouvertures à travers une paroi de l'élément de support (210).

10. Dispositif de traitement tissulaire selon la revendication 9, dans lequel les ouvertures s'enroulent en spirale autour d'un axe longitudinal de la portion de pont de l'élément de support arrière (210).

11. Dispositif de traitement tissulaire selon l'une quelconque des revendications 8 à 10, dans lequel l'une ou plusieurs régions à flexibilité améliorée de la portion de pont sont découpées en un motif de treillis.

12. Dispositif de traitement tissulaire selon l'une quelconque des revendications 1 à 11, dans lequel l'élément de support arrière (210) comprend de l'acier inoxydable plaqué or par immersion au nickel chimique.

13. Dispositif de traitement tissulaire selon l'une quelconque des revendications 1 à 12, comprenant en outre :
un premier conducteur électrique (20) soudé à une portion proximale (250) de l'élément de support arrière (210) ; et
un second conducteur électrique (20) soudé à une portion proximale de l'électrode externe (206), dans lequel :
le premier montant d'écartement (212) est fixé à l'électrode interne (204),
chacun de l'élément de support arrière (210) et du premier montant d'écartement (212) est électriquement conducteur de sorte que le premier conducteur électrique (20), qui est soudé à la portion proximale (250) de l'élément de support arrière (210), est couplé électriquement à l'électrode interne (204), et
la portion proximale (250) de l'élément de support arrière (210), à laquelle le premier conducteur électrique (20) est soudé, est recouverte par un diélectrique pour empêcher qu'un court-circuit ne se produise entre les électrodes interne et externe (204, 206) lorsque le transducteur à ultrasons (200) est placé à l'intérieur d'un fluide électriquement conducteur et qu'une tension est appliquée entre les électrodes interne et externe (204, 206).

14. Dispositif de traitement tissulaire selon la revendication 13, dans lequel le diélectrique, qui recouvre la portion proximale (250) de l'élément de support arrière (210), à laquelle le premier conducteur électrique (20) est soudé, comprend un revêtement conforme incluant au moins l'un parmi le parylène ou le polyimide.

15. Dispositif de traitement tissulaire selon la revendication 13, dans lequel le diélectrique, qui recouvre la portion proximale (250) de l'élément de support arrière (210), à laquelle le premier conducteur électrique (20) est soudé, comprend un tube diélectrique (290).

16. Dispositif de traitement tissulaire selon la revendication 15, comprenant en outre un adhésif diélectrique à l'intérieur d'au moins une portion d'un intérieur du tube diélectrique (290).
